(19) Europäisches Patentamt
European Patent Office
Office européen des brevets

(11) EP 4 741 427 A1

(12) **EUROPEAN PATENT APPLICATION**
published in accordance with Art. 153(4) EPC

(43) Date of publication:
**13.05.2026 Bulletin 2026/20**

(21) Application number: **24862014.8**

(22) Date of filing: **05.09.2024**

(51) International Patent Classification (IPC):
***C08B 37/00*** *(2006.01)* ***A61K 31/715*** *(2006.01)*

(52) Cooperative Patent Classification (CPC):
**A61K 31/715; A61P 35/00; C08B 37/00;**
Y02P 20/55

(86) International application number:
**PCT/CN2024/117022**

(87) International publication number:
**WO 2025/051174 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.09.2023 CN 202311139456**

(71) Applicant: **Peking University**
**Beijing 100871 (CN)**

(72) Inventors:
- **YE, Xinshan**
  **Beijing 100871 (CN)**
- **QIN, Xianjin**
  **Beijing 100871 (CN)**
- **XU, Chenghao**
  **Beijing 100871 (CN)**
- **MO, Juan**
  **Beijing 100871 (CN)**

(74) Representative: **Patentree**
**Edificio Net**
**Rua de Salazares, 842**
**4149-002 Porto (PT)**

(54) **GLYCAN, PREPARATION METHOD THEREFOR AND USE THEREOF, AND ANTI-TUMOR DRUG**

(57) A glycan, a preparation method therefor and a use thereof, and an anti-tumor drug, relating to the technical field of medicines. The glycan having a structure as shown in formula I has good anti-tumor activity and good safety. The results of the test example show that the glycan has a good proliferation inhibition effect on pancreatic cancer cells, has no cytotoxicity to the growth of hepatocytes, and shows better safety characteristics compared with a positive drug gemcitabine; in addition, the pancreatic cancer cells are sensitive to the glycan, thereby overcoming the problem that the pancreatic cancer cells have certain drug resistance to gemcitabine.

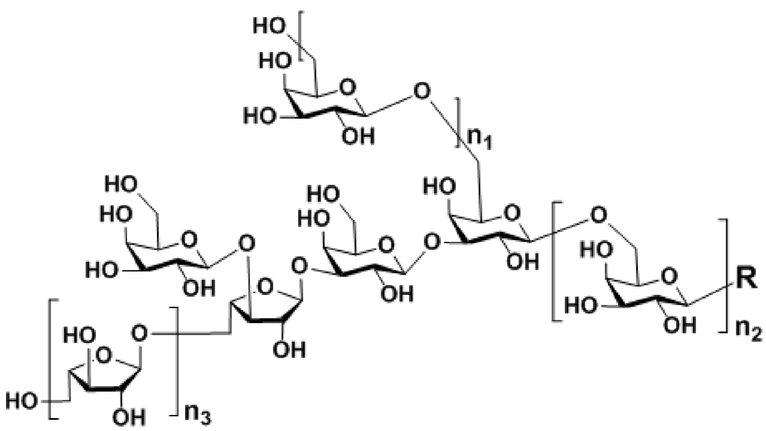

FIG. 1



EP 4 741 427 A1

## Description

The present application claims priority to Chinese Patent Application No. CN202311139456.1 filed with the China National Intellectual Property Administration (CNIPA) on September 05, 2023 and entitled "GLYCAN AND PREPARATION METHOD AND USE THEREOF, AND ANTITUMOR DRUG", which is incorporated herein by reference in its entirety.

## TECHNICAL FIELD

The present disclosure relates to the technical field of pharmaceuticals, and in particular relates to a glycan and a preparation method and use thereof, and an antitumor drug.

## BACKGROUND

Pancreatic cancer, a clinically common digestive system tumor with extremely high malignancy, exhibits a five-year survival rate of not greater than 10%, making it the second leading cause of cancer-related deaths. The lack of sensitive and specific markers for the occurrence and development of pancreatic cancer results in difficulty in early diagnosis, rapid disease progression, and poor prognosis. Since pancreatic cancer develops aggressively, which is characterized by local growth into surrounding nerves and blood vessels and early distant metastasis, most patients are unable to receive complete resection surgery. Current clinical treatment primarily employs comprehensive approaches including radiotherapy, interventional therapy, supportive care, and immunotherapy to alleviate patient symptoms and prolong survival. The first-line drug currently used clinically for treating pancreatic cancer is gemcitabine. While gemcitabine demonstrates significant anti-pancreatic cancer efficacy, gemcitabine generally induces adverse effects such as bone marrow suppression, skin rash, gastrointestinal side effects, and drug resistance while inhibiting tumor growth and alleviating the condition. Furthermore, the side effects inflict systemic damage on the human body. Therefore, there is an urgent clinical need to develop anti-pancreatic cancer drugs that are highly effective and have minimal side effects.

## SUMMARY

An object of the present disclosure is to provide a glycan and a preparation method and use thereof, and an antitumor drug. In the present disclosure, the glycan exhibits a desirable antitumor activity, especially an anti-pancreatic cancer activity, as well as satisfactory safety.

To achieve the above object, the present disclosure provides the following technical solutions:
The present disclosure provides a glycan having a structure shown in Formula I:

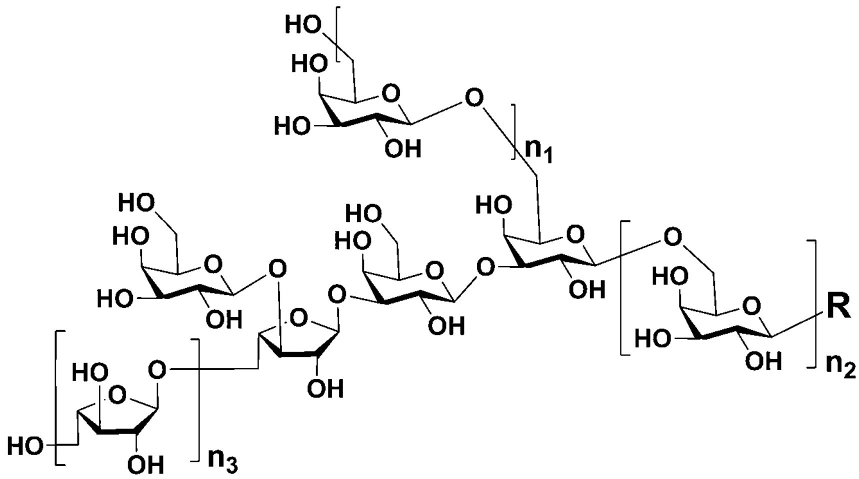

Formula I,

where in the Formula I, $n_1$ is 0 to 6, $n_2$ is 0 to 2, $n_3$ is 0 to 3, and R is C1-C10 alkoxy.

In some embodiments, $n_1$ is 1 to 5.

In some embodiments, $n_1$ is 2 to 4.

In some embodiments, $n_2$ is 0 to 1.

In some embodiments, $n_3$ is 1 to 3.

In some embodiments, $n_3$ is 2 to 3.

In some embodiments, R is selected from the group consisting of methoxy, ethoxy, propoxy, butoxy, pentoxy, hexoxy, heptoxy, octoxy, nonoxy, and decoxy.

In some embodiments, R is C5-C10 alkoxy.

In some embodiments, R is linear alkoxy.

In some embodiments, $n_1$ is 3, $n_2$ is 0, $n_3$ is 3, and R is n-octoxy.

The present disclosure further provides a method for preparing the glycan as described above, including the following steps:

mixing a compound 1-3, silver trifluoromethanesulfonate, p-toluenesulfenyl chloride, and an organic solvent, and conducting a first activation to obtain a first activation product system;
mixing the first activation product system and a compound 1-4, and conducting a first glycosylation coupling to obtain a first coupling product system;
mixing the first coupling product system, the silver trifluoromethanesulfonate, and the p-toluenesulfenyl chloride, and conducting a second activation to obtain a second activation product system;
mixing the second activation product system and a compound 1-5, and conducting a second glycosylation coupling to obtain a compound 1-2; and
subjecting the compound 1-2 to debenzoylation and debenzylation-debenzalization in sequence to obtain the glycan having the structure shown in the Formula I,
where the compound 1-3, the compound 1-4, the compound 1-5, and the compound 1-2 have the following structural formulas, respectively:

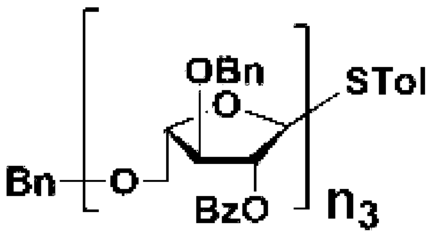

1-3 ,

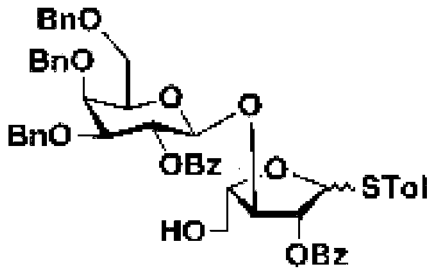

1-4 ,

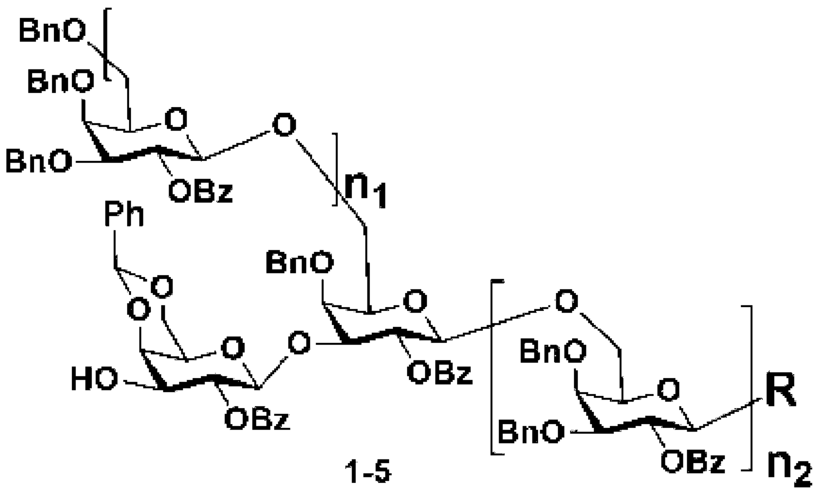

1-5 ,

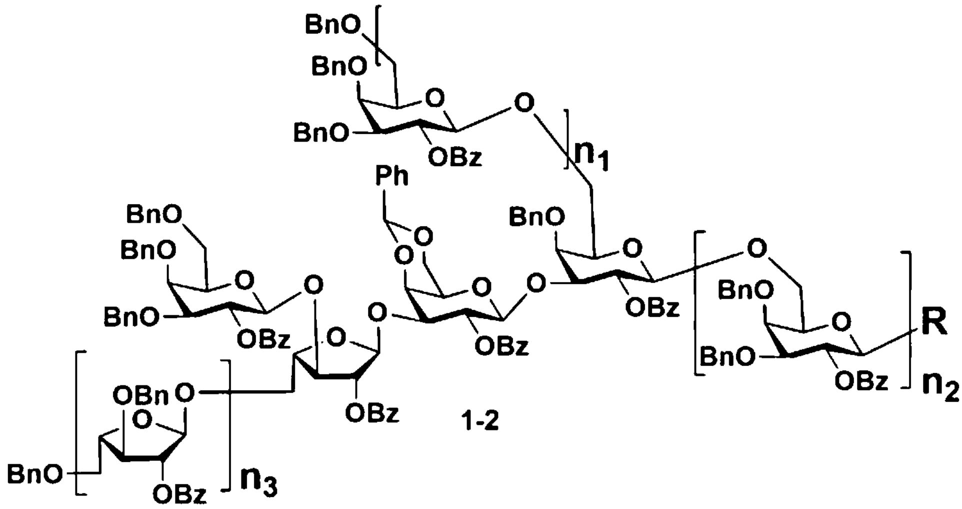

1-2

,

where $n_1$, $n_2$, $n_3$, and R are as defined in the Formula I.

In some embodiments, a molar ratio of the compound 1-3, the silver trifluoromethanesulfonate, and the p-toluenesulfenyl chloride is in a range of 1 : 1.2-5 : 1.

In some embodiments, the first activation is conducted at a temperature of -60°C to -78°C for 2 min to 10 min.

In some embodiments, a molar ratio of the compound 1-3 to the compound 1-4 is in a range of 1 : 0.85 to 1 : 0.92.

In some embodiments, the first glycosylation coupling includes conducting a first stage reaction and a second stage reaction in sequence; where the first stage reaction is conducted at a temperature of -60°C to -78°C for 5 min to 15 min; and after completion of the first stage reaction, an obtained reaction product is subjected to natural heating from the temperature for the first stage reaction to conduct the second stage reaction, and the second stage reaction is conducted for 1.5 h to 2.5 h.

In some embodiments, a molar ratio of the compound 1-4, the silver trifluoromethanesulfonate, and the p-toluenesulfenyl chloride is in a range of 1 : 1.2-5 : 1.

In some embodiments, the second activation is conducted at a temperature of -60°C to -78°C for 2 min to 10 min.

In some embodiments, a molar ratio of the compound 1-4 to the compound 1-5 is in a range of 0.8-0.85 : 1-2.

In some embodiments, the second glycosylation coupling includes conducting a first stage reaction and a second stage reaction in sequence; where the first stage reaction is conducted at a temperature of -60°C to -78°C for 5 min to 15 min; and after completion of the first stage reaction, an obtained reaction product is subjected to natural heating from the temperature of the first stage reaction to conduct the second stage reaction, and the second stage reaction is conducted for 1.5 h to 2.5 h.

In some embodiments, subjecting the compound 1-2 to the debenzoylation and the debenzylation-debenzalization in sequence includes:

mixing the compound 1-2 and an organic solvent, and conducting the debenzoylation in a basic environment to obtain a debenzoylated compound; and
mixing the debenzoylated compound, a palladium-on-carbon catalyst, an organic solvent, and water, and conducting the debenzylation-debenzalization in a hydrogen atmosphere.

In some embodiments, the basic environment has a pH value of 9 to 10; and a basic reagent providing the basic environment is a solution of sodium methoxide in methanol.

In some embodiments, the debenzoylation is conducted at a temperature of 10°C to 30°C for 5 h to 24 h.

In some embodiments, the debenzylation-debenzalization is conducted at a temperature of 10°C to 30°C under a hydrogen pressure of 0.3 MPa to 10 MPa for 5 h to 24 h.

The present disclosure further provides use of the glycan as described above in preparation of an antitumor drug.

In some embodiments, a tumor is selected from the group consisting of pancreatic cancer, gastric cancer, liver cancer, and cervical cancer.

In some embodiments, the antitumor drug includes the glycan and a pharmaceutically acceptable carrier, and the glycan in the antitumor drug has a mass fraction of 0.1% to 99.9%.

In some embodiments, a dosage form of the antitumor drug is selected from the group consisting of an injection and an oral tablet.

In some embodiments, an administration route of the antitumor drug is selected from the group consisting of oral administration, intravenous injection, and subcutaneous injection.

The present disclosure further provides an antitumor drug, including the glycan as described above and a pharmaceutically acceptable carrier.

In some embodiments, the glycan in the antitumor drug has a mass fraction of 0.1% to 99.9%.

The present disclosure provides a glycan having a structure shown in the Formula I. In the present disclosure, the glycan exhibits a desirable antitumor activity, especially an anti-pancreatic cancer activity, as well as satisfactory safety. Results from test examples indicate that the glycan exhibits a significant inhibitory effect on the proliferation of pancreatic cancer cells. Also, the glycan has no cytotoxicity on the growth of hepatocytes, demonstrating a superior safety profile compared with the positive control drug gemcitabine. Additionally, pancreatic cancer cells are sensitive to the glycan, thereby overcoming the issue of certain resistance that pancreatic cancer cells exhibit towards gemcitabine.

## BRIEF DESCRIPTION OF THE DRAWINGS

FIG. 1 shows the chemical structures of the glycans in embodiments of the present disclosure;
FIG. 2 shows the preliminary evaluation results of the anti-pancreatic cancer activity of various compounds; and
FIG. 3 shows the effect of the compound 2-34 on Panc-1 cell apoptosis and cell cycle.

## DETAILED DESCRIPTION OF THE EMBODIMENTS

The present disclosure provides a glycan having a structure shown in Formula I:

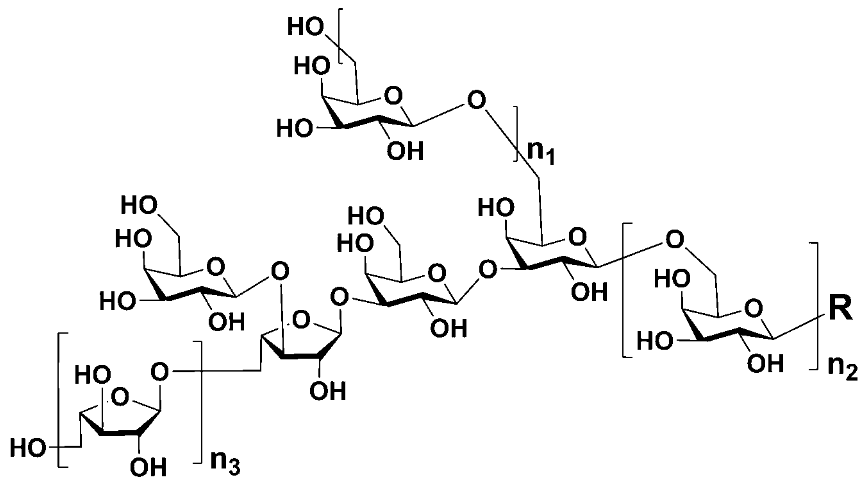

Formula I,

where in the Formula I, $n_1$ is 0 to 6, $n_2$ is 0 to 2, $n_3$ is 0 to 3, and R is C1-C10 alkoxy.

In the present disclosure, in the Formula I, $n_1$ is 0 to 6, $n_2$ is 0 to 2, and $n_3$ is 0 to 3. In some embodiments, $n_1$ is 0, 1, 2, 3, 4, 5, or 6; $n_2$ is 0, 1, or 2; and $n_3$ is 0, 1, 2, or 3. In some embodiments, $n_1$ is 1 to 5, $n_2$ is 0 to 1, and $n_3$ is 1 to 3; more preferably, $n_1$ is 2 to 4, $n_2$ is 0 to 1, and $n_3$ is 2 to 3.

In the present disclosure, R is C1-C10 alkoxy. In some embodiments, R is C5-C10 alkoxy. In some embodiments, R is methoxy, ethoxy, propoxy, butoxy, pentoxy, hexoxy, heptoxy, octoxy, nonoxy, or decoxy. In some embodiments, R is linear alkoxy.

In some embodiments of the present disclosure, $n_1$ is 3, $n_2$ is 0, $n_3$ is 3, and R is n-octoxy.

The present disclosure further provides a method for preparing the glycan, including the following steps:

mixing a compound 1-3, silver trifluoromethanesulfonate, p-toluenesulfenyl chloride, and an organic solvent, and conducting a first activation to obtain a first activation product system;
mixing the first activation product system and a compound 1-4, and conducting a first glycosylation coupling to obtain a first coupling product system;
mixing the first coupling product system, the silver trifluoromethanesulfonate, and the p-toluenesulfenyl chloride, and conducting a second activation to obtain a second activation product system;
mixing the second activation product system and a compound 1-5, and conducting a second glycosylation coupling to

obtain a compound 1-2; and

subjecting the compound 1-2 to debenzoylation and debenzylation-debenzalization in sequence to obtain the glycan having the structure shown in the Formula I,

where the compound 1-3, the compound 1-4, the compound 1-5, and the compound 1-2 have the following structural formulas, respectively:

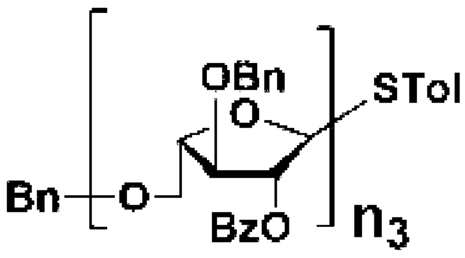

1-3

,

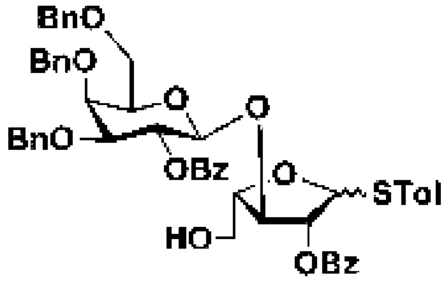

1-4

,

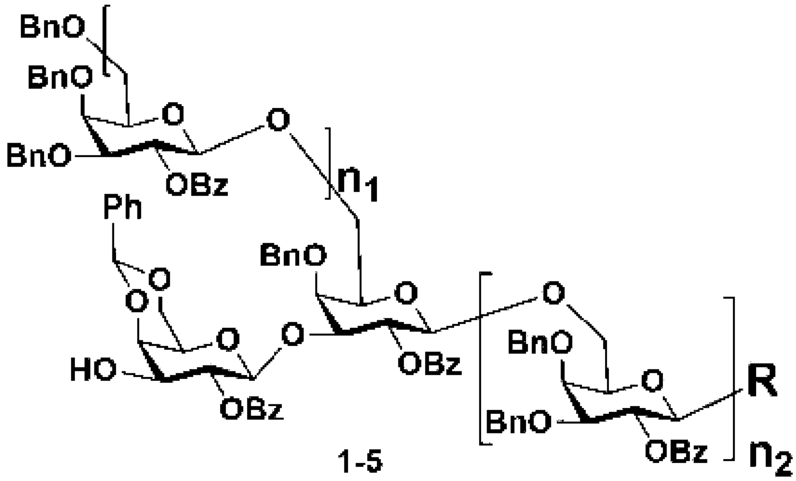

1-5

,

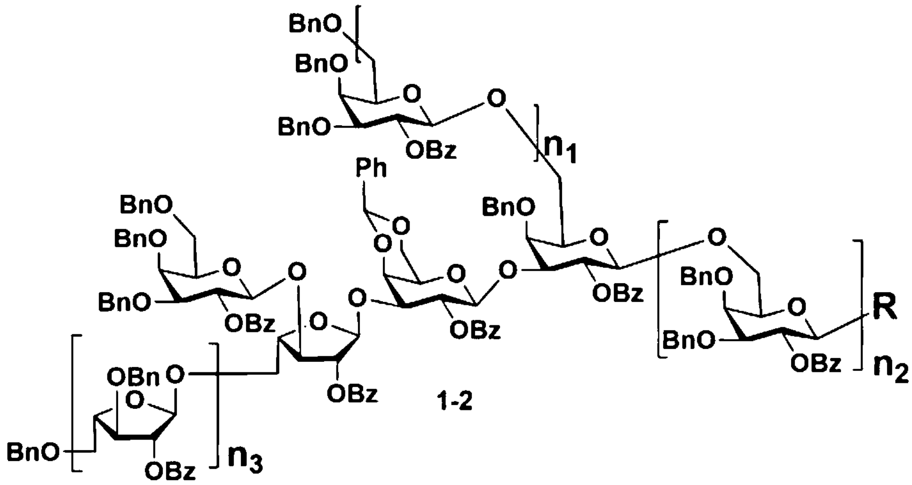

1-2

,

where $n_1$, $n_2$, $n_3$, and R are as defined in the Formula I.

In the present disclosure, unless otherwise specified, the raw materials used are all commercially-available commodities well known to those skilled in the art or prepared by methods well known to those skilled in the art.

In the present disclosure, a compound 1-3, silver trifluoromethanesulfonate, *p*-toluenesulfenyl chloride, and an

organic solvent are mixed and subjected to a first activation to obtain a first activation product system. In some embodiments, a molar ratio of the compound 1-3, the silver trifluoromethanesulfonate (AgOnf, and the *p*-toluenesulfenyl chloride (*p*-TolSCl) is in a range of 1 : (1.2-5) : 1, and more preferably 1 : 2 : 1. The silver trifluoromethanesulfonate and *p*-toluenesulfenyl chloride serve as activators. In some embodiments, the organic solvent is dichloromethane (DCM). There are no specific limitations on a dosage of the organic solvent, as long as the reaction could be proceeded smoothly. In some embodiments, the first activation is conducted in the presence of a 4Å molecular sieve; and the 4Å molecular sieve is activated by baking under vacuum by using a blowtorch and then cooled to room temperature before use. In some embodiments, the compound 1-3, the 4Å molecular sieve, and the organic solvent are mixed, then a resulting mixture is cooled to a temperature required for the first activation, and then silver trifluoromethanesulfonate and p-toluenesulfenyl chloride are added thereto, and the first activation is conducted. In some embodiments, the first activation is conducted at a temperature of -60°C to -78°C, and more preferably -75°C to -78°C for 2 min to 10 min, and more preferably 5 min.

In the present disclosure, after the first activation is completed, no post-treatment is required, and the first activation product system is directly mixed with a compound 1-4, and a first glycosylation coupling is conducted to obtain a first coupling product system. In some embodiments, based on a dosage of compound 1-3, a molar ratio of the compound 1-3 to the compound 1-4 is in a range of 1 : (0.85-0.92), and more preferably 1 : 0.9. In some embodiments, the compound 1-4 is dissolved in an organic solvent and then added dropwise into the first activation product system, and the first glycosylation coupling is conducted. In some embodiments, the first glycosylation coupling includes conducting a first stage reaction and a second stage reaction in sequence; the first stage reaction is conducted at a temperature of -60°C to -78°C, more preferably -75°C to -78°C for 5 min to 15 min, more preferably 10 min; an obtained reaction mixture is subjected to natural heating from the temperature for the first stage reaction to conduct the second stage reaction for 1.5 h to 2.5 h, more preferably 2 h. In some embodiments, conducting the reactions initially at a lower temperature helps avoid decomposition of a reactive intermediate caused by temperature increase. In some embodiments, after the compound 1-4 is added, a resulting mixture is stirred at -78°C for 10 min and then is reacted for 2 h by natural heating.

In the present disclosure, after the first glycosylation coupling is completed, no post-treatment is required, the first coupling product system is directly mixed with the silver trifluoromethanesulfonate and the *p*-toluenesulfenyl chloride, and a second activation is conducted to obtain a second activation product system. In some embodiments, based on a dosage of the compound 1-4, a molar ratio of the compound 1-4, the silver trifluoromethanesulfonate, and the *p*-toluenesulfenyl chloride is in a range of 1 : (1.2-5) : 1, and more preferably 1 : 2 : 1. In some embodiments, the first coupling product system is heated to room temperature and stirred for 20 min to 30 min, then cooled to a temperature required for the second activation, and then silver trifluoromethanesulfonate and p-toluenesulfenyl chloride are added thereto, and the second activation is conducted. In some embodiments, the conditions for the second activation are consistent with those for the first activation and will not be detailed again here.

In the present disclosure, after the second activation is completed, no post-treatment is required, and the second activation product system is directly mixed with a compound 1-5, and a second glycosylation coupling is conducted to obtain a compound 1-2. In some embodiments, based on the dosage of the compound 1-4, a molar ratio of the compound 1-4 to the compound 1-5 is in a range of (0.8-0.85) : (1-2), and more preferably 0.83 : 1.05. In some embodiments, the compound 1-5 is dissolved in an organic solvent and then added dropwise into the second activation product system, and the second glycosylation coupling is conducted. In some embodiments, the conditions for the second glycosylation coupling are consistent with those for the first glycosylation coupling and will not be detailed again here. In some embodiments, after the second glycosylation coupling reaction is completed, triethylamine (TEA) is added to quench the reaction, a resulting reaction solution is filtered through diatomaceous earth, and a resulting filtrate is concentrated and then separated by FLASH (fast low-pressure liquid chromatography) rapid preparative chromatography to obtain the compound 1-2. In some embodiments, taking the preparation of compound 2-82 as an example, a mobile phase used for the FLASH rapid preparative chromatography is a mixture of petroleum ether, ethyl acetate, and DCM, where a volume fraction of ethyl acetate is 40%, and a volume fraction of DCM is 15%.

In the present disclosure, after the compound 1-2 is obtained, the compound 1-2 is subjected to debenzoylation (Bz) and debenzylation (Bn)-debenzalization in sequence to obtain the glycan having the structure shown in the Formula I. In some embodiments, the compound 1-2 is mixed with an organic solvent, and the debenzoylation is conducted under a basic environment to obtain a debenzoylated compound; and the debenzoylated compound, a palladium-on-carbon catalyst, an organic solvent, and water are mixed and subjected to the debenzylation-debenzalization in a hydrogen atmosphere to obtain the glycan having the structure shown in the Formula I. Detailed descriptions are provided below.

In some embodiments of the present disclosure, the organic solvent used for the debenzoylation is DCM and methanol, where a volume ratio of DCM to methanol is in a range of (1.5-3) : 1, and more preferably 2 : 1. There are no specific limitations on the dosage of the organic solvent, as long as the reaction could be proceeded smoothly. In some embodiments, the basic environment has a pH value of 9 to 10; a basic reagent providing the basic environment is a solution of sodium methoxide in methanol, and a concentration of the basic reagent is 30 wt%. In some embodiments, the debenzoylation is conducted at a temperature of 10°C to 30°C, more preferably room temperature for 5 h to 24 h, and more preferably 9 h. In some embodiments, after the debenzoylation is completed, a cation exchange resin is added to a

resulting product system to neutralize the pH value to 7; and the cation exchange resin is removed by filtration, and a resulting filtrate is concentrated and then separated by polystyrene gel exclusion chromatography (an eluent used is ethyl acetate) to obtain the debenzoylated compound.

In some embodiments of the present disclosure, the organic solvent used for the debenzylation-debenzalization is a mixture of ethyl acetate and methanol, where a volume ratio of ethyl acetate to methanol is in a range of 1 : (1-3), and more preferably 1 : 2; a volume ratio of the organic solvent to water is in a range of (2-6) : 1, and more preferably 3 : 1. There are no specific limitations on the total dosage of the organic solvent and water, as long as the reaction could be proceeded smoothly. In some embodiments, a mass content of palladium in the palladium-on-carbon catalyst is 10% (denoted as 10% Pd/C). There are no specific limitations on the dosage of the palladium-on-carbon catalyst, as long as the reaction could be proceeded smoothly. In some embodiments, the debenzylation-debenzalization is conducted at a temperature of 10°C to 30°C, more preferably room temperature under a hydrogen pressure of 0.3 MPa to 10 MPa, and more preferably 0.4 MPa for 5 h to 24 h, and more preferably 9 h. In some embodiments, after the debenzylation-debenzalization is completed, a resulting product is filtered through a microporous filtration membrane to remove the palladium-on-carbon catalyst, and a resulting filtrate is concentrated and then separated by dextran gel LH-20 exclusion chromatography (an eluent used is a mixture of methanol and water in a volume ratio of 1 : 1) to obtain the glycan having the structure shown in the Formula I (FIG. 1).

It should be noted that, in the present disclosure, under a condition that $n_3$ in the Formula I is 0, a compound 1-4-2 (where a hydroxyl group at position 5 of arabinose in the compound 1-4 is replaced by a benzyloxy group, OBn) and the compound 1-5 are primarily used as reactants. The compound 1-2 is prepared by referring to the above technical solutions, and then the glycan having the structure shown in the Formula I is prepared:

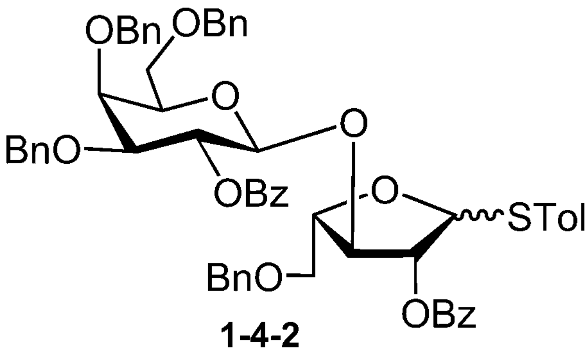

1-4-2

,

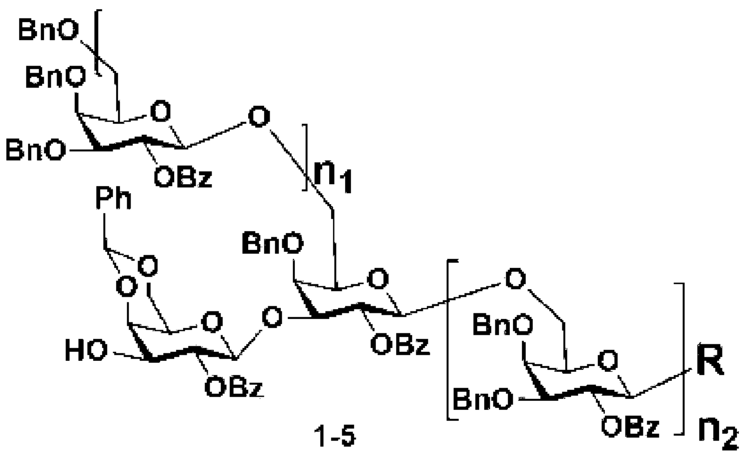

1-5

,

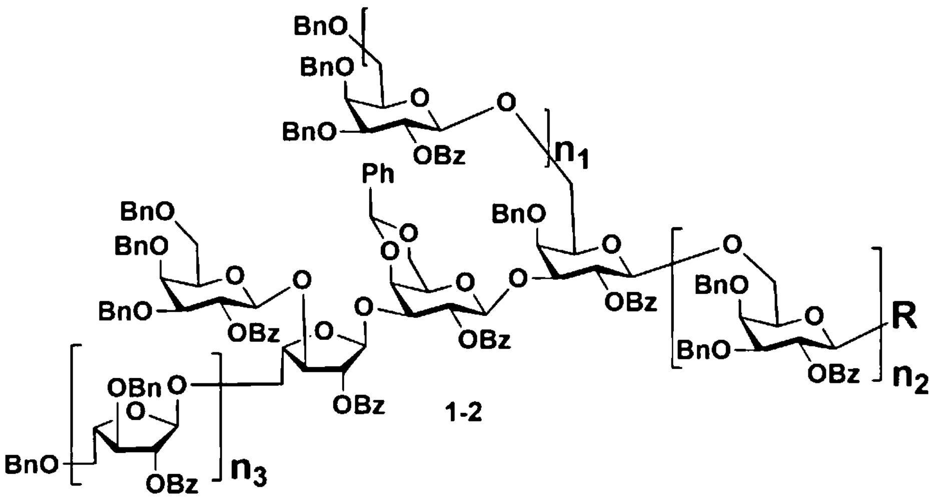

1-2

.

The methods for preparing the reactants required to synthesize the glycan having the structure shown in the Formula I are described below.

In the present disclosure, $n_3$ in the compound 1-3 ranges from 0 to 3, which is specifically categorized as follows: Under a condition that $n_3$ is 0, the compound 1-3 is considered non-existent (i.e., compound 1-3 is not required for preparing the glycan having the structure shown in the Formula I).

Under a condition that $n_3$ is 1, the compound 1-3 is specifically compound 1-6.

Under a condition that $n_3$ is 2 or 3, a process for preparing the compound 1-3 includes the following steps: subjecting the compound 1-6 to activation in the presence of an activator, and subjecting a resulting product to glycosylation coupling with a compound 1-7 to obtain a disaccharide compound (i.e., $n_3$=2); and subjecting the disaccharide compound to activation in the presence of an activator, and subjecting a resulting material to glycosylation coupling with the compound 1-7 to obtain a disaccharide compound (i.e., $n_3$=3). The structural formulas of compound 1-6 and compound 1-7 are as follows:

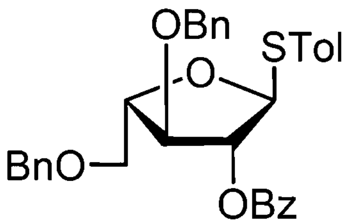

**1-6**

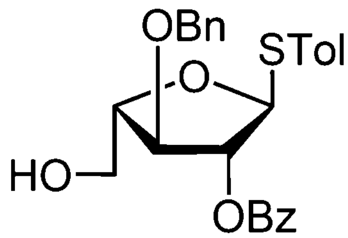

**1-7**

.

In some embodiments of the present disclosure, the reagents and reaction conditions required for the activation and glycosylation coupling involved in preparing the compound 1-3 are consistent with those described in the foregoing technical solutions and will not be detailed again here.

In some embodiments of the present disclosure, a reaction scheme for preparing compound 1-6 is as follows:

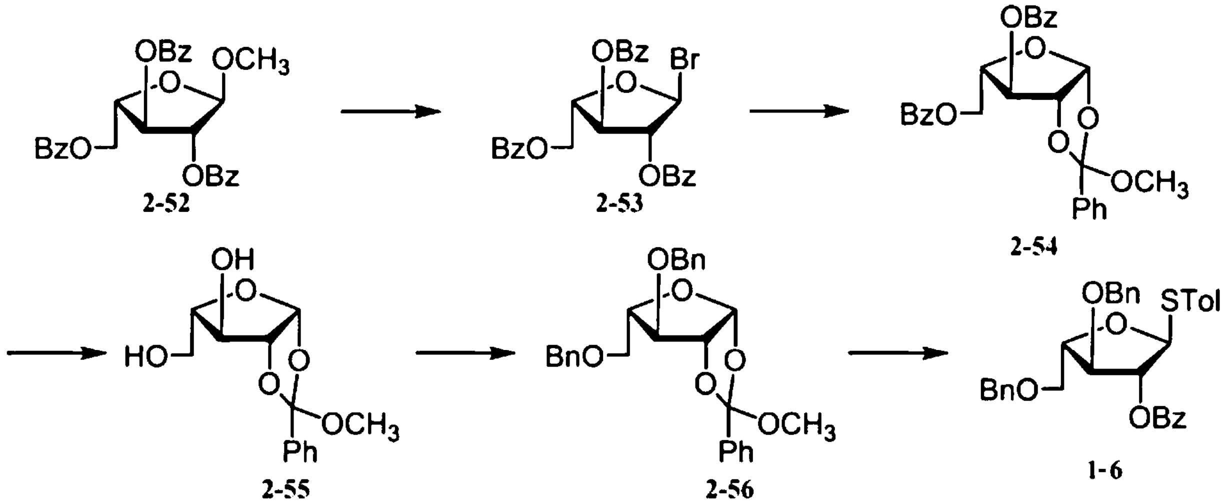

2-52 2-53 2-54 2-55 2-56 1-6

In some embodiments of the present disclosure, a compound 2-52 is used as a starting material, and its hydroxyl groups at 2, 3, and 5 positions are protected by benzoylation, and the methyl glycoside is converted to bromo-sugar, thereby obtaining the compound 2-53. Under the action of 2,6-lutidine, an obtained compound (the compound 2-53) is reacted with methanol to form an orthoester structure, locking 1 and 2 positions to obtain a compound 2-54. Benzoyl groups of an obtained compound (the compound 2-54) at 3 and 5 positions are removed under basic conditions to obtain a compound 2-55, and the compound 2-55 is subjected to benzyl protection at the 3 and 5 positions by using sodium hydride (NaH) to obtain a compound 2-56. Detailed descriptions are provided below. In some embodiments, a process for preparing the compound 2-56 includes the following steps:

mixing the compound 2-52, acetyl bromide, methanol, and DCM, and conducting bromination to obtain a mixture containing the compound 2-53;
mixing the mixture containing the compound 2-53, 2,6-lutidine, and methanol, and conducting orthoester formation to obtain the compound 2-54;
mixing the compound 2-54 and methanol, and conducting debenzoylation under a basic environment to obtain the compound 2-55; and
mixing the compound 2-55, benzyl bromide, sodium hydride, and N,N-dimethylformamide (DMF), and conducting benzyl protection to obtain the compound 2-56.

In some embodiments of the present disclosure, the compound 2-52, acetyl bromide, methanol, and DCM are mixed and subjected to bromination to obtain the mixture containing the compound 2-53. In some embodiments, a molar ratio of the compound 2-52, the acetyl bromide, and the methanol is in a range of 1 : (1.5-3) : (1.5-3), and more preferably 1 : 3 : 2.7. In some embodiments, compound 2-52 is mixed with DCM, acetyl bromide is added thereto under an ice bath condition, and then methanol is dropwise added thereto. In some embodiments, the bromination is conducted at a temperature of 20°C to 40°C, more preferably room temperature for 2 h to 5 h, more preferably 3 h, where the bromination is initiated after the completion of the methanol addition.

In some embodiments of the present disclosure, after the bromination is completed, no post-treatment is required, and the mixture containing the compound 2-53 is directly mixed with 2,6-lutidine and methanol, and orthoester formation is conducted to obtain the compound 2-54. In some embodiments, based on a dosage of compound 2-52, a molar ratio of the compound 2-52, the 2,6-lutidine, and the methanol is in a range of 1 : (3-8) : (5-20), and more preferably 1 : 5 : 10. In some embodiments, 2,6-lutidine and methanol are added to the mixture containing the compound 2-53 under an ice bath condition. In some embodiments, the orthoester formation is conducted at a temperature of 10°C to 35°C, more preferably room temperature for 15 h to 40 h, more preferably 24 h. In some embodiments, after the orthoester formation is completed, a resulting reaction material is diluted with DCM, washed with water and then saturated NaCl solution, dried over anhydrous $Na_2SO_4$, filtered, and a resulting filtrate is concentrated; and a resulting concentrate is subjected to oil pump vacuum for 50 min to 60 min to obtain a pale yellow oil, which is the compound 2-54.

In some embodiments of the present disclosure, after obtaining the compound 2-54, the compound 2-54 and methanol are mixed and subjected to the debenzoylation under the basic environment to obtain the compound 2-55. In some embodiments, the basic environment has a pH value of 9; a basic reagent providing the basic environment is a solution of sodium methoxide in methanol, with a concentration of 30 wt%. In some embodiments, the debenzoylation is conducted at a temperature of 20°C to 40°C, more preferably room temperature for 8 h to 24 h, more preferably 12 h. In some embodiments, after the debenzoylation is completed, a cation exchange resin is added to a resulting product system

to neutralize the pH value to 7; and the cation exchange resin is removed by filtration, and a resulting filtrate is concentrated and then purified by column chromatography (an eluent used is a mixture of petroleum ether, acetone, and TEA (triethylamine, also known as $Et_3N$), where a volume ratio of petroleum ether to acetone is 6 : 1, and a volume fraction of $Et_3N$ is 0.2%) to obtain a pale yellow oil, which is the compound 2-55.

In some embodiments of the present disclosure, after obtaining the compound 2-55, the compound 2-55, benzyl bromide, sodium hydride, and DMF are mixed and subjected to benzyl protection to obtain the compound 2-56. In some embodiments, based on the dosage of the compound 2-52, a molar ratio of the compound 2-52, the benzyl bromide, and the sodium hydride is in a range of 1 : (2-8) : (2-8), and more preferably 1 : 4 : 4; and the sodium hydride is used in a form of a mineral oil solution, with a mass fraction of 60%. In some embodiments, the compound 2-55 is dissolved in DMF, benzyl bromide is added thereto under an ice bath condition, and then a solution of sodium hydride in mineral oil is added thereto in batches. In some embodiments, the benzyl protection includes conducting a first stage reaction and a second stage reaction in sequence; where the first stage reaction is conducted at a temperature of -20°C to 10°C, more preferably 0°C for 1 h to 4 h, more preferably 1 h to 2 h; and the second stage reaction is conducted at a temperature of 20°C to 40°C, more preferably room temperature for 8 h to 24 h, more preferably 12 h. In some embodiments, after the benzyl protection is completed, the reaction is quenched by adding saturated ammonium chloride solution; and a resulting reaction material is concentrated, diluted with DCM, washed with water and then saturated NaCl solution, dried over anhydrous $Na_2SO_4$, filtered, and a resulting filtrate is concentrated and then purified by column chromatography (an eluent used is a mixture of petroleum ether, ethyl acetate, and $Et_3N$, where a volume ratio of petroleum ether to ethyl acetate is 8 : 1, and a volume fraction of $Et_3N$ is 0.2%) to obtain a pale yellow oil, which is the compound 2-56.

In some embodiments of the present disclosure, the compound 2-56 is subjected to ring-opening of an orthoester ring under the action of Lewis acid $SnCl_4$, and electrophilic addition with p-toluenethiol is conducted to ultimately obtain the compound 1-6. In some embodiments, a process for preparing the compound 1-6 includes:
mixing the compound 2-26, p-toluenethiol, $SnCl_4$, and DCM and conducting ring-opening-(and) electrophilic addition to obtain the compound 1-6.

In some embodiments of the present disclosure, a molar ratio of the compound 2-26, the p-thiocresol, and the $SnCl_4$ is in a range of 1 : (2-10) : (0.1-0.2), and more preferably 1 : 2 : 0.1. In some embodiments, the ring-opening-electrophilic addition is conducted in the presence of a 4Å molecular sieve; and the 4Å molecular sieve is activated by baking under vacuum by using a blowtorch and then cooled to room temperature before use. In some embodiments, the 4Å molecular sieve, compound 2-26, and p-toluenethiol are stirred and mixed at room temperature for 10 min to 20 min, then cooled to a temperature required for the ring-opening-electrophilic addition, and $SnCl_4$ is dropwise added thereto in batches. In some embodiments, the ring-opening-electrophilic addition is conducted at a temperature of -10°C to 0°C, more preferably -5°C for 4 h to 12 h, more preferably 10 h to 12 h, where the ring-opening-electrophilic addition is initiated after the completion of the $SnCl_4$ addition. In some embodiments, after the ring-opening-electrophilic addition is completed, TEA is added to quench the reaction; and a resulting reaction material is filtered through diatomaceous earth to remove the molecular sieve, and a resulting filtrate is concentrated and then purified by column chromatography (an eluent used is a mixture of petroleum ether and ethyl acetate in a volume ratio of 20 : 1) to obtain a colorless, transparent oily liquid, which is the compound 1-6.

In some embodiments of the present disclosure, a reaction scheme for preparing the compound 1-7 is as follows:

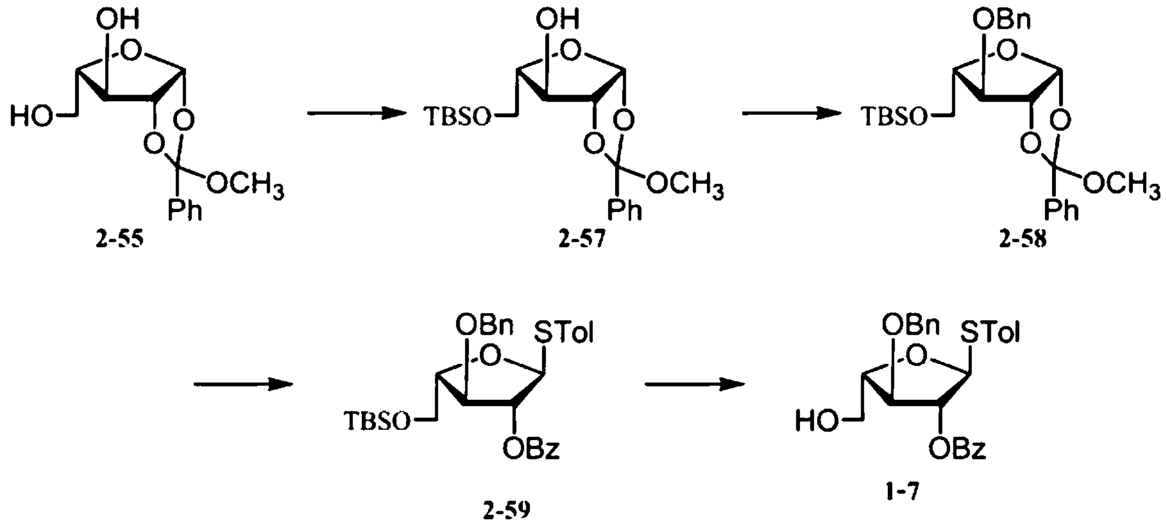

2-55 2-57 2-58

2-59 1-7

In some embodiments of the present disclosure, starting from the key intermediate compound 2-55, and due to

the presence of an orthoester ring, acidic conditions are avoided as much as possible to prevent potential opening of the orthoester ring. Therefore, a TBS (tert-butyldimethylsilyl) protecting group is selectively introduced at position 5 under basic conditions to obtain a compound 2-57. Then, benzyl protection is conducted on the exposed hydroxyl group at position 3 to obtain a compound 2-58. The orthoester ring is opened under the action of Lewis acid $SnCl_4$, and electrophilic addition with p-toluenethiol is conducted to obtain a compound 2-59. Finally, the TBS protecting group is rapidly removed by reaction with Lewis acid boron trifluoride diethyl etherate in acetonitrile as a solvent, thereby obtaining the compound 1-7. Detailed descriptions are provided below.

In some embodiments of the present disclosure, a process for preparing the compound 2-57 includes: mixing the compound 2-55, TEA, 4-dimethylaminopyridine (DMAP), tert-butyldimethylsilyl chloride (TBSCl), and pyridine and conducting TBS protection to obtain the compound 2-57.

In some embodiments of the present disclosure, a molar ratio of the compound 2-55, the TEA, the DMAP, and the TBSCl is in a range of 1 : (2-10) : (0-1) : (1-1.1), and more preferably 1 : 3.5 : 0.01 : 1.1. In some embodiments, the compound 2-55 is dissolved in pyridine, cooled to -5°C, and TEA and then DMAP are added thereto, and then TBSCl is added thereto in batches. In some embodiments, the TBS protection is conducted at a temperature of 0°C to 20°C, more preferably 5°C for 8 h to 24 h, more preferably 12 h. In some embodiments, after the TBS protection is completed, a resulting reaction material is concentrated and then purified by column chromatography (an eluent used is a mixture of petroleum ether, acetone, and $Et_3N$, where a volume ratio of petroleum ether to acetone is 6 : 1, and a volume fraction of $Et_3N$ is 0.2%) to obtain an oily substance, which is the compound 2-57.

In some embodiments of the present disclosure, a process for preparing the compound 2-58 includes: mixing the compound 2-57, benzyl bromide, sodium hydride, and DMF and conducting benzyl protection to obtain the compound 2-58.

In some embodiments of the present disclosure, a molar ratio of the compound 2-57, the benzyl bromide, the sodium hydride is in a range of 1 : (1-2) : (1-2), and more preferably 1 : 1.5 : 1.3; and the sodium hydride is used in a form of a mineral oil solution, with a mass fraction of 60%. In some embodiments, the compound 2-57 is dissolved in DMF, benzyl bromide is added thereto under an ice bath condition, and then a solution of sodium hydride in mineral oil is added thereto in batches. In some embodiments, the benzyl protection includes conducting a first stage reaction and a second stage reaction in sequence; where the first stage reaction is conducted at a temperature of 0°C to 10°C, more preferably 0°C to 5°C for 0.5 h to 2 h, more preferably 30 min to 50 min; and the second stage reaction is conducted at a temperature of 10°C to 30°C, more preferably room temperature for1 h to 20 h, more preferably 4 h. In some embodiments, after the benzyl protection is completed, the reaction is quenched by adding $H_2O$, a resulting reaction product is extracted with DCM, a resulting organic phase is washed with saturated NaCl solution, dried over anhydrous $Na_2SO_4$, filtered, and a resulting filtrate is concentrated and then purified by column chromatography (an eluent used is a mixture of petroleum ether, ethyl acetate, and $Et_3N$, where a volume ratio of petroleum ether to ethyl acetate is 20 : 1, and a volume fraction of $Et_3N$ is 0.2%) to obtain an oily substance, which is the compound 2-58.

In some embodiments of the present disclosure, a process for preparing the compound 1-7 includes:

mixing the compound 2-58, p-toluenethiol, $SnCl_4$, and DCM and conducting ring-opening-(and) electrophilic addition to obtain the compound 2-59; and
mixing the compound 2-59, boron trifluoride diethyl etherate, and acetonitrile and conducting de-TBS protection to obtain the compound 1-7.

In some embodiments of the present disclosure, the compound 2-58, p-toluenethiol, $SnCl_4$, and DCM are mixed and subjected to ring-opening-electrophilic addition to obtain the compound 2-59. In some embodiments, a molar ratio of the compound 2-58, the p-toluenethiol, the $SnCl_4$ is in a range of 1 : (1.5-5) : (0.1-0.3), and more preferably 1 : 2 : 0.1. In some embodiments, the ring-opening-electrophilic addition is conducted in the presence of a 4Å molecular sieve; and the 4Å molecular sieve is activated by baking under vacuum by using a blowtorch and then cooled to room temperature before use. In some embodiments, the 4Å molecular sieve, compound 2-26, and p-toluenethiol are stirred and mixed at room temperature for 10 min to 20 min, cooled to -5°C, and $SnCl_4$ is dropwise added thereto in batches. In some embodiments, the ring-opening-electrophilic addition is conducted at a temperature of -20°C to 0°C, more preferably -5°C for 10 h to 24 h, more preferably 12 h, where the ring-opening-electrophilic addition is initiated after the completion of the $SnCl_4$ addition. In some embodiments, after the ring-opening-electrophilic addition is completed, TEA is added to quench the reaction, and a resulting reaction material is filtered through diatomaceous earth to remove the molecular sieve, and a resulting filtrate is concentrated and then purified by column chromatography (an eluent used is a mixture of petroleum ether, ethyl acetate, and $Et_3N$, where a volume ratio of petroleum ether to ethyl acetate is 100 : 1, and a volume fraction of $Et_3N$ is 0.2%) to obtain a colorless, transparent oily liquid, which is the compound 2-59.

In some embodiments of the present disclosure, after obtaining the compound 2-59, the compound 2-59, boron trifluoride diethyl etherate, and acetonitrile are mixed and subjected to de-TBS protection to obtain the compound 1-7. In some embodiments, based on a dosage of the compound 2-58, a molar ratio of the compound 2-58 to the boron trifluoride

diethyl etherate is in a range of 1 : (1.1-2.0), and more preferably 1 : (1.1-1.5). In some embodiments, the compound 2-59 is dissolved in acetonitrile, and then boron trifluoride diethyl etherate is added dropwise under an ice bath condition. In some embodiments, the de-TBS protection is conducted at a temperature of -10°C to 10°C, more preferably 0°C for 2 h to 8 h, more preferably 4 h, where the de-TBS protection is initiated after the completion of the boron trifluoride diethyl etherate addition. In some embodiments, after the de-TBS protection is completed, TEA is added to quench the reaction; and a resulting reaction material is concentrated and then purified by column chromatography (an eluent used is a mixture of petroleum ether, ethyl acetate, and $Et_3N$, where a volume ratio of petroleum ether to ethyl acetate is 8 : 1, and a volume fraction of $Et_3N$ is 0.2%) to obtain a colorless, transparent oily liquid, which is the compound 1-7.

In some embodiments of the present disclosure, a reaction scheme for preparing the compound 1-4 (where a wavy line in the structural formula indicates a mixture of the α-configuration compound and the β-configuration compound) is as follows:

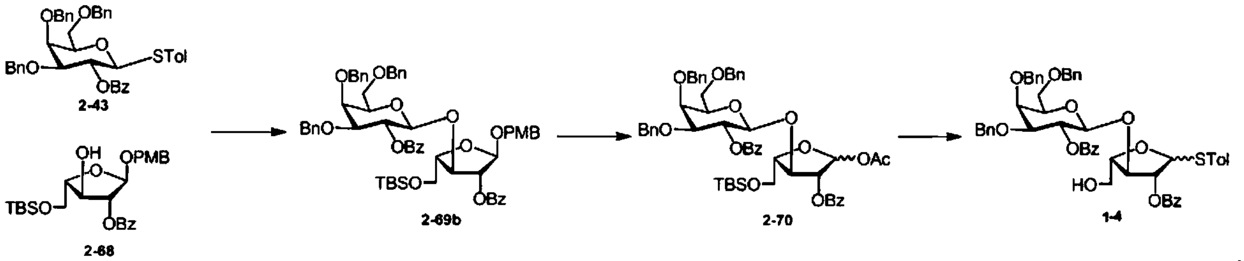
2-43
2-68
2-69b
2-70
1-4

.

In some embodiments of the present disclosure, a compound 2-68 and compound 2-43 are subjected to glycosylation coupling under a activity of an activator (p-TolSCl/AgOTf) to obtain a coupled disaccharide compound 2-69b. A PMB (p-methoxybenzyl) protecting group is then removed by using 2,3-dichloro-5,6-dicyano-1,4-benzoquinone (DDQ) to obtain a disaccharide hemiacetal (a de-PMB protected compound). Subsequently, acetylation protection is conducted by using acetic anhydride to obtain a compound 2-70. Finally, the acetyl group is successfully converted to a p-toluenesulfenyl by using $SnCl_4$ as a Lewis acid. Furthermore, under the reaction conditions, the TBS group at position 5 of the compound 2-70 is also removed, thereby eliminating the need for a subsequent de-TBS step and obtaining the compound 1-4. Detailed descriptions are provided below.

In some embodiments of the present disclosure, a process for preparing the compound 2-69b includes the following steps:

mixing the compound 2-43, 2,4,6-tri-tert-butylpyrimidine (TTBP), silver trifluoromethanesulfonate, p-toluenesulfenyl chloride, and DCM, and conducting activation to obtain an activation product system; and

mixing the activation product system and the compound 2-68, and conducting glycosylation coupling to obtain the compound 2-69b.

In some embodiments of the present disclosure, the compound 2-43, 2,4,6-tri-tert-butylpyrimidine (TTBP), silver trifluoromethanesulfonate, p-toluenesulfenyl chloride, and DCM are mixed and subjected to activation to obtain the activation product system. In some embodiments, a molar ratio of the compound 2-43, the TTBP, the silver trifluoromethanesulfonate, and the p-toluenesulfenyl chloride is in a range of 1 : (1.5-5) : (2-4) : (1-1.5), and more preferably 1 : 2 : 2.4 : 1.2. In some embodiments, the activation is conducted in the presence of a 4Å molecular sieve; and the 4Å molecular sieve is activated by baking under vacuum by using a blowtorch and then cooled to room temperature before use. In some embodiments, the compound 2-43, the 4Å molecular sieve, and DCM are mixed, then TTBP is added thereto, and a resulting mixture is stirred at room temperature for 15 min to 20 min, cooled to a temperature required for the activation, and silver trifluoromethanesulfonate and p-toluenesulfenyl chloride are added thereto, and the activation is conducted. In some embodiments, the activation is conducted at a temperature of -60°C to -80°C, more preferably -78°C for 5 min to 10 min, more preferably 5 min to 7 min.

In some embodiments of the present disclosure, after the activation is completed, no post-treatment is required; and the activation product system is directly mixed with the compound 2-68, and the glycosylation coupling is conducted to obtain the compound 2-69b. In some embodiments, the compound 2-68 is added dropwise into the activation product system. In some embodiments, the glycosylation coupling includes conducting a first stage reaction and a second stage reaction in sequence; where the first stage reaction is conducted at a temperature of -60°C to -78°C, more preferably from -75°C to -78°C for 15 min to 25 min, more preferably 20 min; and an obtained reaction mixture is subjected to natural heating from a temperature of the first stage reaction, and the second stage reaction is conducted for 1.5 h to 2.5 h, more preferably 2 h. In some embodiments, conducting the reactions initially at a lower temperature helps avoid decomposition of the reactive intermediate caused by temperature increase. In some embodiments, after the addition of compound 2-68,

a resulting mixture is stirred at -78°C for 10 min and then is reacted for 2 h by natural heating. In some embodiments, after the glycosylation coupling is completed, 0.2 mL of TEA is added to quench the reaction; and a resulting reaction material is filtered through diatomaceous earth to remove the molecular sieve, and a resulting filtrate is concentrated and then separated by FLASH rapid preparative chromatography (a mobile phase used is a mixture of petroleum ether and ethyl acetate, where a volume fraction of ethyl acetate is 40%) to obtain a pale yellow oil, which is the compound 2-69b.

In some embodiments of the present disclosure, a process for preparing the compound 1-4 includes the following steps:

mixing the compound 2-69b, DDQ, DCM, and water, and conducting de-PMB protection to obtain a de-PMB-protected compound;
mixing the de-PMB-protected compound, acetic anhydride, 4-dimethylaminopyridine, and pyridine, and conducting acetylation to obtain the compound 2-70; and
mixing the compound 2-70, p-toluenethiol, $SnCl_4$, and DCM, and conducting substitution-(and) de-TBS protection to obtain the compound 1-4.

In some embodiments of the present disclosure, the compound 2-69b, DDQ, DCM, and water are mixed and subjected to de-PMB protection to obtain the de-PMB-protected compound. In some embodiments, a molar ratio of compound 2-69b to DDQ is in a range of 1 : (1.2-2.5), and more preferably 1 : 2; a volume ratio of DCM to water is in a range of (15-30) : 1, and more preferably 19 : 1. In some embodiments, the compound 2-69b, DCM, and water are mixed, and then DDQ is added thereto in batches. In some embodiments, the de-PMB protection is conducted at a temperature of 10°C to 35°C, more preferably room temperature for 5 h to 24 h, more preferably 8 h. In some embodiments, after the de-PMB protection is completed, the resulting product is filtered through diatomaceous earth; and a resulting filtrate is diluted with DCM, washed with saturated $NaHCO_3$ solution and then saturated NaCl solution, a resulting organic phase is dried over anhydrous $Na_2SO_4$, filtered, and a resulting filtrate is concentrated to obtain the de-PMB-protected compound.

In some embodiments of the present disclosure, after obtaining the de-PMB-protected compound, the de-PMB-protected compound, acetic anhydride, 4-dimethylaminopyridine, and pyridine are mixed and subjected to acetylation to obtain the compound 2-70. In some embodiments, based on a dosage of the compound 2-69b, a molar ratio of the compound 2-69b, the acetic anhydride, and the 4-dimethylaminopyridine is in a range of 1 : (2-5) : (0.1-0.5), and more preferably 1 : 2 : 0.1. In some embodiments, the de-PMB-protected compound and pyridine are mixed, and then acetic anhydride and 4-dimethylaminopyridine are added thereto in sequence. In some embodiments, the acetylation is conducted at a temperature of 10°C to 35°C, more preferably room temperature for 2 h to 10 h, more preferably 4 h. In some embodiments, after the acetylation is completed, the pyridine is removed from a resulting product system by concentration under reduced pressure; and a resulting residue is diluted with DCM, washed with 1 mol/L of hydrochloric acid, saturated $NaHCO_3$ solution, and saturated NaCl solution in sequence, a resulting organic phase is dried over anhydrous $Na_2SO_4$, filtered, and a resulting filtrate is concentrated and then separated by FLASH rapid preparative chromatography (a mobile phase used is a mixture of petroleum ether and ethyl acetate, where a volume fraction of ethyl acetate is 30%) to obtain a transparent oily liquid, which is the compound 2-70.

In some embodiments of the present disclosure, after obtaining the compound 2-70, the compound 2-70, p-toluenethiol, $SnCl_4$, and DCM are mixed and subjected to substitution-de-TBS protection to obtain the compound 1-4. In some embodiments, based on the dosage of the compound 2-69b, a molar ratio of the compound 2-69b, the p-thiocresol, and the $SnCl_4$ is in a range of 1 : (1.2-2.0) : (0.3-0.5), and more preferably 1 : 1.2 : 0.4. In some embodiments, the compound 2-70 and DCM are mixed, p-toluenethiol is added thereto, and then the $SnCl_4$ is added dropwise to a resulting mixture at 0°C. In some embodiments, the substitution-de-TBS protection is conducted at a temperature of 0°C to 10°C, more preferably 0°C to 2°C for 10 min to 35 min, more preferably 25 min. In some embodiments, after the substitution-de-TBS protection is conducted, TEA is added to quench the reaction; and a resulting reaction material is concentrated and then separated by FLASH rapid preparative chromatography (a mobile phase used is a mixture of petroleum ether and ethyl acetate, where a volume fraction of ethyl acetate is 60%) to obtain a yellow oil, which is the compound 1-4.

In some embodiments of the present disclosure, a reaction scheme for preparing the compound 2-68 is as follows:

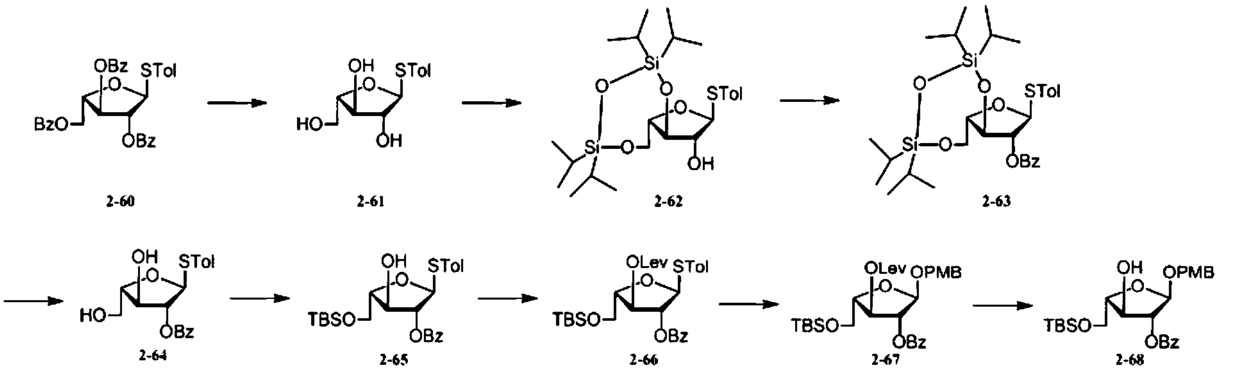

2-60 2-61 2-62 2-63

2-64 2-65 2-66 2-67 2-68

In some embodiments of the present disclosure, starting from a compound 2-60, all benzoyl groups are removed to obtain a compound 2-61. The hydroxyl groups at the 3- and 5-positions are then protected as a silylene structure by using 1,3-dichloro-1,1,3,3-tetraisopropyldisiloxane ($TBDPSCl_2$), obtaining a compound 2-62. Subsequently, the exposed hydroxyl group at the 2-position is protected with a benzoyl group to obtain a compound 2-63. The silylene protecting group is removed by using tetrabutylammonium fluoride (TBAF) to obtain a compound 2-64. Regioselective TBS protection is conducted on the hydroxyl group at the 5-position to obtain a compound 2-65, and Lev (levulinoyl) protection is conducted on the hydroxyl group at the 3-position to obtain a compound 2-66. Then glycosylation with *p*-methoxybenzyl alcohol is conducted to obtain a compound 2-67. Finally, the Lev protecting group is removed by using hydrazine acetate to obtain a compound 2-68. Detailed descriptions are provided below.

In some embodiments of the present disclosure, a process for preparing the compound 2-63 includes the following steps:

mixing the compound 2-60 and methanol and conducting debenzoylation under a basic condition provided by sodium methoxide to obtain the compound 2-61;
mixing the compound 2-61, $TIPDSCl_2$, and pyridine and conducting silylene protection to obtain the compound 2-62; and
mixing the compound 2-62, 4-dimethylaminopyridine, benzoyl chloride, and pyridine and conducting benzoyl protection to obtain the compound 2-63.

In some embodiments of the present disclosure, the compound 2-60 and methanol are mixed, and the debenzoylation is conducted under the basic condition provided by sodium methoxide to obtain the compound 2-61. In some embodiments, the basic environment has a pH value of 9 to 10; and a basic reagent providing the basic environment is a solution of sodium methoxide in methanol, and a concentration of the basic reagent is 30 wt%. In some embodiments, the debenzoylation is conducted at a temperature of 10°C to 30°C, more preferably room temperature for 6 h to 24 h, more preferably 12 h. In some embodiments, after the debenzoylation is completed, a cation exchange resin is added to a resulting product system to neutralize the pH value to 7; and the cation exchange resin is removed by filtration, and a resulting filtrate is concentrated to obtain a colorless oil, which is the compound 2-61.

In some embodiments of the present disclosure, after obtaining the compound 2-61, the compound 2-61, $TIPDSCl_2$, and pyridine are mixed and subjected to the silylene protection to obtain the compound 2-62. In some embodiments, based on a dosage of the compound 2-60, a molar ratio of compound 2-60 to $TIPDSCl_2$ is in a range of 1 : (1.0-1.5), and more preferably 1 : 1.1. In some embodiments, the compound 2-61 and pyridine are mixed, and $TIPDSCl_2$ is added thereto in batches under an ice bath condition. In some embodiments, the silylene protection is conducted at a temperature of -20°C to 10°C, more preferably 0°C for 5 h to 24 h, more preferably 12 h. In some embodiments, after the silylene protection is completed, the reaction is quenched by adding $H_2O$, a resulting reaction product is extracted with DCM, a resulting organic phase is dried over anhydrous $Na_2SO_4$, filtered, and a resulting filtrate is concentrated and then evacuated by using an oil pump for 2 h to obtain the compound 2-62.

In some embodiments of the present disclosure, after obtaining the compound 2-62, the compound 2-62, 4-dimethylaminopyridine, benzoyl chloride, and pyridine are mixed and subjected to the benzoyl protection to obtain the compound 2-63. In some embodiments, based on the dosage of compound 2-60, a molar ratio of the compound 2-60, the 4-dimethylaminopyridine, and the benzoyl chloride is in a range of 1 : (0-0.1) : (1.1-2), and more preferably 1 : 0.01 : 1.2. In some embodiments, the compound 2-62 and pyridine are mixed, 4-dimethylaminopyridine is added thereto under an ice bath condition, and then benzoyl chloride is added dropwise thereto in batches. In some embodiments, the benzoyl protection includes conducting a first stage reaction and a second stage reaction in sequence; where the first stage reaction is conducted at a temperature of -10°C to 0°C, more preferably -2°C to 0°C for 10 min to 60 min, more preferably 30 min, and the first stage reaction is initiated after the completion of the benzoyl chloride addition; and the second stage

reaction is conducted at a temperature of 0°C to 30°C, more preferably room temperature for 5 h to 24 h, more preferably 12 h. In some embodiments, after the benzoyl protection is completed, anhydrous methanol is added to quench the reaction; and a resulting mixture is concentrated, diluted with DCM, washed with 1 mol/L of hydrochloric acid, saturated $NaHCO_3$ solution, and saturated NaCl solution in sequence, dried over anhydrous $Na_2SO_4$, filtered, and a resulting filtrate is concentrated and then purified by column chromatography (an eluent used is a mixture of petroleum ether and ethyl acetate in a volume ratio of 300 : 1) to obtain an oily liquid, which is the compound 2-63.

In some embodiments of the present disclosure, a process for preparing the compound 2-64 includes: mixing the compound 2-63, acetic acid, tetrabutylammonium fluoride, and tetrahydrofuran, and conducting de-silylene protection to obtain the compound 2-64.

In some embodiments of the present disclosure, a molar ratio of the compound 2-63, the acetic acid, and the tetrabutylammonium fluoride is in a range of 1 : (1.5-3.0) : (1.5-3.0), and more preferably 1 : 2.4 : 2.4. In some embodiments, the compound 2-63 and tetrahydrofuran are mixed, and then acetic acid and a solution of tetrabutylammonium fluoride in tetrahydrofuran are added in sequence under an ice bath condition. In some embodiments, the de-silylene protection includes conducting a first stage reaction and a second stage reaction in sequence; where the first stage reaction is conducted at a temperature of -5°C to 10°C, more preferably 0°C for 15 min to 45 min, more preferably 30 min; and the second stage reaction is conducted at a temperature of 10°C to 30°C, more preferably room temperature for 2 h to 6 h, more preferably 4 h. In some embodiments, after the de-silylene protection is completed, a resulting product system is concentrated and then purified by column chromatography (an eluent used is a mixture of DCM and methanol in a volume ratio of 40 : 1) to obtain a colorless oil, which is the compound 2-64.

In some embodiments of the present disclosure, a process for preparing the compound 2-65 includes: mixing the compound 2-64, imidazole, tert-butyldimethylsilyl chloride (TBSCl), and DCM, and conducting TBS protection to obtain the compound 2-65.

In some embodiments of the present disclosure, a molar ratio of the compound 2-64, the imidazole, and the TBSCl is in a range of 1 : (2-10) : (1-1.1), and more preferably 1 : 3 : 1.05. In some embodiments, the compound 2-64 is dissolved in DCM, imidazole is added thereto under an ice bath condition, and then TBSCl is added thereto in batches. In some embodiments, the TBS protection includes conducting a first stage reaction and a second stage reaction in sequence; where the first stage reaction is conducted at a temperature of -10°C to 0°C, more preferably -2°C to 0°C for 0.5 h to 2 h, more preferably 1 h; and the second stage reaction is conducted at a temperature of 10°C to 30°C, more preferably room temperature for 1 h to 10 h, more preferably 3 h. In some embodiments, after the TBS protection is completed, a resulting reaction material is diluted with DCM, washed with water and then saturated NaCl solution, dried over anhydrous $Na_2SO_4$, filtered, and a resulting filtrate is concentrated and then separated by FLASH rapid preparative chromatography (a mobile phase used is a mixture of petroleum ether and acetone, where a volume fraction of acetone is 10%) to obtain a white powder, which is the compound 2-65.

In some embodiments of the present disclosure, a process for preparing the compound 2-66 includes: mixing the compound 2-65, 1-ethyl-3-(3-dimethylaminopropyl)carbodiimide (EDCI), 4-dimethylaminopyridine, levulinic acid, and DCM, and conducting Lev protection to obtain the compound 2-66.

In some embodiments of the present disclosure, a molar ratio of the compound 2-65, the EDCI, the 4-dimethylaminopyridine, and the levulinic acid is in a range of 1 : (1.5-5) : (0.5-2) : (1.2-2), and more preferably 1 : 2 : 1 : 1.5. In some embodiments, the compound 2-65 and DCM are mixed, and then EDCI, 4-dimethylaminopyridine, and levulinic acid are added thereto in sequence. In some embodiments, the Lev protection is conducted at a temperature of 10°C to 35°C, more preferably room temperature for 4 h to 24 h, more preferably 12 h. In some embodiments, after the Lev protection is completed, a resulting product is washed with saturated $NaHCO_3$ solution, 1 mol/L of hydrochloric acid, and saturated NaCl solution in sequence, dried over anhydrous $Na_2SO_4$, filtered, and a resulting filtrate is concentrated and then purified by column chromatography (an eluent used is a mixture of petroleum ether and acetone in a volume ratio of 20 : 1) to obtain a colorless, transparent oil, which is the compound 2-66.

In some embodiments of the present disclosure, a process for preparing the compound 2-67 includes: mixing the compound 2-66, 2,4,6-tri-tert-butylpyrimidine, *p*-methoxybenzyl alcohol, silver trifluoromethanesulfonate, *p*-toluenesulfenyl chloride, and DCM and conducting glycosylation to obtain the compound 2-67.

In some embodiments of the present disclosure, a molar ratio of the compound 2-66, the 2,4,6-tri-tert-butylpyrimidine, the *p*-methoxybenzyl alcohol, the silver trifluoromethanesulfonate, and the *p*-toluenesulfenyl chloride is in a range of 1 : (2-4) : (1.5-4) : (1.5-3) : (1-2), and more preferably 1 : 2 : 2 : 2 : 1.5. In some embodiments, the glycosylation is conducted in the presence of a 4Å molecular sieve; and the 4Å molecular sieve is activated by baking under vacuum by using a blowtorch and then cooled to room temperature before use. In some embodiments, the compound 2-66, the 4Å molecular sieve, and DCM are mixed, and then 2,4,6-tri-tert-butylpyrimidine and p-methoxybenzyl alcohol are added thereto in sequence; and a resulting mixture is stirred at room temperature for 15 min to 20 min, then cooled to a required starting temperature for the glycosylation, and silver trifluoromethanesulfonate and *p*-toluenesulfenyl chloride are added thereto. In some embodiments, the starting temperature for the glycosylation is in a range of -60°C to -80°C, more preferably -78°C; and a reaction is conducted by natural heating from the starting temperature for 2 h to 10 h, more

preferably 3 h. In some embodiments, after the glycosylation is conducted, TEA is added to quench the reaction; and a resulting reaction material is filtered through diatomaceous earth to remove the molecular sieve, and a resulting filtrate is concentrated and then separated by FLASH rapid preparative chromatography (a mobile phase used is a mixture of petroleum ether and ethyl acetate, where a volume fraction of ethyl acetate is 35%) to obtain a colorless oil, which is the compound 2-67.

In some embodiments of the present disclosure, a process for preparing the compound 2-68 includes: mixing the compound 2-67, hydrazine acetate, tetrahydrofuran, and methanol and conducting de-Lev protection to obtain the compound 2-68.

In some embodiments of the present disclosure, a molar ratio of compound 2-67 to hydrazine acetate is in a range of 1 : (3-20), and more preferably 1 : 5; and a volume ratio of tetrahydrofuran to methanol is in a range of (10-30) : 1, and more preferably 19 : 1. In some embodiments, the compound 2-67, tetrahydrofuran, and methanol are mixed, and then hydrazine acetate is added thereto. In some embodiments, the de-Lev protection is conducted at a temperature of 10°C to 30°C, more preferably room temperature for 2 h to 10 h, more preferably 4 h. In some embodiments, after the de-Lev protection is completed, acetone is added, and a resulting mixture is stirred at room temperature for 20 min to 30 min to quench the reaction; and a resulting reaction material is concentrated and then separated by FLASH rapid preparative chromatography (a mobile phase used is a mixture of petroleum ether and ethyl acetate, where a volume fraction of ethyl acetate is 30%) to obtain a pale yellow oil, which is the compound 2-68.

In some embodiments of the present disclosure, a reaction scheme for preparing the compound 1-5 is as follows:

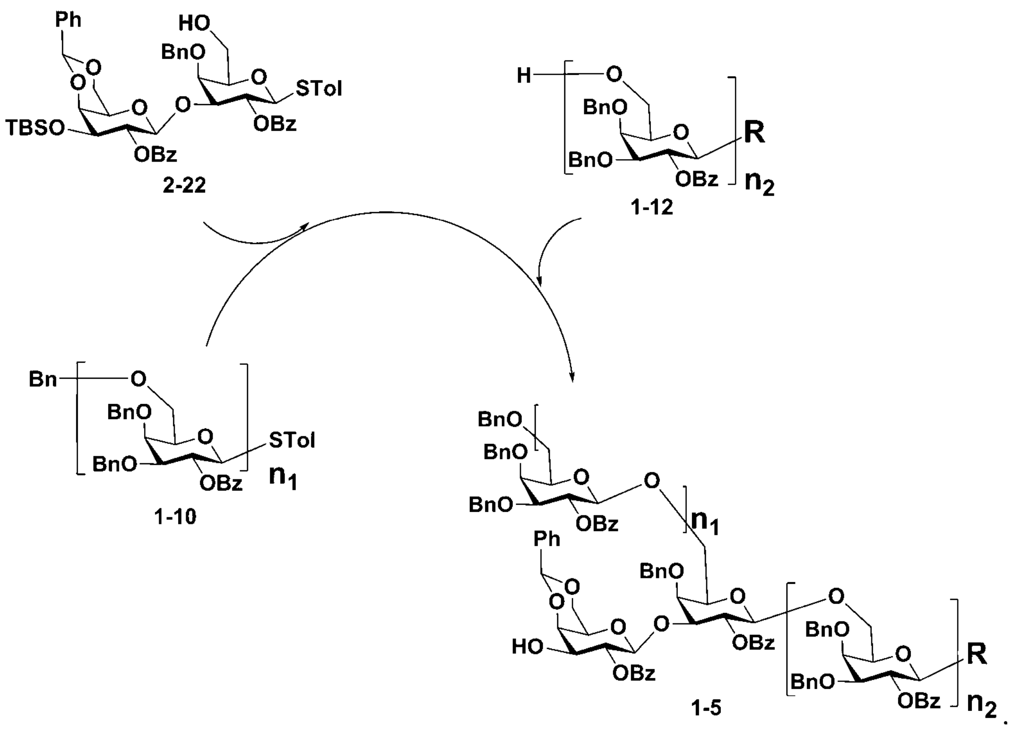

2-22

1-12

1-10

1-5

In some embodiments of the present disclosure, a process for preparing the compound 1-5 includes the following steps:

subjecting the compound 1-10 to activation in the presence of an activator, then conducting glycosylation coupling with the compound 2-22 to obtain a first coupling compound;
subjecting the first coupling compound to activation in the presence of an activator, then conducting glycosylation coupling with the compound 1-12 to obtain a second coupling compound; and
subjecting the second coupling compound to de-TBS protection to obtain the compound 1-5.

In some embodiments of the present disclosure, the reagents and reaction conditions required for the activation and glycosylation coupling involved in preparing the compound 1-5 are consistent with those described in the foregoing technical solutions and will not be detailed again here. In some embodiments, the second coupling compound and an organic solvent are mixed, and a solution of hydrogen fluoride in pyridine is added dropwise under an ice bath condition, after which the de-TBS protection is conducted. In some embodiments, the organic solvent is tetrahydrofuran. There are no specific limitations on the dosage of the organic solvent, as long as the reaction could be proceeded smoothly. In some

embodiments, a molar ratio of hydrogen fluoride in the solution of hydrogen fluoride in pyridine to the second coupling compound is in a range of (4-6) : 1, and more preferably 5 : 1. In some embodiments, the de-TBS protection is conducted at a temperature of 25°C to 40°C, more preferably 35°C for 4 h to 15 h, more preferably 10 h. In some embodiments, after the de-TBS protection is completed, TEA is added to quench the reaction; and after removing the solvent by concentration under reduced pressure, a resulting residue is dissolved in DCM; and a resulting solution is washed with saturated $NaHCO_3$ solution and then saturated NaCl solution, dried over anhydrous $Na_2SO_4$, filtered, and a resulting filtrate is concentrated and then separated by FLASH rapid preparative chromatography (a mobile phase used is a mixture of petroleum ether, ethyl acetate, and DCM, where a volume fraction of ethyl acetate is 70%, and a volume fraction of DCM is 15%) to obtain the compound 1-5.

It should be noted that, in the present disclosure, under a condition that $n_1$ is 0, compounds 2-22-1 and 1-12 are primarily used as reactants to prepare the compound 1-5 by referring to the above technical solutions.

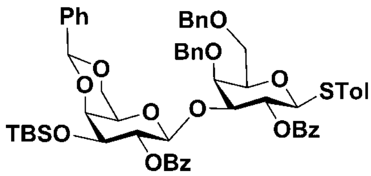

2-22-1

.

In some embodiments of the present disclosure, $n_1$ in the compound 1-10 ranges from 0 to 6, which is specifically categorized as follows:

Under a condition that $n_1$ is 0, the compound 1-10 is considered non-existent (i.e., the compound 1-10 is not required for preparing the compound 1-5).

Under a condition that $n_1$ is 1, the compound 1-10 is specifically compound 2-43.

Under a condition that $n_1$ is 2 to 6, a process for preparing the compound 1-10 includes the following steps:

subjecting the compound 2-43 to activation in the presence of an activator, and conducting glycosylation coupling with compound 2-21 to obtain a coupling compound A (i.e., $n_1$=2);

subjecting the coupling compound A to activation in the presence of an activator, and conducting glycosylation coupling with compound 2-21 to obtain a coupling compound B (i.e., $n_1$=3); and

repeating the activation and glycosylation coupling with compound 2-21 until the compound 1-10 with a desired chain length is obtained;

where a structural formula of compound 2-21 is as follows:

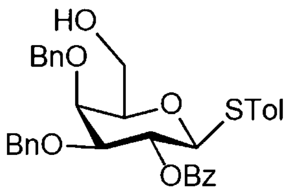

2-21

.

In some embodiments of the present disclosure, the reagents and reaction conditions required for the activation and glycosylation coupling involved in preparing the compound 1-10 are consistent with those described in the foregoing technical solutions and will not be detailed again here.

In some embodiments of the present disclosure, a reaction scheme for preparing the compound 2-22 is as follows:

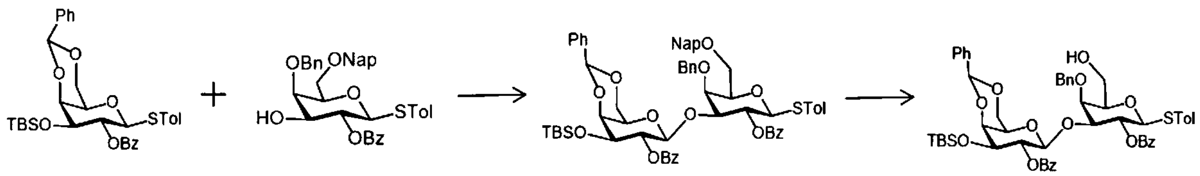

2-45 2-48 2-48-2 2-22

.

In some embodiments of the present disclosure, starting from a compound 2-45 protected with a benzylidene group at the 4- and 6-positions as a donor, activation is conducted by using a *p*-TolSCl/AgOTf activation system. Glycosylation coupling with compound 2-48 in which a 3-position acceptor bearing a Nap (naphthyl) group at the 6-position is conducted to obtain a compound 2-48-2. Finally, the compound 2-22 is obtained by removing the temporary Nap protecting group at the 6-position by using DDQ. Detailed descriptions are provided below.

In some embodiments of the present disclosure, a process for preparing the compound 2-22 includes the following steps:

mixing the compound 2-45, silver trifluoromethanesulfonate, *p*-toluenesulfenyl chloride, and DCM and conducting activation to obtain an activation product system; and
mixing the activation product system and the compound 2-48 and conducting glycosylation coupling to obtain the compound 2-22.

In some embodiments of the present disclosure, a molar ratio of the compound 2-45, the silver trifluoromethanesulfonate, the *p*-toluenesulfenyl chloride, and the compound 2-48 is in a range of 1 : (1.5-2) : (0.95-1.05) : (0.95-1.05), and more preferably 1 : 2 : 1 : 1. In some embodiments, the activation is conducted in the presence of a 4Å molecular sieve; and the 4Å molecular sieve is activated by baking under vacuum by using a blowtorch and then cooled to room temperature before use. In some embodiments, the compound 2-45, the 4Å molecular sieve, and DCM are mixed, then a resulting mixture is cooled to a temperature required for the activation, and silver trifluoromethanesulfonate and *p*-toluenesulfenyl chloride are added thereto and the activation is conducted. In some embodiments, the activation is conducted at a temperature of -60°C to -80°C, more preferably -78°C for 5 min to 10 min, more preferably 5 min to 7 min. In some embodiments, the compound 2-48 is dissolved in DCM and then added dropwise into the activation product system, and the glycosylation coupling is conducted. In some embodiments, the glycosylation coupling includes conducting a first stage reaction and a second stage reaction in sequence; where the first stage reaction is conducted at a temperature of -60°C to -78°C, more preferably -75°C to -78°C for 5 min to 15 min, more preferably 10 min; and an obtained reaction mixture is subjected to natural heating from a temperature of the first stage reaction to conduct the second stage reaction for 2.5 h to 3.5 h, more preferably 3 h. In some embodiments, conducting the reactions initially at a lower temperature helps avoid decomposition of the reactive intermediate caused by temperature increase. In some embodiments, after the compound 2-48 is added, a resulting mixture is stirred at -78°C for 10 min and then is reacted for 3 h by natural heating. In some embodiments, after the glycosylation coupling is completed, TEA is added to quench the reaction; and a resulting reaction material is filtered through diatomaceous earth to remove the molecular sieve, and a resulting filtrate is concentrated and then separated by FLASH rapid preparative chromatography (a mobile phase used is a mixture of petroleum ether, ethyl acetate, and DCM, where a volume fraction of ethyl acetate is 30%, and a volume fraction of DCM is 15%) to obtain a white powder, which is the compound 2-48-2.

In some embodiments of the present disclosure, a reaction scheme for preparing the compound 2-48 is as follows:

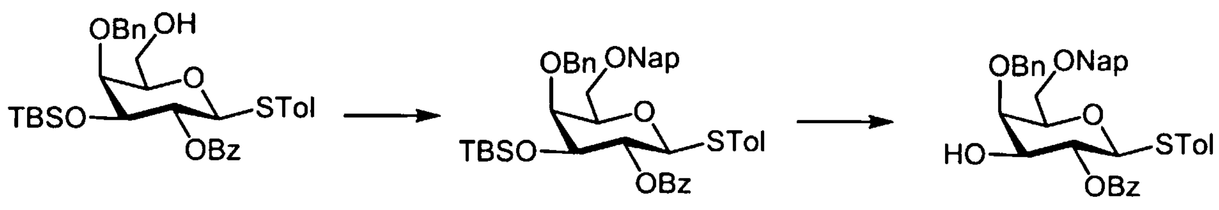

2-46 2-47 2-48

.

In some embodiments of the present disclosure, the Nap protecting group is introduced at the 6-position of compound 2-46 to obtain a compound 2-47. Then, the TBS protecting group at the 3-position is removed under the action of a Lewis acid to obtain the compound 2-48. Detailed descriptions are provided below.

In some embodiments of the present disclosure, a process for preparing the compound 2-48 includes the following steps:

mixing the compound 2-46, NapBr, sodium hydride, tetrahydrofuran, and DMF and conducting Nap protection to obtain the compound 2-47; and
mixing the compound 2-47, boron trifluoride diethyl etherate, and acetonitrile and conducting de-TBS protection to obtain the compound 2-48.

In some embodiments of the present disclosure, the compound 2-46, NapBr, sodium hydride, tetrahydrofuran,

and DMF are mixed and subjected to Nap protection to obtain the compound 2-47. In some embodiments, a molar ratio of the compound 2-46, the NapBr, and the sodium hydride is in a range of 1 : (1.5-3) : (1.2-3), and more preferably 1 : 2 : 1.5; and a volume ratio of tetrahydrofuran to DMF is in a range of (5-10) : 1, and more preferably 9 : 1. In some embodiments, the sodium hydride is used in a form of a mineral oil solution, with a mass fraction of 60%. In some embodiments, the compound 2-46, tetrahydrofuran, and DMF are mixed, NapBr is added thereto under an ice bath condition, and then a solution of sodium hydride in mineral oil is added thereto in batches. In some embodiments, the Nap protection is conducted at a temperature of -10°C to 10°C, more preferably 0°C for 2 h to 10 h, more preferably 5 h. In some embodiments, after the Nap protection is completed, the reaction is quenched by adding saturated $NH_4Cl$ solution, a resulting reaction product is extracted with DCM, a resulting organic phase is dried over anhydrous $Na_2SO_4$, filtered, and a resulting filtrate is concentrated and then separated by FLASH rapid preparative chromatography (a mobile phase used is a mixture of petroleum ether and ethyl acetate, where a volume fraction of ethyl acetate is 25%) to obtain a white powder, which is the compound 2-47.

In some embodiments of the present disclosure, after obtaining the compound 2-47, the compound 2-47, boron trifluoride diethyl etherate, and acetonitrile are mixed and subjected to de-TBS protection to obtain the compound 2-48. In some embodiments, a molar ratio of compound 2-47 to boron trifluoride diethyl etherate is in a range of 1 : (1-3), and more preferably 1 : 2. In some embodiments, the compound 2-47 and acetonitrile are mixed, and boron trifluoride diethyl etherate is added dropwise thereto under an ice bath condition. In some embodiments, the de-TBS protection is conducted at a temperature of -10°C to 10°C, more preferably 0°C for 3 min to 10 min, more preferably 5 min. In some embodiments, after the de-TBS protection is completed, TEA is added to quench the reaction; and the solvent is removed by concentration under reduced pressure, and a resulting residue is dissolved in DCM; and a resulting solution is washed with water and then saturated NaCl solution, a resulting organic phase is dried over anhydrous $Na_2SO_4$, filtered, and a resulting filtrate is concentrated and then purified by column chromatography (an eluent used is a mixture of petroleum ether, ethyl acetate, and DCM in a volume ratio of 4 : 1 : 4) to obtain a white powder, which is the compound 2-48.

In some embodiments of the present disclosure, $n_2$ in the compound 1-12 ranges from 0 to 2, which is specifically categorized as follows:

Under a condition that $n_2$ is 0, the compound 1-12 is specifically H-R, i.e., a C1-C10 alkyl alcohol.

Under a condition that $n_2$ is 1, the compound 1-12 is specifically compound 1-18.

Under a condition that $n_2$ is 2, a process for preparing the compound 1-12 includes the following steps:

subjecting the compound 2-20 to activation in the presence of an activator, and conducting glycosylation coupling with the compound 1-18 to obtain a coupling compound; and

mixing the coupling compound, hydrazine acetate, and an organic solvent and conducting deprotection to obtain the compound 1-12 (where $n_1$ is 2);

where the structural formulas of the compound 2-20 and the compound 1-18 are as follows, respectively:

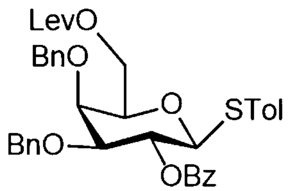

2-20

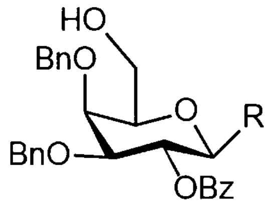

1-18

.

In some embodiments of the present disclosure, the reagents and reaction conditions required for the activation and glycosylation coupling involved in preparing the compound 1-12 are consistent with those described in the foregoing technical solutions and will not be detailed again here. In some embodiments, the organic solvent for the deprotection is a mixture of tetrahydrofuran and methanol, with a volume ratio of tetrahydrofuran to methanol is 10 : 1. There are no specific limitations on the dosage of the organic solvent, as long as the reaction could be proceeded smoothly. In some embodiments, a molar ratio of hydrazine acetate to the coupling compound is in a range of (2-10) : 1, and more preferably 5 : 1. In some embodiments, the deprotection is conducted at a temperature of 10°C to 40°C, more preferably room temperature, for a reaction time determined based on the complete consumption of starting materials. In some embodiments, after the deprotection is completed, acetone is added to quench the reaction; and a resulting reaction material is concentrated and then purified by column chromatography or rapid preparative chromatography to obtain the compound 1-12.

In some embodiments of the present disclosure, the compound 2-21 as a starting material is introduced a Lev protecting group at the 6-position to obtain the compound 20. In some embodiments, a process for preparing the compound 2-20 includes:

mixing the compound 2-21, N,N'-diisopropylcarbodiimide (DIC), 4-dimethylaminopyridine, levulinic acid, and DCM and conducting Lev protection to obtain the compound 2-20.

In some embodiments of the present disclosure, a molar ratio of the compound 2-21, the DIC, the 4-dimethylaminopyridine, and the levulinic acid is in a range of 1 : (1-5) : (0.1-1) : (1.5-3), and more preferably 1 : 1.9 : 0.6 : 2. In some embodiments, the compound 2-21 and DCM are mixed, and then 4-dimethylaminopyridine, levulinic acid, and DIC are added in sequence under an ice bath condition. In some embodiments, the Lev protection includes conducting a first stage reaction and a second stage reaction in sequence; where the first stage reaction is conducted at a temperature of -10°C to 10°C, more preferably 0°C for 10 min to 60 min, more preferably 30 min; and the second stage reaction is conducted at a temperature of 10°C to 40°C, more preferably room temperature for 0.5 h to 5 h, more preferably 1 h. In some embodiments, after the Lev protection is completed, the reaction is quenched by adding saturated $NaHCO_3$ solution; and a resulting reaction material is diluted with DCM, washed with saturated $NaHCO_3$ solution and then saturated NaCl solution, dried over anhydrous $Na_2SO_4$, filtered, and a resulting filtrate is concentrated and then purified by column chromatography (an eluent used is a mixture of petroleum ether, ethyl acetate, and DCM in a volume ratio of 8 : 1 : 4) to obtain a white powder, which is the compound 2-20.

The present disclosure further provides use of the glycan as described above in preparation of an antitumor drug.

In some embodiments of the present disclosure, a tumor is pancreatic cancer, gastric cancer, liver cancer, or cervical cancer. In some embodiments, the antitumor drug includes the glycan and a pharmaceutically acceptable carrier. In some embodiments, a mass fraction of the glycan in the antitumor drug is in a range of 0.1% to 99.9%, more preferably 50% to 99%, and further preferably 80% to 95%. There are no specific limitations on the particular type of the pharmaceutically acceptable carrier, and any pharmaceutically acceptable carrier well-known to those skilled in the art may be used. In some embodiments, a dosage form of the antitumor drug includes an injection or an oral tablet. In some embodiments, an administration route of the antitumor drug is oral administration, intravenous injection, or subcutaneous injection.

The present disclosure further provides an antitumor drug, including the glycan as described above and a pharmaceutically acceptable carrier. There are no specific limitations on a preparation process of the antitumor drug, and any method well-known to those skilled in the art may be used.

The technical solutions of the present disclosure will be clearly and completely described below with reference to the examples of the present disclosure. Apparently, the examples described are merely some rather than all of the examples of the present disclosure. All other examples obtained by a person of ordinary skill in the art based on the examples of the present application without inventive efforts shall fall within the scope of the present application.

Unless otherwise specified, all chemical reagents used in the present disclosure are of analytical grade and require no further purification before use; dry $CH_2Cl_2$, DMF, and pyridine are obtained by atmospheric distillation of commercial solvents over $CaH_2$; dry $CH_3OH$ is obtained by atmospheric distillation of commercial solvents over magnesium turnings; dry toluene is distilled over sodium metal; and dry $Et_2O$ and THF are obtained by atmospheric distillation of commercial solvents over sodium-potassium alloy under an argon atmosphere. Molecular sieves are activated at 400°C in a muffle furnace and dried under vacuum before use. All glycosylation reactions are conducted under an argon atmosphere in dried glass reaction vessels by using freshly distilled solvents. Reactions are monitored by TLC (Merck silica gel 60 $F_{254}$ (1.0554)): after development, spots are visualized under UV light, by direct heating of the plate, and/or by dipping the plate in a developer solution ($Ce(NH_4)_2(NO_3)_6$ (0.50 g, 0.9 mmol) and $(NH_4)_6Mo_7O_{24}•4H_2O$ (24.00 g, 19.4 mmol) dissolved in 5% aqueous sulfuric acid (500 mL)) followed by heating. Column chromatography is conducted by using 200-300 mesh silica gel (Qingdao Ocean Chemical Factory, China). The packing materials for gel columns are Bio-Beads S-X1, LH-20, and Toyopearl HW 55F (TOSOH). Solvents are removed by rotary evaporation under reduced pressure with a water bath maintained below 40°C.

NMR (nuclear magnetic resonance) data are recorded on a Bruker AVANCE III-400 or Bruker AVANCE III-600 NMR spectrometer at 25°C by using $CDCl_3$, $CD_3OD$, and $D_2O$ as deuterated solvents. Chemical shifts are reported relative to tetramethylsilane (TMS) as an internal standard or referenced to the chemical shift of a deuterated solvent (when TMS is not present in the deuterated solvent itself); and signal assignments in $^1H$ and $^{13}C$ NMR spectra are assisted by COSY (correlation spectroscopy), HSQC (heteronuclear single quantum coherence), and HMBC (heteronuclear multiple bond correlation) experiments. High-resolution mass spectra (HRMS) are acquired by using a Waters Xevo G2 Qtof or a Waters SYNAPT G2-Si mass spectrometer; MALDI-TOF MS (matrix-assisted laser desorption/ionization time-of-flight mass spectrometry) are acquired on an AB SCIEX 5800 spectrometer. Specific optical rotations are measured with a Rudolph Research Analytical Autopol IV automatic polarimeter. GPC (gel permeation chromatography) analysis of fully protected glycans is conducted on a Waters e2659 system equipped with a 2414 RI detector (columns: HT2, HT3, HT4, 7.8×300 mm, 10 μm; elution solvent: THF; flow rate: 1 mL/min). HPLC (high performance liquid chromatography) analysis of fully deprotected glycans is conducted on a Thermo UltiMate 3000 liquid chromatograph equipped with an ELSD (evaporative light-scattering detector) detector (columns: BRT102, BRT103, 4.6×250 mm; elution solvent: water; flow rate: 0.7 mL/min).

The following abbreviations are used: s = singlet; d = doublet; t = triplet; m = multiplet; dd = doublet of doublets; dt =

doublet of triplets; td = triplet of doublets; br = broad.

**Preparation Example 1** Preparation of Compound 1-6

(1) Compound 2-52 (20 g, 41.97 mmol, 1.0 equivalent (eq)) was dissolved in dry DCM (50 mL). Acetyl bromide (9.3 mL, 125.92 mmol, 3.0 eq) was added thereto under an ice bath condition, and then anhydrous methanol (4.6 mL, 113.33 mmol, 2.7 eq) was added dropwise thereto. A reaction was conducted on a resulting mixture at room temperature for 3 h. TLC indicated the reaction was complete, obtaining a mixture containing compound 2-53. Under an ice bath condition, 2,6-lutidine (24.5 mL, 209.87 mmol, 5.0 eq) and anhydrous methanol (17 mL, 419.74 mmol, 10.0 eq) were added thereto in sequence. A reaction was conducted on a resulting mixture at room temperature for 24 h. TLC indicated the reaction was complete. A resulting reaction material was diluted with DCM (100 mL), washed with water and then saturated NaCl solution, dried over anhydrous $Na_2SO_4$, filtered, and a resulting filtrate was concentrated. A resulting residue was subjected to an oil pump vacuum for 1 h to obtain a pale yellow oil, which was compound 2-54. Compound 2-54 was dissolved in anhydrous methanol (150 mL), and a solution of sodium methoxide (30 wt%) in methanol was added thereto to adjust a pH value to 9. A reaction was conducted on a resulting mixture at room temperature for 12 h. TLC indicated the reaction was complete. A cation exchange resin was added to a resulting reaction system to neutralize the pH value to 7. The cation exchange resin was removed by filtration, and a resulting filtrate was concentrated and purified by column chromatography (eluent: a mixture of petroleum ether, acetone, and $Et_3N$ in a volume ratio of petroleum ether to acetone of 6 : 1, with a volume fraction of $Et_3N$ of 0.2%) to obtain a pale yellow oil, which was compound 2-55. Compound 2-55 was dissolved in DMF (100 mL). Benzyl bromide (19.94 mL, 167.90 mmol, 4.0 eq) was added thereto under an ice bath condition, and then a solution of sodium hydride (6.72 g, 167.90 mmol, 4.0 eq, 60 wt% in mineral oil) in mineral oil was added thereto in batches. A reaction was conducted on a resulting mixture at 0°C for 1 h and then heated to room temperature (25°C) and reacted for 12 h. TLC monitoring showed the reaction was complete. The reaction was quenched by adding saturated ammonium chloride solution (20 mL). A resulting reaction material was concentrated, diluted with DCM (200 mL), washed with water and then saturated NaCl solution, dried over anhydrous $Na_2SO_4$, filtered, and a resulting filtrate was concentrated and purified by column chromatography (eluent: a mixture of petroleum ether, ethyl acetate, and $Et_3N$ in a volume ratio of petroleum ether to ethyl acetate of 8 : 1, with a volume fraction of $Et_3N$ of 0.2%) to obtain a pale yellow oil, which was compound 2-56 (12.5 g, 66% yield, identified as 1,2-*O*-(α-Methoxybenzylidene)-3-*O*-benzyl-5-*O*-benzyl-α-L-arabinofuranoside).

$R_f$ = 0.25 (petroleum ether/ethyl acetate, 15 : 1). [α]24 D +39.0 (c 0.11, $CHCl_3$); $^1$H NMR (400 MHz, $CDCl_3$) δ 7.58 - 7.51 (m, 2H, Ar), 7.37 - 7.23 (m, 11H, Ar), 7.16 - 7.11 (m, 2H, Ar), 6.19 (d, *J* = 4.3 Hz, 1H, H-1), 4.93 (d, *J* = 4.3 Hz, 1H, H-2), 4.59 (d, *J* = 12.0 Hz, 1H, $PhCH_2$), 4.55 (d, *J* = 12.0 Hz, 1H, $PhCH_2$), 4.36 - 4.30 (m, 2H, H-4, $PhCH_2$), 4.20 (d, *J* = 12.0 Hz, 1H, $PhCH_2$), 3.98 (d, *J* = 1.7 Hz, 1H, H-3), 3.28 (dd, *J* = 9.9, 6.4 Hz, 1H, H-5a), 3.20 - 3.12 (m, 4H, H-5b, $OCH_3$).

$^{13}$C NMR (101 MHz, $CDCl_3$) δ 137.96 (Ar), 137.18 (Ar), 136.55 (Ar), 129.30 (Ar), 128.53 (Ar), 128.34 (Ar), 128.15 (Ar), 127.98 (Ar), 127.89 (Ar), 127.67 (Ar), 127.63 (Ar), 126.34 (Ar), 123.17 (Ar), 106.20 (C-1), 85.38 (C-2), 84.68 (C-4), 82.83 (C-3), 73.19 ($PhCH_2$), 71.60 ($PhCH_2$), 69.95 (C-5), 50.71 ($OCH_3$).

HRMS (ESI) Calcd for $C_{27}H_{28}NaO_6$ $[M+Na]^+$: 471.1784, found: 471.1782.

(2) A two-necked flask, along with a stir bar, was dried by heating under vacuum. After cooling, the two-necked flask was filled with argon. 4Å molecular sieves (6 g) were added thereto, and the molecular sieves were activated by heating under vacuum with a blowtorch, and cooled to room temperature under an argon atmosphere. Compound 2-56 (13.6 g, 30.32 mmol, 1.0 eq), p-toluenethiol (7.53 g, 60.64 mmol, 2.0 eq), and dry DCM (60 mL) were added thereto in sequence. A resulting mixture was stirred at room temperature for 20 min, then cooled to -5°C. $SnCl_4$ (0.354 mL, 3.032 mmol, 0.1 eq) was added dropwise thereto in batches. A reaction was conducted at -5°C for 12 h. TLC indicated the reaction was complete. The reaction was quenched by adding TEA (2 mL). A resulting reaction material was filtered through diatomaceous earth to remove the molecular sieves. A resulting filtrate was concentrated and purified by column chromatography (eluent: petroleum ether: ethyl acetate = 20 : 1, v (volume) /v) to obtain a colorless, transparent oily liquid, which was compound 1-6 (12.4 g, 87% yield, identified as *p*-Tolyl-2-*O*-benzoyl-3-*O*-benzyl-5-*O*-benzyl-1-thio-α-L-arabinofuranoside).

$R_f$ = 0.34 (petroleum ether/ethyl acetate, 15 : 1). [α]24 D -108.3 (c 0.13, $CHCl_3$); $^1$H NMR (400 MHz, $CDCl_3$) δ 7.99 - 7.94 (m, 2H, Ar), 7.56 (t, *J* = 7.4 Hz, 1H, Ar), 7.47 - 7.37 (m, 5H, Ar), 7.36 - 7.24 (m, 9H, Ar), 7.10 (d, *J* = 7.9 Hz, 2H, Ar), 5.65 (s, 1H, H-1), 5.56 (t, *J* = 1.5 Hz, 1H, H-2), 4.81 (d, *J* = 12.0 Hz, 1H, $PhCH_2$), 4.61 (d, *J* = 12.0 Hz, 1H, $PhCH_2$), 4.58 - 4.55 (m, 1H, H-4), 4.55 (d, *J* = 11.7 Hz, 2H, $PhCH_2$), 4.49 (d, *J* = 12.0 Hz, 1H, $PhCH_2$), 4.14 (d, *J* = 5.4 Hz, 1H, H-3), 3.71 (dd, *J* = 10.9, 4.0 Hz, 1H, H-5a), 3.65 (dd, *J* = 10.8, 4.6 Hz, 1H, H-5b), 2.31 (s, 3H, tolyl $CH_3$).

$^{13}$C NMR (100 MHz, $CDCl_3$) δ 165.42 (C=O), 137.98 (Ar), 137.61 (Ar), 137.52 (Ar), 133.40 (Ar), 132.45 (Ar),

130.62 (Ar), 129.82 (Ar), 129.73 (Ar), 129.36 (Ar), 128.47 (Ar), 128.42 (Ar), 128.32 (Ar), 127.96 (Ar), 127.85 (Ar), 127.64 (Ar), 127.61 (Ar), 91.62 (C-1), 83.24 (C-3), 82.32 (C-2), 82.05 (C-4), 73.40 (PhC), 72.33 (PhC), 68.93 (C-5), 21.13 (tolyl $CH_3$).

HRMS (ESI) Calcd for $C_{33}H_{32}NaO_5S$ $[M+Na]^+$: 563.1868, found:563.1876.

**Preparation Example 2** Preparation of Compound 1-7

(1) Compound 2-55 (13.4 g, 49.95 mmol, 1.0 eq) was dissolved in dry pyridine (90 mL). A resulting solution was cooled to -5°C, and then TEA (24.30 mL, 174.83 mmol, 3.5 eq) and DMAP (61.03 mg, 0.499 mmol, 0.01 eq) were added thereto in sequence. TBSCl (8.28 g, 54.95 mmol, 1.1 eq) was added thereto in batches. A reaction was conducted at 5°C for 12 h. TLC indicated the reaction was complete. A resulting reaction material was concentrated and purified by column chromatography (eluent: a mixture of petroleum ether, acetone, and $Et_3N$ in a volume ratio of petroleum ether to acetone of 6 : 1, with a volume fraction of $Et_3N$ of 0.2%) to obtain an oily substance, which was compound 2-57 (16.5 g, 86% yield, identified as 1,2-*O*-(α-Methoxybenzylidene)-5-*O*-tert-butyldimethylsilyl-α-L-arabinofuranoside).

$R_f$= 0.49 (petroleum ether/acetone, 3 : 1). [α]24 D +28.0 (c 0.13, $CHCl_3$); $^1H$ NMR (400 MHz, $CDCl_3$) δ 7.58 (dt, *J* = 7.2, 3.3 Hz, 2H, Ar), 7.42 - 7.31 (m, 3H, Ar), 6.20 (d, *J* = 4.3 Hz, 1H, H-1), 4.84 (d, *J* = 4.3 Hz, 1H, H-2), 4.32 (d, *J* = 4.0 Hz, 1H, H-3), 4.05 (dd, *J* = 9.6, 5.5 Hz, 1H, H-4), 3.48 (dd, *J* = 10.0, 5.5 Hz, 1H, H-5a), 3.25 (t, *J* = 9.8 Hz, 1H, H-5b), 3.15 (s, 3H, $CH_3$), 1.97 (d, *J* = 4.2 Hz, 1H, OH), 0.76 (s, 9H, TBS), -0.16 (s, 3H, TBS), -0.18 (s, 3H, TBS).

$^{13}C$ NMR (100 MHz, $CDCl_3$) δ 136.19 (Ar), 129.33 (Ar), 128.14 (Ar), 126.31 (Ar), 123.02 (Ar), 110.01(PhC), 106.11(C-1), 88.92(C-4), 87.11(C-2), 76.17(C-3), 63.00(C-5), 50.78($OCH_3$), 25.80(TBS), 18.19(TBS), -5.58(TBS), -5.66(TBS).

HRMS (ESI) Calcd for $C_{19}H_{30}O_6Si$ $[M+Na]^+$: 405.1709, found: 405.1706.

(2) Compound 2-57 (16.5 g, 43.13 mmol, 1.0 eq) was dissolved in DMF (150 mL). Under an ice bath condition, benzyl bromide (7.68 mL, 64.70 mmol, 1.5 eq) and a solution of sodium hydride (2.24 g, 56.07 mmol, 1.3 eq, 60 wt% in mineral oil) in mineral oil were added thereto in sequence. A reaction was conducted at 0°C for 30 min and then heated to room temperature and conducted for 4 h. TLC indicated the reaction was complete. The reaction was quenched by adding $H_2O$ (20 mL). A resulting mixture was extracted with DCM twice. A resulting combined organic phases were washed twice with saturated NaCl solution, dried over anhydrous $Na_2SO_4$, filtered, and a resulting filtrate was concentrated and purified by column chromatography (eluent: a mixture of petroleum ether, ethyl acetate, and $Et_3N$ in a volume ratio of petroleum ether to ethyl acetate of 20 : 1, with a volume fraction of $Et_3N$ of 0.2%) to obtain an oily substance, which was compound 2-58 (17.4 g, 85% yield, identified as 1,2-*O*-(α-Methoxybenzylidene)-3-*O*-benzyl-5-*O*-tert-butyldimethylsilyl-α-L-arabinofuranoside).

$R_f$= 0.38 (petroleum ether/ethyl acetate, 15 : 1). [α]24 D +29.3 (c 0.44, $CHCl_3$); $^1H$ NMR (400 MHz, $CDCl_3$) δ 7.61 - 7.54 (m, 2H, Ar), 7.41 - 7.27 (m, 8H, Ar), 6.20 (d, *J* = 4.3 Hz, 1H, H-1), 4.94 (d, *J* = 4.3 Hz, 1H, H-2), 4.59 (d, *J* = 12.0 Hz, 1H, $PhCH_2$), 4.55 (d, *J* = 12.0 Hz, 1H $PhCH_2$), 4.23 (dd, *J* = 9.8, 5.4 Hz, 1H, H-4), 4.05 (s, 1H, H-3), 3.46 (d, *J* = 10.0 Hz, 1H, H-5a), 3.20 (t, *J* = 9.9 Hz, 1H, H-5b), 3.15 (s, 3H, $OCH_3$), 0.75 (s, 9H, TBS), -0.18 (s, 3H, TBS), -0.21 (s, 3H, TBS).

$^{13}C$ NMR (100 MHz, $CDCl_3$) δ 137.34(Ar), 136.26(Ar), 129.32(Ar), 128.52(Ar), 128.15(Ar), 127.91(Ar), 127.78(Ar), 126.88(Ar), 126.30(Ar), 122.94(PhC), 106.43(C-1), 86.67(C-4), 85.39(C-2), 82.62(C-3), 71.43($PhCH_2$), 62.80(C-5), 50.80(-$OCH_3$), 25.81(t-Bu), 18.15(t-Bu), -5.62($CH_3$), -5.64($CH_3$).

HRMS (ESI) Calcd for $C_{26}H_{36}O_6Si$ $[M+Na]^+$: 459.2179, found: 459.2186.

(3) A two-necked flask, along with a stir bar, was dried by heating under vacuum. After cooling, the two-necked flask was filled with argon. 4Å molecular sieves (6 g) were added thereto, and the molecular sieves were activated by heating under vacuum with a blowtorch, and cooled to room temperature under an argon atmosphere. Compound 2-58 (11 g, 23.27 mmol, 1.0 eq), p-toluenethiol (5.78 g, 46.55 mmol, 2.0 eq), and dry DCM (60 mL) were added thereto in sequence. A resulting mixture was stirred at room temperature for 20 min, then cooled to -5°C. $SnCl_4$ (0.272 mL, 2.33 mmol, 0.1 eq) was added dropwise thereto in batches. A reaction was conducted at -5°C for 12 h. TLC indicated the reaction was complete. The reaction was quenched by adding TEA (2 mL). A resulting reaction material was filtered through diatomaceous earth to remove the molecular sieves. A resulting filtrate was concentrated and purified by column chromatography (eluent: a mixture of petroleum ether, ethyl acetate, and $Et_3N$ in a volume ratio of petroleum ether to ethyl acetate of 100 : 1, with a volume fraction of $Et_3N$ of 0.2%) to obtain a colorless, transparent oily liquid, which was compound 2-59. The compound 2-59 was dissolved in anhydrous acetonitrile (60 mL). Boron trifluoride diethyl etherate (3.16 mL, 1.1 eq) was added dropwise thereto under an ice bath condition. A reaction was conducted at 0°C for 4 h. TLC indicated the reaction was complete. The reaction was quenched by adding TEA (5 mL). A resulting reaction material was concentrated and purified by column chromatography (eluent: a mixture of petroleum ether, ethyl acetate, and $Et_3N$ in a volume ratio of petroleum ether to ethyl acetate of 8 : 1, with a volume fraction of $Et_3N$ of 0.2%) to obtain a colorless,

transparent oily liquid, which was compound 1-7 (6.8 g, 65% yield over two steps, identified as*p*-Tolyl-2-*O*-benzoyl-3-*O*-benzyl-1-thio-α-L-arabinofuranoside).

$R_f$= 0.24 (petroleum ether/ethyl acetate, 4 : 1). [α]24 D -138.1 (c 0.13, $CHCl_3$); $^1$H NMR (400 MHz, $CDCl_3$) δ 8.03 - 7.98 (m, 2H, Ar), 7.61 - 7.55 (m, 1H, Ar), 7.44 (dt, *J* = 7.5, 3.6 Hz, 5H, Ar), 7.39 - 7.26 (m, 6H, Ar), 7.13 (d, *J* = 7.9 Hz, 2H, Ar), 5.62 (s, 1H, H-1), 5.55 (t, *J* = 1.6 Hz, 1H, H-2), 4.85 (d, *J* = 12.0 Hz, 1H, $PhCH_2$), 4.63 (d, *J* = 12.0 Hz, 1H, $PhCH_2$), 4.52 - 4.48 (m, 1H, H-4), 4.17 (dd, *J* = 5.6, 0.7 Hz, 1H, H-3), 3.90 (dd, *J* = 12.3, 2.9 Hz, 1H, H-5a), 3.70 (dd, *J* = 12.3, 3.7 Hz, 1H, H-5b), 2.32 (s, 3H, tolyl $CH_3$).

$^{13}$C NMR (101 MHz, $CDCl_3$) δ 165.36 (C=O), 137.97 (Ar), 137.42 (Ar), 133.54 (Ar), 132.81 (Ar), 130.27 (Ar), 129.83 (Ar), 129.78 (Ar), 129.28 (Ar), 128.55 (Ar), 128.50 (Ar), 127.97 (Ar), 127.92 (Ar), 91.86 (C-1), 83.10 (C-4), 82.71 (C-3), 82.33 (C-2), 72.57 ($PhCH_2$), 61.67 (C-5), 21.15 (tolyl $CH_3$).

HRMS (ESI) Calcd for $C_{26}H_{26}O_5S$ $[M+Na]^+$: 473.1399, found: 473.1399.

**Preparation Example 3** Preparation of Compound 1-4

(1) Compound 2-60 (130 g, 228.62 mmol, 1.0 eq) was dissolved in anhydrous methanol (450 mL) to form a suspension. A solution of sodium methoxide (30 wt%) in methanol was added dropwise thereto under an ice bath condition to adjust a pH value to 9. A reaction was conducted at room temperature for 12 h. TLC indicated the reaction was complete. A cation exchange resin was added thereto to adjust the pH value to 7. The resin was removed by filtration, and a resulting filtrate was concentrated to obtain a colorless oil, which was compound 2-61. The compound 2-61 was dissolved in anhydrous pyridine (350 mL). 1,3-Dichloro-1,1,3,3-tetraisopropyldisiloxane ($TIPDSCl_2$, 71.41 mL, 251.48 mmol, 1.1 eq) was added thereto in batches under an ice bath condition. A reaction was conducted at 0°C for 12 h. TLC indicated the reaction was complete. The reaction was quenched by adding $H_2O$ (100 mL), a resulting mixture was extracted with DCM, and a resulting combined organic phases were dried over anhydrous $Na_2SO_4$, filtered, and a resulting filtrate was concentrated. A resulting concentrate was subjected to an oil pump vacuum for 2 h to obtain a yellow oil, which was a crude product of compound 2-62. The crude product of compound 2-62 was dissolved in anhydrous pyridine (300 mL). DMAP (279.30 mg, 2.29 mmol, 0.01 eq) was added thereto under an ice bath condition, and then benzoyl chloride (31.87 mL, 274.34 mmol, 1.2 eq) was added dropwise thereto in batches. A reaction was conducted at 0°C for 30 min and then heated to room temperature and conducted for 12 h. TLC indicated the reaction was complete. The reaction was quenched by adding anhydrous methanol (50 mL). A resulting reaction material was concentrated, diluted with DCM (400 mL), washed with 1 mol/L of hydrochloric acid, saturated $NaHCO_3$ solution, and saturated NaCl solution in sequence, dried over anhydrous $Na_2SO_4$, filtered, and a resulting filtrate was concentrated and purified by column chromatography (eluent: petroleum ether: ethyl acetate = 300 : 1, v (volume) /v) to obtain an oily liquid, which was compound 2-63 (81 g, 59% yield over three steps, identified as *p*-Tolyl-2-*O*-benzoyl-3,5-*O*-(tetraisopropylsiloxane-1,3-diyl)-1-thio-α-L-arabinofuranoside).

$R_f$= 0.58 (petroleum ether/acetone, 10 : 1). [α]24 D -43.2 (c 0.12, $CHCl_3$); $^1$H NMR (400 MHz, $CDCl_3$) δ 8.11 - 8.04 (m, 2H, Ar), 7.66 - 7.58 (m, 1H, Ar), 7.49 (dd, *J* = 10.4, 4.8 Hz, 4H, Ar), 7.13 (d, *J* = 7.9 Hz, 2H, Ar), 5.64 (dd, *J* = 5.2, 3.7 Hz, 1H, H-2), 5.51 (d, *J* = 3.7 Hz, 1H, H-1), 4.61 (dd, *J* = 7.9, 5.3 Hz, 1H, H-3), 4.27 (dt, *J* = 7.7, 3.7 Hz, 1H, H-4), 4.14 (dd, *J* = 12.6, 3.2 Hz, 1H, H-5a), 4.07 (dd, *J* = 12.7, 4.5 Hz, 1H, H-5b), 2.34 (s, 3H, tolyl $CH_3$), 1.21 - 1.07 (m, 22H, i-Pr), 1.06 - 1.01 (m, 3H, i-Pr), 1.00 - 0.94 (m, 3H, i-Pr).

$^{13}$C NMR (101 MHz, $CDCl_3$) δ 165.44 (PhC=O), 137.52 (Ar), 133.37 (Ar), 132.26 (Ar), 130.69 (Ar), 129.78 (Ar), 129.65 (Ar), 129.43 (Ar), 128.47 (Ar), 89.71 (C-1), 83.24 (C-2), 80.99 (C-4), 75.66 (C-3), 61.48 (C-5), 21.11 (tolyl $CH_3$), 17.49 (*i*-Pr), 17.37 (*i*-Pr), 17.03 (*i*-Pr), 16.96 (*i*-Pr), 16.95 (*i*-Pr), 16.92 (*i*-Pr), 13.52 (*i*-Pr), 13.23 (*i*-Pr), 12.89 (*i*-Pr), 12.53 (*i*-Pr).

HRMS (ESI) Calcd for $C_{31}H_{50}NO_6SSi_2$ $[M+NH_4]^+$: 620.2897, found: 620.2905.

(2) Compound 2-63 (104 g, 172.49 mmol, 1.0 eq) was dissolved in dry THF (200 mL). Acetic acid (23.70 mL, 413.98 mmol, 2.4 eq) and then a solution (413.98 mL) of tetrabutylammonium fluoride (413.98 mmol, 2.4 eq) in THF were added thereto under an ice bath condition. A reaction was conducted at 0°C for 30 min and then heated to room temperature and conducted for 4 h. TLC indicated the reaction was complete. A resulting reaction material was concentrated and purified by column chromatography (eluent: DCM: methanol = 40 : 1, v (volume) /v) to obtain a colorless oil, which was compound 2-64 (57.2 g, 92% yield, identified as *p*-Tolyl-2-*O*-benzoyl-1-thio-a-L-arabinofuranoside).

$R_f$= 0.58 (petroleum ether/acetone, 1 : 1). [α]24 D -107.2 (c 0.29, $CHCl_3$); $^1$H NMR (400 MHz, $CDCl_3$) δ 8.08 - 8.01 (m, 2H, Ar), 7.68 - 7.58 (m, 1H, Ar), 7.50 - 7.44 (m, 4H, Ar), 7.17 (d, *J* = 7.9 Hz, 2H, Ar), 5.70 (d, *J* = 3.1 Hz, 1H, H-1), 5.18 (t, *J* = 3.4 Hz, 1H, H-2), 4.37 (dt, *J* = 6.8, 3.3 Hz, 1H, H-4), 4.31 (dd, *J* = 7.4, 3.6 Hz, 1H, H-3), 3.98 (dd, *J* = 12.4, 2.9 Hz, 1H, H-5a), 3.83 (dd, *J* = 12.4, 3.8 Hz, 1H, H-5b), 2.36 (s, 1H, tolyl $CH_3$).

$^{13}C$ NMR (100 MHz, $CDCl_3$) δ 167.29 (Ar), 138.27 (Ar), 133.81 (Ar), 132.88 (Ar), 129.91 (Ar), 129.58 (Ar), 128.84 (Ar), 128.59 (Ar), 89.66 (C-1), 87.22 (C-2), 82.80 (C-4), 76.27 (C-3), 61.33 (C-5), 21.18 (tolyl $CH_3$).

HRMS (ESI) Calcd for $C_{19}H_{20}O_5S$ $[M+Na]^+$: 383.0929, found: 383.0923.

(3) Compound 2-64 (10 g, 27.75 mmol, 1.0 eq) was dissolved in dry DCM (60 mL). Imidazole (5.67 g, 83.24 mmol, 3.0 eq) was added thereto under an ice bath condition, and then TBSCl (4.39 g, 29.13 mmol, 1.05 eq) was added thereto in batches. A reaction was conducted at 0°C for 1 h and then heated to room temperature and conducted for 3 h. TLC indicated the reaction was complete. A resulting reaction material was diluted with DCM (100 mL), washed with water and then saturated NaCl solution, dried over anhydrous $Na_2SO_4$, filtered, and a resulting filtrate was concentrated and purified by FLASH rapid preparative chromatography (mobile phase: a mixture of petroleum ether and acetone, with a volume fraction of acetone of 10%) to obtain a white powder, which was compound 2-65 (12.1 g, 92% yield, identified as *p*-Tolyl-2-*O*-benzoyl-5-*O*-tert-butyldimethylsilyl-1-thio-α-L-arabinofuranoside).

$R_f$ = 0.72 (petroleum ether/acetonre, 1 : 1). [α]24 D -56.6 (*c* 0.20, $CHCl_3$); $^1H$ NMR (400 MHz, $CDCl_3$) δ 8.03 (dd, *J* = 8.3, 1.1 Hz, 2H, Ar), 7.66 - 7.56 (m, 1H, Ar), 7.46 (t, *J* = 7.7 Hz, 4H, Ar), 7.13 (d, *J* = 7.9 Hz, 2H, Ar), 5.64 (d, *J* = 3.7 Hz, 1H, H-1), 5.13 (t, *J* = 3.6 Hz, 1H, H-2), 4.31 - 4.23 (m, 2H, H-3, H-4), 3.91 (dd, *J* = 11.3, 3.3 Hz, 1H, H-5a), 3.85 (dd, *J* = 11.3, 3.9 Hz, 1H, H-5b), 3.34 (s, 1H, OH), 2.34 (s, 3H, $SPhCH_3$), 0.87 (s, 9H, TBS), 0.06 (s, 3H, TBS), 0.06 (s, 3H, TBS).

$^{13}C$ NMR (100 MHz, $CDCl_3$) δ 167.29 (C=O), 137.92 (Ar), 133.69 (Ar), 132.67 (Ar), 129.91 (Ar), 129.85 (Ar), 129.74 (Ar), 129.00 (Ar), 128.5 (Ar), 89.28 (C-1), 87.00 (C-2), 83.18 (C-3), 76.89 (C-4), 62.60 (C-5), 25.85 (TBS), 21.13 (tolyl $CH_3$), 18.31 (TBS), -5.33 (TBS), -5.38 (TBS).

HRMS (ESI) Calcd for $C_{25}H_{34}O_5SSi$ $[M+Na]^+$: 497.1794, found: 497.1801.

(4) Compound 2-65 (12.5 g, 26.33 mmol, 1.0 eq) and DCM (150 mL) were added to an eggplant-shaped flask. After dissolution of a resulting mixture, EDCI (1-Ethyl-3-(3-dimethylaminopropyl)carbodiimide hydrochloride)_ (10.1 g, 52.67 mmol, 2.0 eq), DMAP (3.22 g, 26.33 mmol, 1.0 eq), and levulinic acid (4.59 g, 39.50 mmol, 1.5 eq) were added thereto in sequence. A reaction was conducted at room temperature for 12 h. TLC indicated the reaction was complete. A resulting mixture was washed with saturated $NaHCO_3$ solution, 1 mol/L of hydrochloric acid, and saturated NaCl solution (three times each) in sequence, dried over anhydrous $Na_2SO_4$, filtered, and a resulting filtrate was concentrated and purified by column chromatography (eluent: petroleum ether: acetone = 20 : 1, v (volume) /v) to obtain a colorless, transparent oil, which was compound 2-66 (14 g, 93% yield, identified as *p*-Tolyl-2-*O*-benzoyl-3-*O*-levulinyl-5-*O*-tert-butyldimethylsilyl-thiol-α-L-arabinofuranoside).

$R_f$ = 0.31 (petroleum ether/ethyl acetate, 5 : 1). [α]24 D -99.7 (*c* 0.13, $CHCl_3$). $^1H$ NMR (400 MHz, $CDCl_3$) δ 8.08 - 8.00 (m, 2H, Ar), 7.63 - 7.54 (m, 1H, Ar), 7.49 - 7.39 (m, 4H, Ar), 7.14 (d, *J* = 7.9 Hz, 2H, Ar), 5.64 (d, *J* = 2.5 Hz, 1H, H-1), 5.49 (t, *J* = 2.4 Hz, 1H, H-2), 5.38 (dd, *J* = 5.0, 2.1 Hz, 1H, H-3), 4.40 (dd, *J* = 9.1, 4.1 Hz, 1H, H-5), 3.96 - 3.93 (m, 2H, H-5), 2.89 - 2.75 (m, 2H, Lev), 2.74 - 2.59 (m, 2H, Lev), 2.35 (s, 3H, tolyl $CH_3$), 2.21 (s, 3H, Lev), 0.91 (s, 9H, TBS), 0.09 (s, 3H, TBS), 0.09 (s, 3H, TBS).

$^{13}C$ NMR (101 MHz, $CDCl_3$) δ 206.18 (Lev), 171.75 (Lev), 165.37 (C=O), 137.87 (Ar), 133.45 (Ar), 132.78 (Ar), 129.91 (Ar), 129.86 (Ar), 129.74 (Ar), 129.20 (Ar), 128.41 (Ar), 90.96 (C-1), 82.98 (C-4), 82.22 (C-2), 77.31 (C-3), 62.74 (C-5), 37.93 (Lev), 29.78 (Lev), 27.98 (Lev), 25.87 (TBS), 21.14 (tolyl $CH_3$), 18.33 (TBS), -5.34 (TBS), -5.41 (TBS).

HRMS (ESI) Calcd for $C_{30}H_{40}NaO_7SSi$ $[M+Na]^+$: 595.2162, found: 595.2164.

(5) A two-necked flask, along with a stir bar, was dried by heating under vacuum. After cooling, the two-necked flask was filled with argon. 4Å molecular sieves (5 g) were added thereto, and the molecular sieves were activated by heating under vacuum with a blowtorch, and cooled to room temperature. Compound 2-66 (1 g, 1.75 mmol, 1.0 eq) dissolved in DCM (30 mL) was added to the two-necked flask, and then TTBP (867.39 mg, 3.49 mmol, 2.0 eq) and *p*-methoxybenzyl alcohol (482.43 mg, 3.49 mmol, 2.0 eq) were added thereto in sequence. A resulting mixture was stirred at room temperature for 20 min, then cooled to -78°C. AgOTf (silver trifluoromethanesulfonate, 897.13 mg, 3.49 mmol, 2.0 eq) and *p*-TolSCl (p-toluenesulfinyl chloride, 400 μL, 2.62 mmol, 1.5 eq) were added thereto. A reaction was conducted by natural heating from a starting temperature of -78°C for 3 h. TLC indicated the reaction was complete. The reaction was quenched by adding TEA (2 mL). A resulting reaction material was filtered through diatomaceous earth to remove the molecular sieves. A resulting filtrate was concentrated and purified by FLASH rapid preparative chromatography (mobile phase: a mixture of petroleum ether and ethyl acetate, with a volume fraction of ethyl acetate of 35%) to obtain a colorless oil, which was compound 2-67 (864 mg, 84% yield, identified as *p*-Methoxybenzyl-2-*O*-benzoyl-3-*O*-levulinyl-5-*O*-tert-butyldimethylsilyl-α-L-arabinofuranoside)

[α]24 D -43.0 (c 0.13, $CHCl_3$). $^1H$ NMR (400 MHz, $CDCl_3$) δ 8.07 - 8.00 (m, 2H, Ar), 7.58 (dd, *J* = 10.5, 4.3 Hz, 1H, Ar), 7.44 (t, *J* = 7.7 Hz, 2H, Ar), 7.34 (d, *J* = 8.6 Hz, 2H, Ar), 6.94 - 6.87 (m, 2H, Ar), 5.36 (d, *J* = 1.3 Hz, 1H, H-2), 5.28 (dd, *J* = 5.3, 1.1 Hz, 1H, H-3), 5.24 (s, 1H, H-1), 4.76 (d, *J* = 11.6 Hz, 1H, PMB $CH_2$), 4.55 (d, *J* = 11.6 Hz, 1H, PMB $CH_2$), 4.22 (dd, *J* = 8.8, 4.9 Hz, 1H, H-4), 3.96 (dd, *J* = 11.3, 3.6 Hz, 1H, H-5), 3.91 (dd, *J* = 11.3, 4.9 Hz, 1H, H-5), 3.82 (s, 3H, PMB $CH_3$), 2.85 - 2.71 (m, 2H, Lev), 2.70 - 2.56 (m, 2H, Lev), 2.16 (s, 3H, Lev), 0.92 (s, 9H, TBS), 0.11 (s, 6H, TBS).

$^{13}C$ NMR (101 MHz, $CDCl_3$) δ 206.22 (Lev), 171.86 (Lev), 165.40 (Lev), 159.29 (Ar), 133.36 (Ar), 129.88 (Ar), 129.59 (Ar), 129.43 (Ar), 129.33 (Ar), 128.37 (Ar), 113.83 (Ar), 104.56 (C-1), 83.13 (C-4), 82.43 (C-2), 77.23 (C-3), 68.40 (PMB $CH_2$), 62.80 (C-5), 55.27 (PMB $CH_3$), 37.87 (Lev), 29.75 (Lev), 27.94 (Lev), 25.91 (TBS), 18.40 (TBS), -5.27 (TBS),

-5.34 (TBS).

HRMS (ESI) Calcd for $C_{31}H_{46}NO_9Si$ $[M+NH_4]^+$: 604.2942, found: 604.2945.

(6) Compound 2-67 (580 mg, 988.49 μmol, 1.0 eq), tetrahydrofuran (18 mL), and methanol (2 mL) were added to an eggplant-shaped flask. After dissolution of a resulting mixture, hydrazine acetate (455.19 mg, 4.94 mmol, 5.0 eq) was added thereto. A reaction was conducted at room temperature for 4 h. TLC indicated the reaction was complete. Acetone (2 mL) was added thereto, and a resulting mixture was stirred at room temperature for 30 min to quench the reaction. A resulting reaction material was concentrated and purified by FLASH rapid preparative chromatography (mobile phase: a mixture of petroleum ether and ethyl acetate, with a volume fraction of ethyl acetate of 30%) to obtain a pale yellow oil, which was compound 2-68 (430 mg, 89% yield, identified as *p*-Methoxybenzyl-2-*O*-benzoyl-5-*O*-tert-butyldimethylsilyl-α-L-arabinofuranoside).

[α]24 D -98.5 (c 0.13, $CHCl_3$). $R_f$= 0.70 (petroleum ether/ethyl acetate, 2 : 1). $^1H$ NMR (400 MHz, $CDCl_3$) δ 8.02 - 7.97 (m, 2H, Ar), 7.62 - 7.54 (m, 1H, Ar), 7.49 - 7.38 (m, 2H, Ar), 7.33 - 7.27 (m, 2H, Ar), 6.92 - 6.85 (m, 2H, Ar), 5.29 (s, 1H, H-1), 5.15 (dd, *J* = 2.5, 0.9 Hz, 1H, H-2), 4.76 (d, *J* = 11.5 Hz, 1H, PMB $CH_2$), 4.53 (d, *J* = 11.5 Hz, 1H, PMB $CH_2$), 4.21 - 4.14 (m, 2H, H-4, H-3), 3.87 - 3.85 (m, 2H, H-5), 3.79 (s, 3H, PMB $CH_3$), 0.89 (s, 9H, TBS), 0.08 (s, 6H, TBS).

$^{13}C$ NMR (101 MHz, $CDCl_3$) δ 166.63 (Lev), 159.39 (Ar), 133.53 (Ar), 129.81 (Ar), 129.73 (Ar), 129.28 (Ar), 129.22 (Ar), 128.47 (Ar), 113.91 (Ar), 104.15 (C-1), 85.89 (C-4), 84.60 (C-2), 76.87 (C-3), 68.73 (PMB $CH_2$), 62.96 (C-5), 55.27 (PMB $CH_3$), 25.91 (TBS), 18.39 (TBS), -5.27 (TBS).

HRMS (ESI) Calcd for $C_{26}H_{40}NO_7Si$ $[M+NH_4]^+$: 506.2574, found: 506.2576.

(7) A two-necked flask, along with a stir bar, was dried by heating under vacuum. After cooling, the two-necked flask was filled with argon. 4Å molecular sieves (1 g) were added thereto, and the molecular sieves were activated by heating under vacuum with a blowtorch, and cooled to room temperature. A glycosyl donor compound 2-43 (100 mg, 151.33 μmol, 1.0 eq) was dissolved in dry DCM (10 mL) and added to the two-necked flask, and then TTBP (75.18 mg, 302.65 μmol, 2.0 eq) was added thereto. A resulting mixture was stirred at room temperature for 20 min, then cooled to -78°C. AgOTf (93.31 mg, 363.18 μmol, 2.4 eq) and *p*-TolSCl (74.60 μL, 19.21 μmol, 1.2 eq) were added thereto. A resulting mixture was stirred at -78°C for 5 min. TLC monitoring showed the complete consumption of the glycosyl donor compound 2-43, indicating pre-activation was complete. A glycosyl acceptor compound 2-68 (66.55 mg, 136.19 μmol, 0.9 eq) dissolved in dry DCM (3 mL) was slowly added dropwise to the two-necked flask. A reaction was conducted at -78°C for 20 min and then continued the reaction by naturally heating for 3 h. TLC monitoring showed the disappearance of the glycosyl acceptor compound 2-68, indicating the reaction was complete. The reaction was quenched by adding TEA (0.2 mL). A resulting reaction material was filtered through diatomaceous earth to remove the molecular sieves. A resulting filtrate was concentrated and purified by FLASH rapid preparative chromatography (mobile phase: a mixture of petroleum ether and ethyl acetate, with a volume fraction of ethyl acetate of 40%) to obtain a pale yellow oil, which was compound 2-69b (120 mg, 86% yield, identified as *p*-Methoxybenzyl-2-*O*-benzoyl-3,4,6-*O*-tri-benzyl-β-D-galactopyranosyl-(1→3)-2-*O*-benzoyl-5-*O*-tert-butyldimethylsilyl-α-L-arabinofuranoside).

$R_f$= 0.55 (toluene/acetonitrile, 15 : 1). [α]24 D -25.6 (c 0.36, $CHCl_3$).$^1H$ NMR (400 MHz, $CDCl_3$) δ 8.08 - 8.03 (m, 2H, Ar), 7.98 - 7.93 (m, 2H, Ar), 7.53 (dd, *J* = 15.8, 7.4 Hz, 2H, Ar), 7.36 (ddd, *J* = 10.9, 8.8, 6.4 Hz, 8H, Ar), 7.29 (ddd, *J* = 8.2, 6.2, 3.2 Hz, 5H, Ar), 7.23 (t, *J* = 3.7 Hz, 2H, Ar), 7.19 - 7.12 (m, 4H, Ar), 6.94 (d, *J* = 8.6 Hz, 2H, Ar), 6.72 (d, *J* = 8.7 Hz, 2H, Ar), 5.67 (dd, *J* = 10.0, 8.1 Hz, 1H, gal H-2), 5.16 (s, 1H), 5.03 - 4.95 (m, 3H, gal H-1), 4.62 (d, *J* = 11.9 Hz, 2H), 4.49 (d, *J* = 12.4 Hz, 1H, $PhCH_2$), 4.39 - 4.29 (m, 4H), 4.26 (d, *J* = 12.2 Hz, 1H, $PhCH_2$), 4.12 (dd, *J* = 8.3, 4.2 Hz, 1H), 4.02 (d, *J* = 2.6 Hz, 1H, gal H-4), 3.84 - 3.77 (m, 2H), 3.75 (s, 3H), 3.71 (dd, *J* = 10.1, 2.8 Hz, 1H, gal H-3), 3.68 - 3.59 (m, 2H), 3.51 (dd, *J* = 7.6, 3.8 Hz, 1H), 0.78 (s, 9H, TBS), -0.03 (s, 3H, TBS), -0.04 (s, 3H, TBS).

$^{13}C$ NMR (101 MHz, $CDCl_3$) δ 165.57 (C=O), 165.36 (C=O), 158.94 (Ar), 138.59 (Ar), 137.87 (Ar), 137.81 (Ar), 133.30 (Ar), 132.62 (Ar), 130.51 (Ar), 129.90 (Ar), 129.82 (Ar), 129.57 (Ar), 129.54 (Ar), 129.30 (Ar), 128.97 (Ar), 128.59 (Ar), 128.55 (Ar), 128.44 (Ar), 128.36 (Ar), 128.33 (Ar), 128.31 (Ar), 128.23 (Ar), 127.90 (Ar), 127.80 (Ar), 127.62 (Ar), 127.60 (Ar), 127.57 (Ar), 113.59 (Ar), 103.83 (ara C-1), 100.09 (gal C-1), 83.43, 82.50, 81.47, 80.27 (gal C-3), 79.16, 77.28, 74.59, 73.57, 73.46, 72.69 (gal C-4), 71.92 (gal C-2), 71.71, 68.22, 67.27, 62.10, 55.23, 25.93 (TBS), 18.38 (TBS), -5.25 (TBS), -5.35 (TBS).

HRMS (ESI) Calcd for $C_{60}H_{72}NO_{13}Si$ $[M+NH_4]^+$: 1042.4773, found: 1042.4788.

(8) Compound 2-69b (120 mg, 117.04 μmol, 1.0 eq) was dissolved in DCM (9.5 mL) and water (0.5 mL) in an eggplant-shaped flask. DDQ (53.14 mg, 234.08 μmol, 2.0 eq) was added thereto in batches. A reaction was conducted at room temperature for 8 h. TLC monitoring indicated the reaction was complete. A resulting reaction material was filtered through diatomaceous earth. A resulting filtrate was diluted with DCM (20 mL), washed with saturated $NaHCO_3$ solution and then saturated NaCl solution (three times each). A resulting organic phase was dried over anhydrous $Na_2SO_4$, filtered, and a resulting filtrate was concentrated to obtain a pale yellow oily crude product. The pale yellow oily crude product was dissolved in pyridine (10 mL). Acetic anhydride (22.13 μL, 234.08 μmol, 2.0 eq) and DMAP (1.43 mg, 11.70 μmol, 0.1 eq) were added thereto in sequence under an ice bath condition. A reaction was conducted at room temperature for 4 h. TLC monitoring indicated the reaction was complete. The pyridine was removed from a resulting reaction material by concentration under reduced pressure. A resulting residue was diluted with DCM (20 mL), washed with 1 mol/L of

hydrochloric acid, saturated $NaHCO_3$ solution, and saturated NaCl solution (three times each) in sequence. A resulting organic phase was dried over anhydrous $Na_2SO_4$, filtered, and a resulting filtrate was concentrated and purified by FLASH rapid preparative chromatography (mobile phase: a mixture of petroleum ether and ethyl acetate, with a volume fraction of ethyl acetate of 30%) to obtain a transparent oily liquid, which was compound 2-70 (72 mg, 76.02 μmol, 65% yield). The compound 2-70 was subjected to an oil pump vacuum for 1 h, then dissolved in dry DCM (10 mL). p-Toluenethiol (11.33 mg, 91.22 μmol, 1.2 eq) was added thereto, and a resulting mixture was added dropwise with $SnCl_4$ (3.56 μL, 30.41 μmol, 0.4 eq) at 0°C. A reaction was conducted at 0°C for 25 min. TLC monitoring indicated the reaction was complete. The reaction was quenched by adding TEA (0.1 mL). A resulting reaction material was concentrated and purified by FLASH rapid preparative chromatography (mobile phase: a mixture of petroleum ether and ethyl acetate, with a volume fraction of ethyl acetate of 60%) to obtain a yellow oil, which was compound 1-4 (36 mg, 53% yield, identified as *p*-Tolyl-2-*O*-benzoyl-3,4,6-*O*-tri-benzyl-β-D-galactopyranosyl-(1→3)-2-*O*-benzoyl-L-arabinofur anoside).

$R_f$= 0.22 (toluene/acetonitrile, 15 : 1). [α]24 D -32.3 (c 0.91, $CHCl_3$).$^1$H NMR (400 MHz, $CDCl_3$) δ 8.16 - 8.08 (m, 2H), 8.08 - 7.92 (m, 2H), 7.65 - 7.53 (m, 2H), 7.51 - 7.29 (m, 15H), 7.29 - 7.18 (m, 6H), 7.17 - 7.02 (m, 2H), 5.86 - 5.74 (m, 1H), 5.67 - 5.60 (m, 1H), 5.57 - 5.50 (m, 1H), 5.45 - 5.36 (m, 1H), 5.06 - 5.00 (m, 1H), 4.95 - 4.75 (m, 1H), 4.65 (m, 2H), 4.57 - 4.37 (m, 4H), 4.26 - 4.06 (m, 1H), 4.01 - 3.85 (m, 2H), 3.79 - 3.51 (m, 4H), 2.38 - 2.30 (m, 3H).

$^{13}$C NMR (101 MHz, $CDCl_3$) δ 170.31 (C=O), 166.11 (C=O), 165.52 (C=O), 165.26 (C=O), 138.29 (Ar), 137.94 (Ar), 137.83 (Ar), 137.75 (Ar), 137.64 (Ar), 137.58 (Ar), 137.40 (Ar), 133.58 (Ar), 133.43 (Ar), 133.37 (Ar), 132.86 (Ar), 132.68 (Ar), 132.11 (Ar), 132.05 (Ar), 130.62 (Ar), 130.52 (Ar), 130.34 (Ar), 130.17 (Ar), 130.02 (Ar), 129.93 (Ar), 129.86 (Ar), 129.83 (Ar), 129.81 (Ar), 129.74 (Ar), 129.64 (Ar), 129.32 (Ar), 129.22 (Ar), 128.59 (Ar), 128.53 (Ar), 128.50 (Ar), 128.41 (Ar), 128.38 (Ar), 128.35 (Ar), 128.29 (Ar), 128.14 (Ar), 128.06 (Ar), 127.98 (Ar), 127.96 (Ar), 127.80 (Ar), 127.73 (Ar), 101.09, 97.06, 91.44, 90.66, 82.81, 82.73, 82.63, 82.41, 82.19, 81.83, 80.13, 77.41, 75.11, 74.56, 74.18, 74.00, 73.80, 73.63, 73.54, 73.27, 72.67, 72.15, 71.53, 71.48, 70.52, 69.85, 68.94, 68.52, 68.37, 67.75, 67.35, 61.58, 60.73, 21.18, 21.11.

HRMS (ESI) Calcd for $C_{53}H_{56}NO_{11}S$ $[M+NH_4]^+$: 914.3574, found: 914.3586.

**Preparation Example 4**

Preparation of compound 2-81. The reaction scheme is as follows:

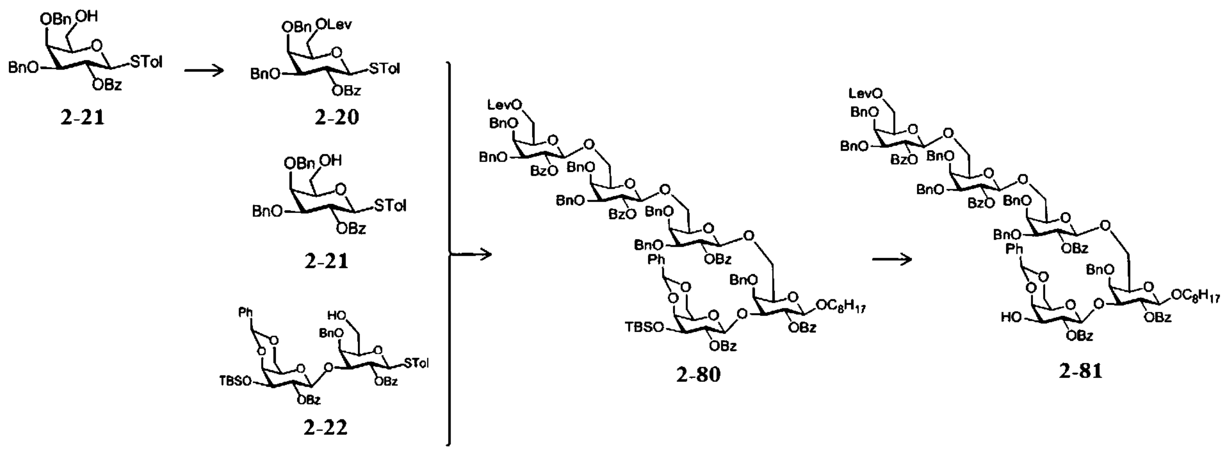

(1) Compound 2-21 (6.1 g, 9.13 mmol, 1.0 eq) was dissolved in 100 mL of dry DCM. DMAP (786 mg, 5.48 mmol, 0.6 eq), levulinic acid (2.2 mL, 18.2 mmol, 2.0 eq), and DIC (N,N'-diisopropylcarbodiimide, 3.17 mL, 17.3 mmol, 1.9 eq) were added thereto in sequence under an ice bath condition. A reaction was conducted under the ice bath for 30 min and then at room temperature for another 1 h by stirring. TLC indicated the reaction was complete. The reaction was quenched by adding saturated $NaHCO_3$ solution. A resulting mixture was diluted with DCM, washed with saturated $NaHCO_3$ solution and then saturated NaCl solution (three times each), dried over anhydrous $Na_2SO_4$, filtered, and a resulting filtrate was concentrated and purified by column chromatography (eluent: petroleum ether: ethyl acetate: DCM = 8 : 1 : 4, v (volume) /v/v) to obtain a white powder, which was compound 2-20 (5.8 g, 95.1% yield, identified as *p*-Tolyl-2-*O*-benzoyl-3-*O*-benzyl-4-*O*-benzyl-6-levulinyl-1-thio-β-D-galactopyranoside).

$R_f$= 0.30 (toluene/acetonitrile, 20 : 1). [α]24 D +41.0 (c 0.16, $CHCl_3$); $^1$H NMR (400 MHz, $CDCl_3$) δ 8.02 (d, *J* = 7.2 Hz, 2H, Ar), 7.59 (t, *J* = 7.4 Hz, 1H, Ar), 7.46 (t, *J* = 7.7 Hz, 2H, Ar), 7.39 - 7.27 (m, 7H, Ar), 7.23 - 7.11 (m, 5H, Ar), 7.02 (d, *J* = 8.0 Hz, 2H, Ar), 5.66 (t, *J* = 9.7 Hz, 1H, H-2), 5.01 (d, *J* = 11.6 Hz, 1H, $PhCH_2$), 4.70 (d, *J* = 9.9 Hz, 1H, H-1), 4.69 - 4.61 (m,

2H, $PhCH_2$), 4.55 (d, *J* = 12.2 Hz, 1H, $PhCH_2$), 4.32 (dd, *J* = 11.1, 6.8 Hz, 1H, H-6a), 4.17 (dd, *J* = 11.2, 5.8 Hz, 1H, H-6b), 3.97 (d, *J* = 2.0 Hz, 1H, H-4), 3.74 - 3.63 (m, 1H, H-3 H-5), 2.76 - 2.70 (m, 2H, Lev), 2.55 - 2.47 (m, 2H, Lev), 2.29 (s, 3H, Lev), 2.18 (s, 1H, tolyl $CH_3$).

$^{13}C$ NMR (101 MHz, $CDCl_3$) δ 206.53 (C=O, Lev), 172.43 (C=O, Lev), 165.25 (C=O), 138.19 (Ar), 137.73 (Ar), 137.47 (Ar), 133.04 (Ar), 132.72 (Ar), 130.13 (Ar), 129.91 (Ar), 129.63 (Ar), 129.47 (Ar), 128.36 (Ar), 128.28 (Ar), 128.21 (Ar), 127.80 (Ar), 127.78 (Ar), 127.65 (Ar), 87.12 (C-1), 81.11 (C-3), 76.07 (C-5) 74.22 (PhC), 72.31 (C-4), 72.00 (PhC), 70.30 (C-2), 63.32 (C-6), 37.95 (Lev), 29.84 (Lev), 27.85 (Lev), 21.14 (tolyl $CH_3$).

HRMS (ESI) Calcd for $C_{39}H_{44}NO_8S$ $[M+NH_4]^+$: 686.2788, found: 686.2791.

(2) Compound 2-46 (5 g, 8.41 mmol, 1.0 eq) was dissolved in 90 mL of dry THF and 10 mL of dry DMF. NapBr (3.7 g, 16.8 mmol, 2.0 eq) was added thereto under an ice bath condition, and then a solution of sodium hydride (500 mg, 12.6 mmol, 1.5 eq, 60 wt% in mineral oil) in mineral oil was added thereto in batches. A reaction was conducted at 0°C for 5 h. TLC indicated the reaction was complete. The reaction was quenched by adding saturated $NH_4Cl$ solution, a resulting mixture was extracted with DCM, and a resulting combined organic phases were dried over anhydrous $Na_2SO_4$, filtered, and a resulting filtrate was concentrated and purified by FLASH rapid preparative chromatography (mobile phase: a mixture of petroleum ether and ethyl acetate, with a volume fraction of ethyl acetate of 25%) to obtain a white powder, which was compound 2-47 (5.6 g, 91% yield, identified as *p*-Tolyl-2-*O*-benzoyl-3-*O*-tert-butyldimethylsilyl-4-*O*-benzyl-6-*O*-(2-naphthylmethyl)-1-thio-β-D-galactopyranoside).

$R_f$= 0.52 (petroleum ether/ethyl acetate, 4 : 1). $^1H$ NMR (400 MHz, $CDCl_3$) δ 8.07 - 8.05 (m, 1H, Ar), 8.05 - 8.03 (m, 1H, Ar), 7.85 - 7.78 (m, 3H, Ar), 7.73 (s, 1H, Ar), 7.59 - 7.54 (m, 1H, Ar), 7.51 - 7.39 (m, 5H, Ar), 7.34 (d, *J* = 8.1 Hz, 2H, Ar), 7.29 - 7.23 (m, 5H, Ar), 6.95 (d, *J* = 8.0 Hz, 2H, Ar), 5.61 (t, *J* = 9.3 Hz, 1H, H-2), 5.06 (d, *J* = 11.4 Hz, 1H, $PhCH_2$), 4.72 (d, *J* = 9.8 Hz, 1H, H-1), 4.65 (d, *J* = 11.9 Hz, 1H, $NapCH_2$), 4.59 (d, *J* = 11.9 Hz, 1H, Nap $CH_2$), 4.50 (d, *J* = 11.4 Hz, 1H, $PhCH_2$), 3.96 (d, *J* = 8.6 Hz, 1H, H-3), 3.84 (d, *J* = 2.5 Hz, 1H, H-4), 3.80 - 3.66 (m, 3H, H-5 H-6a H-6b), 2.24 (s, 3H, tolyl $CH_3$), 0.76 (s, 9H, TBS), 0.09 (s, 3H, TBS), -0.11 (s, 3H, TBS).

$^{13}C$ NMR (100 MHz, $CDCl_3$) δ 165.21 (C=O), 138.74 (Ar), 137.48 (Ar), 135.48 (Ar), 133.29 (Ar), 133.06 (Ar), 132.91 (Ar), 132.50 (Ar), 130.45 (Ar), 129.96 (Ar), 129.84 (Ar), 129.47 (Ar), 128.30 (Ar), 128.23 (Ar), 128.16 (Ar), 127.94 (Ar), 127.72 (Ar), 127.69 (Ar), 127.37 (Ar), 126.72 (Ar), 126.16 (Ar), 125.96 (Ar), 125.89 (Ar), 87.32 (C-1), 77.77 (C-5), 77.25 (C-4), 75.82 (C-3) 75.04 ($NapCH_2$), 73.70 ($PhCH_2$), 71.25 (C-2), 69.02 (C-6), 25.55 (TBS), 21.11 (tolyl $CH_3$), 17.81 (TBS), -4.05 (TBS), -5.06 (TBS).

HRMS (ESI) Calcd for $C_{44}H_{54}NO_6SSi$ $[M+NH_4]^+$: 752.3441, found: 752.3443.

(3) Compound 2-47 (10 g, 13.6 mmol, 1.0 eq) was dissolved in 200 mL of anhydrous acetonitrile. Boron trifluoride diethyl etherate (3.36 mL, 27.2 mmol, 2.0 eq) was added dropwise thereto under an ice bath condition. A reaction was conducted at 0°C for 5 min. TLC indicated the reaction was complete. The reaction was quenched by adding TEA (4 mL). The solvent was removed by concentration under reduced pressure. A resulting residue was dissolved in 200 mL of DCM, washed with water and then saturated NaCl solution (three times each). A resulting combined organic phases were dried over anhydrous $Na_2SO_4$, filtered, and a resulting filtrate was concentrated and purified by column chromatography (eluent: petroleum ether: ethyl acetate: DCM = 4 : 1 : 4, v (volume) /v/v) to obtain a white powder, which was compound 2-48 (8.1 g, 96% yield, identified as *p*-Tolyl-2-*O*-benzoyl-4-*O*-benzyl-6-*O*-(2-naphthylmethyl)-1-thio-β-D-galactopyranoside).

$R_f$= 0.14 (petroleum ether/ethyl acetate, 4 : 1). [α]24 D +31.9 (c 0.14, $CHCl_3$); $^1H$ NMR (400 MHz, $CDCl_3$) δ 8.05 (d, *J* = 7.4 Hz, 2H, Ar), 7.85 - 7.77 (m, 3H, Ar), 7.75 (s, 1H, Ar), 7.54 (t, *J* = 7.4 Hz, 1H, Ar), 7.48 - 7.45 (m, 2H, Ar), 7.42 (dd, *J* = 10.6, 4.6 Hz, 3H, Ar), 7.35 (d, *J* = 8.0 Hz, 2H, Ar), 7.29 - 7.21 (m, 5H, Ar), 6.98 (d, *J* = 8.0 Hz, 2H, Ar), 5.24 (t, *J* = 9.7 Hz, 1H, H-2), 4.73 (d, *J* = 10.1 Hz, 2H, H-1, $NapCH_2$), 4.69 - 4.58 (m, 3H, 2×$PhCH_2$, $NapCH_2$ ), 3.95 (d, *J* = 3.2 Hz, 1H, H-4), 3.81 (dd, *J* = 9.2, 3.1 Hz, 1H, H-3), 3.78 - 3.67 (m, 3H, H-5, H-6a, H-6b), 2.61 (d, *J* = 9.2 Hz, 1H,OH), 2.23 (d, *J* = 9.4 Hz, 3H, tolyl $CH_3$).

$^{13}C$ NMR (101 MHz, $CDCl_3$) δ 166.68 (Ar), 138.19 (Ar), 137.99 (Ar), 135.29 (Ar), 133.33 (Ar), 133.12 (Ar), 133.04 (Ar), 130.03 (Ar), 129.85 (Ar), 129.65 (Ar), 129.00 (Ar), 128.50 (Ar), 128.46 (Ar), 128.34 (Ar), 127.98 (Ar), 127.82 (Ar), 127.80 (Ar), 127.69 (Ar), 126.78 (Ar), 126.29 (Ar), 126.09 (Ar), 125.89 (Ar), 86.38 (C-1), 77.67 (C-5), 76.80 (C-4), 75.34 ($NapCH_2$), 74.45 (C-3), 73.73 ($PhCH_2$), 72.38 (C-2), 68.59 (C-6), 21.21 (tolyl $CH_3$).

HRMS (ESI) Calcd for $C_{38}H_{40}NO_6S$ $[M+NH_4]^+$: 638.2576, found: 638.2584.

(4) A two-necked flask, along with a stir bar, was dried by heating under vacuum. After cooling to room temperature, the two-necked flask was filled with argon. 4Å molecular sieves (15 g) were added thereto, and the molecular sieves were activated by heating under vacuum with a blowtorch, cooled to room temperature, and the two-necked flask was filled with argon. Compound 2-45 (3 g, 5.06 mmol, 1.0 eq) and dry DCM (100 mL) were added to the two-necked flask in sequence. A resulting mixture was stirred at room temperature for 20 min, then cooled to -78°C and stirred for another 5 min. AgOTf (2.6 g, 10.12 mmol, 2.0 eq) and *p*-TolSCl (698.10 μL, 1.0 eq) were added thereto in sequence. A resulting mixture was stirred at -78°C for 5 min. TLC showed the complete disappearance of a glycosyl donor compound 2-45, indicating pre-activation was complete. A glycosyl acceptor compound 2-48 (3.14 g, 5.06 mmol, 1.0 eq), dissolved in dry DCM (15 mL), was added dropwise to the two-necked flask. A reaction was conducted at -78°C for 10 min and then naturally heated to continue the reaction for another 3 h. TLC showed the glycosyl acceptor compound 2-48 was

completely consumed, indicating the reaction was complete. The reaction was quenched by adding TEA (3 mL). A resulting reaction material was filtered through diatomaceous earth to remove the molecular sieves. A resulting filtrate was concentrated and purified by FLASH rapid preparative chromatography (mobile phase: a mixture of petroleum ether, ethyl acetate, and DCM, with a volume fraction of ethyl acetate of 30% and a volume fraction of DCM of 15%) to obtain a white powder, which was compound 2-48-2 (3.9 g, 71% yield, identified as *p*-Tolyl-2-*O*-benzoyl-3-*O*-tert-butyldimethylsilyl-4,6-*O*-benzylidene-β-D-galactopyranosyl-(1-3) -2-*O*-benzoyl-4-*O*-benzyl-6-*O*-(2-naphthylmethyl)-1-thio-β-D-galactopyranoside).

$R_f$= 0.36 (petroleum ether/ethyl acetate, 2 : 1). [α]24 D +43.6 (*c* 0.10, $CHCl_3$); $^1H$ NMR (400 MHz, $CDCl_3$) δ 7.80 - 7.72 (m, 5H, Ar), 7.68 (d, *J* = 7.0 Hz, 3H, Ar), 7.54 - 7.48 (m, 3H, Ar), 7.46 - 7.39 (m, 3H, Ar), 7.37 - 7.24 (m, 10H, Ar), 7.17 (t, *J* = 7.8 Hz, 2H, Ar), 7.13 - 7.08 (m, 3H, Ar), 6.88 (d, *J* = 8.0 Hz, 2H, Ar), 5.58 - 5.47 (m, 3H, $PhCHO_2$, H-2[a], H-2[b]), 5.11 (d, *J* = 11.9 Hz, 1H, $NapCH_2$), 4.82 (d, *J* = 7.9 Hz, 1H, H-1[b]), 4.71 (d, *J* = 9.9 Hz, 1H, H-1[a]), 4.68 (d, *J* = 11.8 Hz, 1H, $NapCH_2$), 4.55 (d, *J* = 11.8 Hz, 1H, $PhCH_2$), 4.46 (d, *J* = 11.8 Hz, 1H, $PhCH_2$), 4.31 (d, *J* = 12.0 Hz, 1H, H-6a[a]), 4.19 (d, *J* = 2.5 Hz, 1H, H-4[a]), 4.14 - 4.06 (m, 2H, H-3[a], H-5[b]), 4.07 - 3.99 (m, 2H, H-4[b], H-6b[b]), 3.85 (dd, *J* = 9.6, 2.8 Hz, 1H, H-3[b]), 3.74 - 3.65 (m, 2H, H-5[a], H-6a[a]), 3.50 (dd, *J* = 12.1, 7.9 Hz, 1H, H-6b[a]), 2.17 (s, 3H, tolyl $CH_3$), 0.66 (s, 9H, TBS), -0.07 (s, 3H, TBS), -0.26 (s, 3H, TBS).

$^{13}C$ NMR (101 MHz, $CDCl_3$) δ 164.94 (Ar), 164.61 (Ar), 138.78 (Ar), 137.86 (Ar), 137.36 (Ar), 135.72 (Ar), 133.33 (Ar), 133.04 (Ar), 132.79 (Ar), 132.40 (Ar), 132.11 (Ar), 130.52 (Ar), 130.04 (Ar), 130.01 (Ar), 129.74 (Ar), 129.64 (Ar), 129.52 (Ar), 128.81 (Ar), 128.25 (Ar), 128.16 (Ar), 127.98 (Ar), 127.71 (Ar), 127.16 (Ar), 126.54 (Ar), 126.25 (Ar), 126.04 (Ar), 125.92 (Ar), 125.83 (Ar), 101.94 (C-1[b]), 101.03 ($PhCHO_2$), 87.64 (C-1[a]), 80.81 (C-5[b]), 78.31 (C-5[a]), 76.43 (C-4[b]), 75.78 (C-4[a]), 74.51 ($NapCH_2$), 73.57 ($PhCH_2$), 72.31 (C-2[b]), 71.94 (C-3[b]), 70.81 (C-2[a]), 69.70 (C-6[a]), 69.10 (C-6[b]), 25.42 (TBS), 21.06 (tolyl $CH_3$), 17.85 (TBS), -4.60 (TBS), -4.84 (TBS).

HRMS (ESI) Calcd for $C_{64}H_{72}NO_{12}SSi$ $[M+NH_4]^+$: 1106.4544, found: 1106.4563.

(5) Compound 2-48-2 (4 g, 3.67 mmol, 1.0 eq) was dissolved in 60 mL of DCM. $H_2O$ (3 mL) was added thereto, and a resulting mixture was stirred under an ice bath for 10 min. DDQ (1.67 g, 7.34 mmol, 2.0 eq) and β-pinene (1.17 mL, 7.34 mmol, 2.0 eq) were added thereto in batches. A reaction was conducted at 0°C for 10 min and then heated to room temperature and conducted for 12 h. TLC indicated the reaction was complete. The reaction was quenched by adding saturated $NaHCO_3$ solution (5 mL). A resulting reaction material was filtered through diatomaceous earth. A resulting filtrate was diluted with 100 mL of DCM, washed with water three times, then washed once with saturated NaCl solution, dried over anhydrous $Na_2SO_4$, filtered, and a resulting filtrate was concentrated and purified by FLASH rapid preparative chromatography (mobile phase: a mixture of petroleum ether, ethyl acetate, and DCM, with a volume fraction of ethyl acetate of 50% and a volume fraction of DCM of 15%) to obtain a white powder, which was compound 2-22 (3.71 g, 78% yield, identified as *p*-Tolyl-2-*O*-benzoyl-3-*O*-tert-butyldimethylsilyl-4,6-*O*-benzylidene-β-D-galactopyranosyl-(1→3)-2-*O*-benzoyl-4-*O*-benzyl-1-thio-β-D-galactopyranoside).

$R_f$= 0.18 (petroleum ether/ethyl acetate, 2 : 1). [α]24 D +12.0 (c 0.15, $CHCl_3$); $^1H$ NMR (400 MHz, $CDCl_3$) δ 7.77 - 7.73 (m, 2H, Ar), 7.70 - 7.66 (m, 2H, Ar), 7.58 - 7.50 (m, 3H, Ar), 7.45 (t, *J* = 7.4 Hz, 1H, Ar), 7.37 (dt, *J* = 13.0, 4.7 Hz, 4H, Ar), 7.31 - 7.26 (m, 3H, Ar), 7.20 (dt, *J* = 12.2, 6.7 Hz, 7H, Ar), 6.98 (d, *J* = 8.0 Hz, 2H, Ar), 5.59 - 5.46 (m, 3H, $PhCHO_2$, H-2[a], H-2[b]), 5.04 (d, *J* = 12.0 Hz, 1H, $PhCH_2$), 4.79 (m, 2H, H-1[b], $PhCH_2$), 4.68 (d, *J* = 10.0 Hz, 1H, H-1[a]), 4.35 (d, *J* = 11.4 Hz, 1H, H-6a[b]), 4.15 - 4.02 (m, 4H, H-6b[b], H-3[a], H-5[b], H-4[b]), 3.93 - 3.83 (m, 1H, H-3[b]), 3.72 (dd, *J* = 11.4, 6.6 Hz, 1H, H-6a[a]), 3.52 - 3.45 (m, 2H, H-4[a], H-5[a]), 3.40 (dd, *J* = 11.2, 4.9 Hz, 1H, H-6b[a]), 2.25 (s, 3H, tolyl $CH_3$), 0.67 (s, 9H, TBS), -0.05 (s, 3H, TBS), -0.25 (s, 3H, TBS).

$^{13}C$ NMR (101 MHz, $CDCl_3$) δ 164.94 (PhC=O), 164.53 (PhC=O), 138.31 (Ar), 137.71 (Ar), 137.60 (Ar), 132.78 (Ar), 132.40 (Ar), 132.24 (Ar), 130.14 (Ar), 129.98 (Ar), 129.94 (Ar), 129.75 (Ar), 129.62 (Ar), 129.55 (Ar), 129.27 (Ar), 128.82 (Ar), 128.25 (Ar), 128.21 (Ar), 128.12 (Ar), 127.97 (Ar), 127.59 (Ar), 126.13 (Ar), 101.98 ($PhCHO_2$), 101.01 (C-1[b]), 87.64 (C-1[a]), 80.66 (C-5[b]), 78.96 (C-5[a]), 76.43 (C-4[b]), 74.42 (C-3[a]), 74.04 (PhC), 72.30 (C-2[b]), 71.83 (C-3[b]), 70.79 (C-2[a]), 69.04 (C-6[b]), 66.82 (C-4[a]), 61.99 (C-6[a]), 25.35 (TBS), 21.07 (tolyl $CH_3$), 17.81 (TBS), -4.63 (TBS), -4.89 (TBS).

HRMS (ESI) Calcd for $C_{53}H_{64}NO_{12}SSi$ $[M+NH_4]^+$: 966.3918, found: 966.3947.

(6) A two-necked flask, along with a stir bar, was dried by heating under vacuum. After cooling, the two-necked flask was filled with argon. 4Å molecular sieves (6 g) were added thereto, and the molecular sieves were activated by heating under vacuum with a blowtorch, and cooled to room temperature. A glycosyl donor compound 2-20 (200 mg, 299.04 μmol, 1.0 eq), TTBP (594.29 mg, 2.39 mmol, 8.0 eq), and dry DCM (30 mL) were added thereto in sequence. A resulting mixture was stirred at room temperature for 20 min, then cooled to -78°C. AgOTf (153.67 mg, 598.09 μmol, 2.0 eq) and *p*-TolSCl (40.20 μL, 299.04 μmol, 1.0 eq) were added thereto. A resulting mixture was stirred at -78°C for 5 min. TLC monitoring showed the complete disappearance of the glycosyl donor compound 2-20, indicating pre-activation was complete. A glycosyl acceptor compound 2-21 (157.01 mg, 275.12 μmol, 0.92 eq), dissolved in dry DCM (8 mL), was added dropwise to the two-necked flask. A reaction was conducted at -78°C for 10 min and then naturally heated to continue the reaction for another 1 h. TLC monitoring showed the disappearance of the glycosyl acceptor compound 2-21, indicating the reaction was complete. A resulting reaction material containing a disaccharide donor was quickly heated to room temperature and stirred for 20 min, then cooled to -78°C. AgOTf (141.37 mg, 550.24 μmol, 1.84 eq) and *p*-TolSCl

(36.99 μL, 275.12 μmol, 0.92 eq) were added thereto. A resulting mixture was stirred at -78°C for 5 min. TLC monitoring showed the complete disappearance of the disaccharide donor, indicating pre-activation was complete. The glycosyl acceptor compound 2-21 (145.06 mg, 254.19 μmol, 0.85 eq), dissolved in dry DCM (8 mL), was added dropwise to the two-necked flask. A reaction was conducted at -78°C for 10 min and then naturally heated to continue the reaction for another 1 h. TLC showed the disappearance of the glycosyl acceptor compound 2-21, indicating the reaction was complete. A resulting reaction material containing a trisaccharide donor was quickly heated to room temperature and stirred for 20 min, then cooled to -78°C. AgOTf (130.62 mg, 508.37 μmol, 1.7 eq) and *p*-TolSCl (34.17 μL, 254.19 μmol, 0.85 eq) were added thereto. A resulting mixture was stirred at -78°C for 5 min. TLC showed the complete disappearance of the trisaccharide donor, indicating pre-activation was complete. A glycosyl acceptor compound 2-22 (219.95 mg, 230.26 μmol, 0.77 eq), dissolved in dry DCM (8 mL), was added dropwise to the two-necked flask. A reaction was conducted at -78°C for 10 min and then naturally heated to continue the reaction for another 3 h. TLC indicated the reaction was complete. A resulting reaction material containing a pentasaccharide donor was cooled to -78°C. AgOTf (118.32 mg, 460.53 μmol, 1.54 eq) and *p*-TolSCl (31.76 μL, 230.26 μmol, 0.77 eq) were added thereto. A resulting mixture was stirred at -78°C for 5 min. TLC showed the complete disappearance of the pentasaccharide donor, indicating pre-activation was complete. n-Octanol (0.48 mL, 2.99 mmol, 10.0 eq) was added dropwise to the two-necked flask. A reaction was conducted at -78°C for 10 min and then naturally heated to continue the reaction for another 2 h. TLC indicated the reaction was complete. The reaction was quenched by adding TEA (2 mL). A resulting reaction material was filtered through diatomaceous earth. A resulting filtrate was concentrated and purified by FLASH rapid preparative chromatography (mobile phase: a mixture of petroleum ether, ethyl acetate, and DCM, with a volume fraction of ethyl acetate of 60% and a volume fraction of DCM of 15%) to obtain a white foamy powder, which was compound 2-80 (452 mg, 63% yield, identified as Octyl-2-*O*-benzoyl-3,4-*O*-benzyl-5-levulinyl-β-D-galactopyranosyl-(1→6)-2-*O*-benzoyl-3,4-*O*-b enzyl-β-D-galactopyranosyl-(1→6)-2-*O*-benzoyl-3,4-*O*-benzyl-β-D-galactopyranosyl-(1→6)-[2-*O*-benzoyl-3-*O*-tert-butyldimethylsilyl-4,6-*O*-benzylidene-β-D-galactopyranosyl]-(1→3)-2-*O*-be nzoyl-4-*O*-benzyl-1-thio-β-D-galactopyranoside).

$R_f$= 0.62 (toluene/acetonitrile, 6 : 1). [α]24 D +32.1 (c 0.25, $CHCl_3$); $^1$H NMR (600 MHz, $CDCl_3$) δ 8.01 (d, *J* = 7.2 Hz, 2H, Ar), 7.97 (d, *J* = 7.2 Hz, 2H, Ar), 7.91 - 7.89 (m, 2H, Ar), 7.67 - 7.62 (m, 4H, Ar), 7.55 - 7.49 (m, 3H, Ar), 7.49 - 7.45 (m, 3H, Ar), 7.44 - 7.37 (m, 11H, Ar), 7.35 (dd, *J* = 8.4, 3.8 Hz, 3H, Ar), 7.34 - 7.30 (m, 4H, Ar), 7.30 - 7.25 (m, 7H, Ar), 7.19 - 7.15 (m, 5H, Ar), 7.12 - 7.08 (m, 5H, Ar), 7.05 (dd, *J* = 16.2, 8.2 Hz, 7H, Ar), 7.00 - 6.97 (m, 2H, Ar), 5.68 (dd, *J* = 10.0, 8.0 Hz, 1H, H-2), 5.53 (dd, *J* = 10.0, 8.0 Hz, 1H, H-2), 5.49 (s, 1H, $PhCHO_2$), 5.43 (dd, *J* = 9.9, 7.9 Hz, 2H, H-2 × 2), 5.33 (dd, *J* = 9.9, 8.1 Hz, 1H, H-2), 5.05 (d, *J* = 11.6 Hz, 1H, $PhCH_2$), 5.01 (d, *J* = 11.9 Hz, 1H, $PhCH_2$), 4.90 (d, *J* = 11.2 Hz, 1H, $PhCH_2$), 4.78 (d, *J* = 11.5 Hz, 1H, $PhCH_2$), 4.74 - 4.56 (m, 8H, H-1, $PhCH_2$ × 7), 4.49 (d, *J* = 7.9 Hz, 1H, H-1), 4.39 (d, *J* = 12.3 Hz, 1H, $PhCH_2$), 4.37 (d, *J* = 7.9 Hz, 1H, H-1),4.30 - 4.20 (m, 6H, H-1, H-6 × 3, $PhCH_2$ × 2), 4.17 - 4.10 (m, 2H, H-1, H-6), 4.07 - 3.97 (m, 5H, H-6 × 2, $PhCH_2$), 3.96 - 3.94 (m, 1H), 3.91 (s, 1H), 3.87 (d, *J* = 1.8 Hz, 2H), 3.85 (s, 1H), 3.77 (dd, *J* = 10.2, 2.4 Hz, 1H), 3.68 (dd, *J* = 10.2, 2.5 Hz, 1H), 3.65 (t, *J* = 6.5 Hz, 1H), 3.60 - 3.56 (m, 2H, H-6 ×2), 3.48 - 3.43 (m, 3H, H-6 × 2), 3.43 - 3.39 (m, 2H), 3.39 - 3.34 (m, 2H, linker), 3.32 - 3.24 (m, 2H), 2.94 (ddd, *J* = 9.8, 7.7, 5.5 Hz, 1H, linker), 2.67 - 2.60 (m, 2H, Lev), 2.43 - 2.37 (m, 2H, Lev), 2.12 (s, 3H, Lev), 1.12 (dt, *J* = 14.5, 7.2 Hz, 3H, linker), 1.03 - 0.96 (m, 3H, linker), 0.89 - 0.84 (m, 4H, linker), 0.79 (t, *J* = 7.3 Hz, 5H, linker), 0.65 (s, 9H, TBS), -0.06 (s, 3H, TBS), -0.26 (s, 3H, TBS).

$^{13}$C NMR (151 MHz, $CDCl_3$) δ 206.47 (Lev), 172.33 (Lev), 165.21 (PhC=O), 165.07 (PhC=O), 165.03 (PhC=O), 164.69 (PhC=O), 164.12 (PhC=O), 138.53 (Ar), 138.43 (Ar), 138.40 (Ar), 138.04 (Ar), 137.77 (Ar), 137.56 (Ar), 137.56 (Ar), 137.32 (Ar), 134.36 (Ar), 133.21 (Ar), 133.01 (Ar), 132.66 (Ar), 132.30 (Ar), 132.11 (Ar), 130.11 (Ar), 129.97 (Ar), 129.83 (Ar), 129.79 (Ar), 129.58 (Ar), 129.40 (Ar), 129.35 (Ar), 129.32 (Ar), 128.94 (Ar), 128.88 (Ar), 128.80 (Ar), 128.58 (Ar), 128.52 (Ar), 128.44 (Ar), 128.36 (Ar), 128.27 (Ar), 128.24 (Ar), 128.17 (Ar), 128.08 (Ar), 128.05 (Ar), 128.02 (Ar), 127.96 (Ar), 127.93 (Ar), 127.87 (Ar), 127.83 (Ar), 127.74 (Ar), 127.73 (Ar), 127.70 (Ar), 127.67 (Ar), 127.65 (Ar), 127.37 (Ar), 127.36 (Ar), 127.27 (Ar), 127.20 (Ar), 127.02 (Ar), 127.00 (Ar), 126.20 (Ar), 102.12 (C-1), 101.50 (C-1), 101.35 (C-1), 101.12 (C-1), 101.00 ($PhCHO_2$), 100.95 (C-1), 80.11, 79.56, 79.42, 76.24, 75.29, 74.60 ($PhCH_2$), 74.37 ($PhCH_2$), 74.36 ($PhCH_2$), 74.05 ($PhCH_2$), 73.02, 72.45, 72.16, 72.02, 71.83 ($PhCH_2$), 71.79 (C-2 × 2), 71.77 (C-2), 71.73 (C-2 × 2), 71.42, 71.14 ($PhCH_2$), 70.90 ($PhCH_2$), 69.47 (C-6), 68.89 (linker), 68.82 (C-6), 66.97 (C-6), 66.51, 65.75 (C-6), 62.79 (C-6), 37.75 (Lev), 31.56 (linker), 29.65 (Lev), 29.02 (linker), 28.85 (linker), 27.67 (Lev), 25.54 (linker), 25.24 (TBS), 22.45 (linker), 17.67 (TBS), 13.95 (linker), -4.78 (TBS), -5.00 (TBS).

MALDI-TOF MS Calcd for $C_{140}H_{154}KO_{33}S$ $[M+K]^+$ m/z: 2429.9779, found: 2429.8745.

(7) A polytetrafluoroethylene (PTFE)-lined reaction vessel was charged with compound 2-80 (1.0 g, 417.92 μmol, 1.0 eq) and THF (20 mL). A resulting mixture was stirred under an ice bath for 10 min. A solution (2 mL) of hydrogen fluoride (5.0 eq) in pyridine was added dropwise thereto in batches. A reaction was conducted at 35°C for 10 h by stirring. TLC indicated the reaction was complete. The reaction was quenched by adding TEA (5 mL). The THF was removed by concentration under reduced pressure. A resulting residue was dissolved in 30 mL of DCM, washed with saturated $NaHCO_3$ solution and then saturated NaCl solution (three times each), dried over anhydrous $Na_2SO_4$, filtered, and a resulting filtrate was concentrated and purified by FLASH rapid preparative chromatography (mobile phase: a mixture of petroleum ether, ethyl acetate, and DCM, with a volume fraction of ethyl acetate of 70% and a volume fraction of DCM of 15%) to obtain a white foamy powder, which was compound 2-81 (820 mg, 86% yield, identified as Octyl-2-*O*-benzoyl-3,4-*O*-benzyl-5-levulinyl-β-D-galactopyranosyl-(1→6)-2-*O*-benzoyl-3,4-*O*-b enzyl-β-D-galactopyranosyl-(1→6)-2-*O*-benzoyl-3,4-*O*-benzyl-β-D-galactopyranosyl-(1→6)-[2-*O*-benzoyl-4,6-*O*-benzylidene-β-D-galactopyranosyl]-(1→3)-2-*O*-benzoyl-4-*O*-benzyl-1-thio-β-D-galactopyranoside).

$R_f$= 0.19 (toluene/acetonitrile, 5 : 1). [α]24 D +24.5 (c 0.35, $CHCl_3$); $^1$H NMR (600 MHz, $CDCl_3$) δ 8.01 (dd, *J* = 8.4, 7.3 Hz, 2H, Ar), 7.96 (dd, *J* = 7.2, 6.0 Hz, 2H, Ar), 7.91 (dd, *J* = 8.8, 7.5 Hz, 2H, Ar), 7.71 - 7.67 (m, 2H, Ar), 7.66 (dd, *J* = 8.2, 1.1 Hz, 2H, Ar), 7.55 - 7.52 (m, 3H, Ar), 7.48 (t, *J* = 7.4 Hz, 1H, Ar), 7.43 (dd, *J* = 7.9, 3.7 Hz, 3H, Ar), 7.38 (dd, *J* = 8.6, 7.4 Hz, 8H, Ar), 7.33 - 7.28 (m, 10H, Ar), 7.23 - 7.21 (m, 3H, Ar), 7.18 (ddd, *J* = 12.1, 6.8, 4.5 Hz, 8H, Ar), 7.11 - 7.05 (m, 12H, Ar), 7.01 - 6.99 (m, 2H, Ar), 5.66 (dd, *J* = 10.0, 7.9 Hz, 1H, H-2), 5.50 - 5.46 (m, 2H, $PhCHO_2$ , H-2), 5.43 (dd, *J* = 10.0, 7.9 Hz, 1H, H-2), 5.37 - 5.30 (m, 2H, H-2 × 2), 5.05 (dd, *J* = 11.7, 5.4 Hz, 2H, $PhCH_2$ × 2), 4.88 (d, *J* = 11.2 Hz, 1H, $PhCH_2$), 4.78 (d, *J* = 11.4 Hz, 1H, $PhCH_2$), 4.74 - 4.65 (m, 6H, H-1, $PhCH_2$ × 5), 4.62 (d, *J* = 11.4 Hz, 1H, $PhCH_2$), 4.58 (d, *J* = 12.3 Hz, 1H, $PhCH_2$), 4.46 (d, *J* = 7.9 Hz, 1H, H-1), 4.40 (d, *J* = 12.3 Hz, 1H, $PhCH_2$), 4.32 (d, *J* = 12.4 Hz, 1H, $PhCH_2$), 4.30 - 4.25 (m, 3H, H-1 × 2, H-6), 4.23 - 4.20 (m, 2H, H-6 × 2), 4.17 (d, *J* = 8.0 Hz, 1H, H-1), 4.14 - 4.11 (m, 2H, $PhCH_2$ × 2), 4.09 - 4.05 (m, 1H, H-6), 4.00 - 3.97 (m, 4H, H-6 × 2), 3.93 (d, *J* = 2.3 Hz, 1H), 3.88 (d, *J* = 2.3 Hz, 1H), 3.79 (dd, *J* = 10.1, 2.7 Hz, 1H), 3.69 (ddd, *J* = 12.8, 10.2, 3.0 Hz, 2H), 3.64 (t, *J* = 6.3 Hz, 1H), 3.60-3.51 (m, 3H, H-6 × 3), 3.46 (s, 1H), 3.44 - 3.38 (m, 5H, H-6, linker), 3.29 (dd, *J* = 9.9, 1.9 Hz, 2H), 3.00 - 2.95 (m, 1H, linker), 2.68 - 2.65 (m, 2H, Lev), 2.43 - 2.40 (m, 2H, Lev), 2.14 (s, 3H, Lev), 1.12 (dt, *J* = 14.5, 7.3 Hz, 3H, linker), 1.02 - 0.97 (m, 2H, linker), 0.90 - 0.85 (m, 6H, linker), 0.79 (t, *J* = 7.3 Hz, 4H, linker).

$^{13}$C NMR (151 MHz, $CDCl_3$) δ 206.59 (Lev), 172.49 (Lev), 166.02 (PhC=O), 165.32 (PhC=O), 165.25 (PhC=O), 165.19 (PhC=O), 164.42 (PhC=O), 138.70 (Ar), 138.60 (Ar), 138.17 (Ar), 137.73 (Ar), 137.72 (Ar), 137.52 (Ar), 137.47 (Ar), 133.35 (Ar), 133.12 (Ar), 132.84 (Ar), 132.57 (Ar), 132.52 (Ar), 130.26 (Ar), 130.15 (Ar), 130.10 (Ar), 129.99 (Ar), 129.78 (Ar), 129.74 (Ar), 129.61 (Ar), 129.48 (Ar), 129.46 (Ar), 129.27 (Ar), 128.91 (Ar), 128.65 (Ar), 128.49 (Ar), 128.42 (Ar), 128.40 (Ar), 128.27 (Ar), 128.21 (Ar), 128.21 (Ar), 128.17 (Ar), 128.12 (Ar), 128.10 (Ar), 128.00 (Ar), 127.98 (Ar), 127.88 (Ar), 127.87 (Ar), 127.83 (Ar), 127.50 (Ar), 127.45 (Ar), 127.41 (Ar), 127.36 (Ar), 127.26 (Ar), 127.16 (Ar), 126.59 (Ar), 102.00 (C-1), 101.84 (C-1), 101.59 ($PhCHO_2$), 101.51 (C-1), 101.31 (C-1), 101.06 (C-1), 80.73, 79.67, 79.58, 79.49, 75.58, 75.42, 74.72 ($PhCH_2$), 74.55 ($PhCH_2$), 74.52 ($PhCH_2$), 74.48 ($PhCH_2$), 73.17, 72.72 (C-2), 72.30, 72.19, 72.16, 71.91 (C-2 × 2, $PhCH_2$), 71.88 (C-2), 71.81 (C-2), 71.54, 71.52, 71.28 ($PhCH_2$), 71.07 ($PhCH_2$), 69.57 (C-6), 69.11 (linker), 68.90 (C-6), 67.25 (C-6), 66.54, 65.98 (C-6), 62.94 (C-6), 37.92 (Lev), 31.72 (linker), 29.81 (Lev), 29.18 (linker), 29.01 (linker), 27.81 (Lev), 25.69 (linker), 22.60 (linker), 14.08 (linker).

MALDI-TOF MS Calcd for $C_{134}H_{140}NaO_{33}$ $[M+Na]^+$ m/z: 2299.9175, found: 2299.4739.

**Example 1**

(1) Preparation of Compound 2-82: the structural formula and English name of compound 2-82 are as follows:

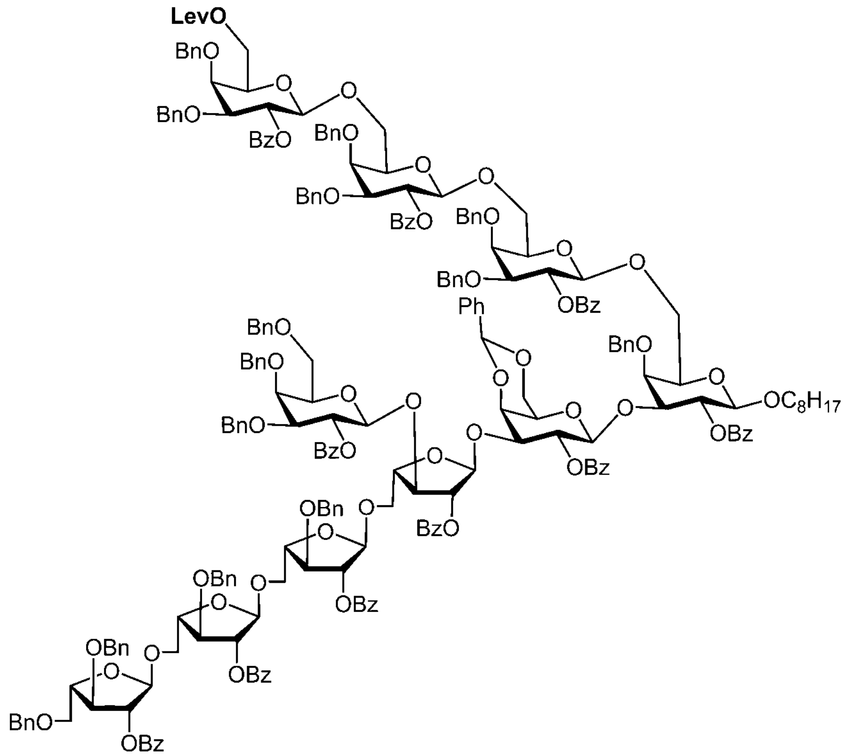

Octyl-2-*O*-benzoyl-3,4-di-*O*-benzyl-6-*O*-levulinyl-β-D-galactopyranosyl-(1→6)-2-*O*-be nzoyl-3,4-di-*O*-benzyl-β-D-galactopyranosyl-(1→6)-2-*O*-benzoyl-3,4-di-*O*-benzyl-β-D-galactop yranosyl-{2-*O*-benzoyl-3,5-*O*-di-benzyl-α-L-arabinofuranosyl-(1→5)-2-*O*-benzoyl-3-*O*-benzyl-α-L-arabinofuranosyl-(1→5)-2-*O*-benzoyl-3-*O*-benzyl-α-L-arabinofuranosyl-(1→5)-[2-*O*-benzo yl-3,4,6-*O*-tri-benzyl-β-D-galactopyranosyl-(1→3)]-2-*O*-benzoyl-α-L-arabinofuransyl-2-*O*-benz oyl-3,5-*O*-benzylidene-β-D-galactopyranosyl}-2-*O*-benzoyl-4-*O*-benzyl-β-D-galactopyranoside.

A two-necked flask, along with a stir bar, was dried by heating under vacuum. After cooling, the two-necked flask was filled with argon. 4Å molecular sieves (6 g) were added thereto, and the molecular sieves were activated by heating under vacuum with a blowtorch, and cooled to room temperature. A glycosyl donor compound 1-6 (300 mg, 554.86 μmol, 1.0 eq) was dissolved in dry DCM (20 mL) and added to the two-necked flask, and TTBP (1.38 g, 5.55 mmol, 10.0 eq) was added thereto. A resulting mixture was stirred at room temperature for 20 min, then cooled to -78°C. AgOTf (285.12 mg, 1.11 mmol, 2.0 eq) and *p*-TolSCl (74.60 μL, 554.86 μmol, 1.0 eq) were added thereto. A resulting mixture was stirred at -78°C for 5 min. TLC showed the complete disappearance of the glycosyl donor compound 1-6, indicating pre-activation was complete. A glycosyl acceptor compound 1-7 (237.49 mg, 527.12 μmol, 0.95 eq), dissolved in dry DCM (8 mL), was slowly added dropwise to the two-necked flask. A reaction was conducted at -78°C for 20 min. TLC showed the disappearance of the glycosyl acceptor 1-7, indicating the reaction was complete. A resulting reaction material containing a disaccharide donor was quickly heated to room temperature and stirred for 20 min. A resulting mixture was then cooled to -78°C. AgOTf (270.87 mg, 1.05 mmol, 1.90 eq) and *p*-TolSCl (70.87 μL, 527.12 μmol, 0.95 eq) were added thereto. A resulting mixture was stirred at -78°C for 5 min. TLC showed the complete disappearance of the disaccharide donor, indicating pre-activation was complete. The glycosyl acceptor compound 1-7 (224.99 mg, 499.38 μmol, 0.9 eq), dissolved in dry DCM (8 mL), was added dropwise to the two-necked flask. A reaction was conducted at -78°C for 20 min. TLC showed the disappearance of the glycosyl acceptor compound 1-7, indicating the reaction was complete. A resulting reaction material containing a trisaccharide donor was quickly heated to room temperature and stirred for 20 min. A resulting mixture was then cooled to -78°C. AgOTf (256.61 mg, 998.75 μmol, 1.8 eq) and *p*-TolSCl (67.14 μL, 499.38 μmol, 0.9 eq) were added thereto. A resulting mixture was stirred at -78°C for 5 min. TLC showed the complete disappearance of the trisaccharide donor, indicating pre-activation was complete. A glycosyl acceptor compound 1-4 (357.79 mg, 460.54 μmol, 0.83 eq), dissolved in dry DCM (8 mL), was slowly added dropwise to the two-necked flask. A resulting reaction was conducted at -78°C for 10 min and then naturally heated to continue the reaction for another 2 h. TLC showed the disappearance of the glycosyl acceptor compound 1-4, indicating the reaction was complete. A resulting reaction material containing a pentasaccharide donor was quickly heated to room temperature and stirred for 20 min. A resulting mixture was then cooled to -78°C. AgOTf (236.65 mg, 921.07 μmol, 1.66 eq) and *p*-TolSCl (61.92 μL, 460.54 μmol, 0.83 eq) were added thereto. A resulting mixture was stirred at -78°C for 5 min. TLC showed the complete disappearance of the pentasaccharide donor, indicating pre-activation was complete. A glycosyl acceptor compound 2-81 (1.33 g, 582.61 μmol, 1.05 eq), dissolved in dry DCM (8 mL), was added dropwise to the two-necked flask. A reaction was conducted at -78°C for

10 min and then naturally heated to continue the reaction for another 2 h. TLC indicated the reaction was complete. The reaction was quenched by adding TEA (2 mL). A resulting reaction material was filtered through diatomaceous earth. A resulting filtrate was concentrated and purified by FLASH rapid preparative chromatography (mobile phase: a mixture of petroleum ether, ethyl acetate, and DCM, with a volume fraction of ethyl acetate of 40% and a volume fraction of DCM of 15%) to obtain a white foamy powder, which was compound 2-82 (1.21 g, 64% yield).

$R_f$ = 0.55 (toluene/acetonitrile, 6 : 1). [α]24 D -3.3 (c 0.60, $CHCl_3$); $^1$H NMR (600 MHz, $CDCl_3$) δ 8.03 - 8.00 (m, 7H, Ar), 7.98 - 7.93 (m, 5H, Ar), 7.88 (d, *J* = 7.2 Hz, 2H, Ar), 7.85 - 7.83 (m, 2H, Ar), 7.57 - 7.48 (m, 14H, Ar), 7.44 - 7.37 (m, 18H, Ar), 7.34 (dd, *J* = 14.4, 7.4 Hz, 5H, Ar), 7.31 - 7.26 (m, 4H, Ar), 7.24 - 7.21 (m, 16H, Ar), 7.21 - 7.16 (m, 23H, Ar), 7.16 - 7.13 (m, 7H, Ar), 7.10 - 7.06 (m, 13H, Ar), 7.05 - 7.01 (m, 5H, Ar), 6.98 - 6.93 (m, 4H, Ar), 5.67 (dd, *J* = 10.0, 7.9 Hz, 1H, gal H-2), 5.55 - 5.51 (m, 2H, gal IH-2 × 2), 5.49 (s, 1H, $PhCHO_2$), 5.43 - 5.37 (m, 3H, ara H-2, gal H-2 × 2), 5.34 (s, 2H, ara H-2 × 2), 5.29 (dd, *J* = 9.9, 8.2 Hz, 1H, gal H-2), 5.23 (s, 1H, ara H-1), 5.23 (s, 1H, ara H-1), 5.11 (d, *J* = 3.0 Hz, 1H, ara H-2), 5.07 - 5.02 (m, 3H, ara H-1 × 2, $PhCH_2$), 4.98 (d, *J* = 12.0 Hz, 1H, $PhCH_2$), 4.89 (d, *J* = 11.2 Hz, 1H, $PhCH_2$), 4.83 (d, *J* = 11.7 Hz, 1H, $PhCH_2$), 4.76 (d, *J* = 11.4 Hz, 1H, $PhCH_2$), 4.73 (d, *J* = 11.5 Hz, 1H, $PhCH_2$), 4.71 (d, *J* = 11.4 Hz, 1H, $PhCH_2$), 4.69 (d, *J* = 11.5 Hz, 1H, $PhCH_2$), 4.66 - 4.62 (m, 4H, gal H-1, $PhCH_2$ × 3), 4.60 (d, *J* = 7.6 Hz, 1H, $PhCH_2$), 4.56 (dd, *J* = 10.1, 4.8 Hz, 3H, gal H-1, $PhCH_2$ × 2), 4.53 (d, *J* = 11.6 Hz, 1H, $PhCH_2$), 4.51 - 4.48 (m, 4H, gal H-1, $PhCH_2$ × 3), 4.44 (dd, *J* = 12.1, 3.5 Hz, 2H, $PhCH_2$ × 2), 4.40 - 4.34 (m, 5H, gal H-1 × 2, $PhCH_2$ × 3), 4.28 (d, *J* = 11.7 Hz, 1H, $PhCH_2$), 4.26 - 4.21 (m, 5H, gal H-6 × 2, $PhCH_2$), 4.18 (dd, *J* = 11.7, 7.3 Hz, 3H, $PhCH_2$ × 2), 4.13 (dd, *J* = 9.5, 5.4 Hz, 5H, gal H-6), 4.10 - 4.08 (m, 3H, gal H-1, gal H-6), 4.02 (dd, *J* = 11.0, 5.3 Hz, 2H, ara H-5, $PhCH_2$), 3.99 (s, 1H), 3.96 (d, *J* = 2.1 Hz, 1H), 3.92 - 3.88 (m, 3H, ara H-5, gal H-6), 3.85 (d, *J* = 5.2 Hz, 3H), 3.84 - 3.79 (m, 2H, ara H-5 × 2), 3.74 (dd, *J* = 10.4, 3.1 Hz, 1H), 3.69 - 3.62 (m, 5H, ara H-5 × 2), 3.56 (ddd, *J* = 14.3, 10.2, 4.1 Hz, 4H, ara H-5, gal H-6 × 3), 3.50 - 3.46 (m, 6H, ara H-5, gal H-6 × 2), 3.43 - 3.36 (m, 4H, gal H-6 × 2), 3.33 - 3.25 (m, 3H, linker), 3.15 (s, 1H), 2.92 - 2.83 (m, 1H, linker), 2.68 - 2.61 (m, 2H, Lev), 2.44 - 2.38 (m, 2H, Lev), 2.13 (s, 3H, Lev), 1.16 - 1.05 (m, 3H, linker), 1.05 - 0.94 (m, 3H, linker), 0.90 - 0.81 (m, 5H, linker), 0.78 (t, *J* = 7.3 Hz, 4H, linker).

$^{13}$C NMR (151 MHz, $CDCl_3$) δ 206.65 (Lev), 172.52 (Lev), 165.34 (C=O), 165.31 (C=O), 165.31 (C=O), 165.30 (C=O), 165.27 (C=O), 165.20 (C=O), 164.68 (C=O), 164.50 (C=O), 164.22 (C=O), 138.65 (Ar), 138.61 (Ar), 138.49 (Ar), 138.43 (Ar), 138.24 (Ar), 138.01 (Ar), 137.94 (Ar), 137.90 (Ar), 137.85 (Ar), 137.80 (Ar), 137.76 (Ar), 137.75 (Ar), 137.73 (Ar), 137.66 (Ar), 137.52 (Ar), 133.39 (Ar), 133.16 (Ar), 132.77 (Ar), 132.74 (Ar), 132.31 (Ar), 131.84 (Ar), 130.30 (Ar), 130.14 (Ar), 130.07 (Ar), 130.01 (Ar), 129.98 (Ar), 129.86 (Ar), 129.83 (Ar), 129.79 (Ar), 129.77 (Ar), 129.74 (Ar), 129.63 (Ar), 129.56 (Ar), 129.52 (Ar), 129.47 (Ar), 129.46 (Ar), 129.37 (Ar), 129.30 (Ar), 129.16 (Ar), 128.66 (Ar), 128.56 (Ar), 128.53 (Ar), 128.49 (Ar), 128.45 (Ar), 128.43 (Ar), 128.40 (Ar), 128.37 (Ar), 128.36 (Ar), 128.33 (Ar), 128.29 (Ar), 128.25 (Ar), 128.21 (Ar), 128.18 (Ar), 128.12 (Ar), 128.08 (Ar), 128.04 (Ar), 127.98 (Ar), 127.92 (Ar), 127.90 (Ar), 127.86 (Ar), 127.79 (Ar), 127.69 (Ar), 127.68 (Ar), 127.67 (Ar), 127.65 (Ar), 127.57 (Ar), 127.54 (Ar), 127.50 (Ar), 127.47 (Ar), 127.41 (Ar), 127.32 (Ar), 127.18 (Ar), 126.24 (Ar), 106.95 (C-1), 106.26 (C-1), 106.21 (C-1), 105.95 (C-1), 102.58 (C-1), 101.51 (C-1), 101.50 (C-1), 101.32 (C-1), 101.28 (C-1), 100.69 ($PhCHO_2$), 100.43 (C-1), 83.49, 83.28, 82.78 (ara C-2), 82.43, 82.14, 82.05, 81.88 (ara C-2), 81.76 (ara C-2 × 2), 81.65, 80.31, 80.24, 79.82, 79.71, 79.67, 79.54, 76.32, 75.89, 75.40, 74.91 ($PhCH_2$), 74.81 ($PhCH_2$), 74.55 ($PhCH_2$), 74.52 ($PhCH_2$), 74.44 ($PhCH_2$), 74.10 ($PhCH_2$), 73.48 ($PhCH_2$), 73.44 ($PhCH_2$), 73.33 ($PhCH_2$), 73.12, 72.52 ($PhCH_2$), 72.45 ($PhCH_2$), 72.39 ($PhCH_2$), 72.37 ($PhCH_2$), 72.30 ($PhCH_2$), 72.14 ($PhCH_2$), 72.01 ($PhCH_2$), 71.97 ($PhCH_2$), 71.94 (gal C-2), 71.90 (gal C-2), 71.87 (gal C-2), 71.65 (gal C-2), 71.56 (gal C-2), 71.35 ($PhCH_2$), 71.23 ($PhCH_2$), 71.05 ($PhCH_2$), 70.76 (gal C-2), 69.54 (gal C-6), 69.26 (gal C-6), 68.89 (linker), 68.68 (gal C-6), 68.19 (gal C-6), 66.77 (gal C-6), 66.71, 65.97 (ara C-5), 65.65 (ara C-5), 65.53 (ara C-5), 65.31 (ara C-5), 63.06 (gal C-6), 60.43, 37.94 (Lev), 31.74 (linker), 29.83 (Lev), 29.20 (linker), 29.19 (linker), 29.02 (linker), 27.85 (Lev), 25.70 (linker), 22.62 (linker), 14.11 (linker).

MALDI-TOF MS Calcd for $C_{244}H_{244}NaO_{59}$ $[M+Na]^+$ m/z: 4140.5990, found: 4140.7446.

(2) Preparation of Compound 2-34: the structural formula and English name of compound 2-34 are as follows:

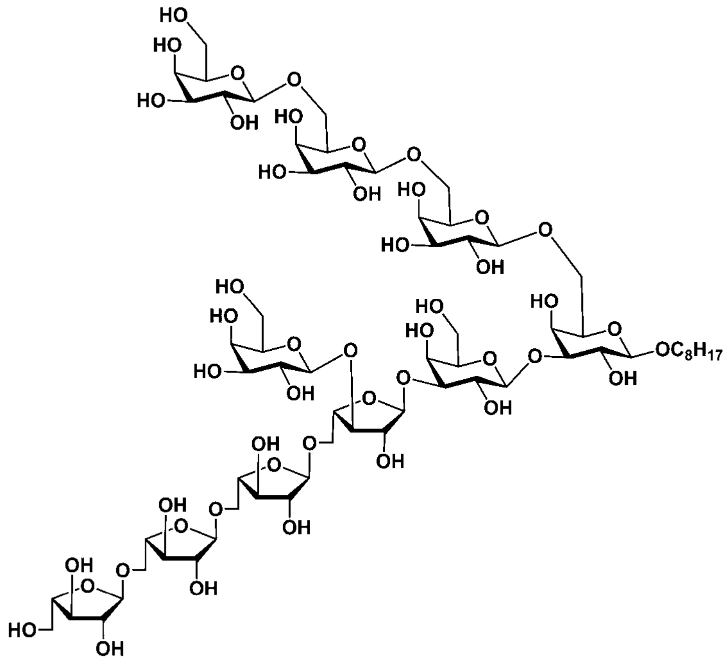

.

Octylβ-D-galactopyranosyl-(1→6)-β-D-galactopyranosyl-(1→6)-β-D-galactopyranosyl-( 1→6)-{α-L-arabinofuranosyl-(1→5)-α-L-arabinofuranosyl-(1→5)-α-L-arabinofuransyl-(1→5)-[ β-D-galactopyranosyl-(1→3)]-α-L-arabinofuranosyl-(1→3)-β-D-galactopyranosyl-(1→3)}-β-D-galactopyranoside.

Compound 2-82 (142 mg, 34.46 μmol, 1.0 eq) was dissolved in DCM (10 mL) and methanol (5 mL). A solution of sodium methoxide (30 wt%) in methanol was added dropwise thereto to adjust a pH value to 9-10. A reaction was conducted at room temperature for 9 h. TLC monitoring indicated the reaction was complete. A cation exchange resin was added thereto to neutralize the pH value to 7. The resin was removed by filtration, and a resulting filtrate was concentrated and purified by polystyrene gel exclusion chromatography (eluent: ethyl acetate) to obtain a crude debenzoylated product. The crude debenzoylated product was dissolved in a solvent mixture (ethyl acetate: methanol : water = 3 mL : 6 mL : 3 mL). 10% Pd/C (200 mg) was added thereto. A reaction was conducted at room temperature under a hydrogen pressure of 0.4 MPa for 18 h. A resulting reaction material was filtered through a microporous filtration membrane to remove the Pd/C. A resulting filtrate was concentrated and purified by dextran gel LH-20 exclusion chromatography (eluent: a mixture of methanol and water in a volume ratio of 1 : 1) to obtain a white glassy product, which was compound 2-34 (41 mg, 73% yield).

$^1$H NMR (600 MHz, $D_2O$) δ 5.27 (s, 1H, ara H-1), 5.08 (s, 2H, ara H-1 × 2), 5.08 (d, *J* = 1.1 Hz, 1H, ara H-1), 4.66 (d, *J* = 7.5 Hz, 1H, gal H-1), 4.53 (d, *J* = 7.9 Hz, 1H, gal H-1), 4.46 (dt, *J* = 12.3, 8.2 Hz, 4H, gal H-1 × 4), 4.41 - 4.38 (m, 2H), 4.23 - 4.18 (m, 4H), 4.13 - 4.11 (m, 3H), 4.10 - 4.07 (m, 2H), 4.05 - 4.02 (m, 2H, gal H-6 × 2), 4.00 (dt, *J* = 5.6, 2.7 Hz, 3H), 3.96 - 3.86 (m, 15H, ara H-5 × 2, gal H-6 × 3), 3.84 - 3.74 (m, 12H, ara H-5 × 6, gal H-6, linker), 3.73 - 3.68 (m, 8H, ara H-5 × 2), 3.68 - 3.63 (m, 4H), 3.53 (dd, *J* = 17.2, 7.8 Hz, 4H), 1.65 - 1.59 (m, 2H, linker), 1.31 - 1.24 (m, 10H, linker), 0.87 - 0.84 (m, 3H, linker).

$^{13}$C NMR (151 MHz, $D_2O$) δ 110.09 (ara C-1), 108.25 (ara C-1), 108.21 (ara C-1), 108.15 (ara C-1), 104.90 (gal C-1), 104.15 (gal C-1 ×2), 103.93 (gal C-1), 103.45 (gal C-1), 103.07 (gal C-1), 85.20, 84.70, 83.14, 83.08, 83.03, 82.80, 81.65, 81.55, 81.48, 80.47, 80.32, 77.47, 77.41, 77.25, 75.97, 75.88, 75.68, 74.48, 74.45, 74.11, 73.48, 73.33, 73.30, 73.24, 71.48 (gal C-6), 71.43, 70.92, 70.53, 70.08 (gal C-6), 69.96 (gal C-6), 69.77 (gal C-6), 69.35, 69.25, 69.20, 67.58 (gal C-6), 67.50 (gal C-6), 67.38 (ara C-5), 61.91 (ara C-5), 61.75 (ara C-5), 61.68 (ara C-5), 61.58 (linker), 31.86 (linker), 29.50 (linker), 29.25 (linker), 29.16 (linker), 25.82 (linker), 22.79 (linker), 14.20 (linker).

HRMS (ESI) Calcd for $C_{63}H_{107}O_{46}$ $[M-H]^-$: 1629.6139; found: 1629.6140.

**Test Example 1 Bioactivity Evaluation**

1. Cell Culture Conditions

Three pancreatic cancer cell lines (Aspc-1, Bxpc-3, Panc-1) and the hepatocyte cell line LO2 used in the experiments were provided by Peking University People's Hospital and the Institute of Materia Medica, Chinese Academy of Medical Sciences. The culture conditions for Aspc-1 and Bxpc-3 cells were RPMI 1640 medium (Gibco, Thermo Fisher Scientific, USA) supplemented with 10% fetal bovine serum (HyClone, Thermo Fisher Scientific, USA) and 1% penicillin/streptomycin (Life Technologies, Thermo Fisher Scientific, USA). The culture conditions for Panc-1 and LO2 cells were DMEM medium (Gibco, Thermo Fisher Scientific, USA) supplemented with 10% fetal bovine serum (HyClone, Thermo Fisher Scientific, USA) and 1% penicillin/streptomycin (Life Technologies, Thermo Fisher Scientific, USA). Cell culture was conducted at 37°C with adequate humidity and 5% $CO_2$ concentration.

2. Cell Thawing

The cell cryovials were retrieved from the liquid nitrogen tank and quickly thawed in a constant-temperature water bath pre-warmed to 37°C. The cells were centrifuged at 1,000 rpm for 5 min, the supernatant was discarded, and the cell pellet was transferred to complete medium (pre-warmed RPMI 1640 or DMEM). The medium and cell suspension were mixed evenly and then placed in an incubator for culture.

3. Cell Subculture

When the adherent cells grew to 80% to 90% confluence, they were subcultured. Specifically, the existing medium in the culture dish was completely removed. The cells were washed 2 times with sterile PBS (pH=7.4). Trypsin was then added and the cells were digested at room temperature for about 2 min. After most cells detached, an equal volume of complete medium was added to terminate the digestion. The adherent cells were gently pipetted until they completely detached. The suspension was centrifuged at 1,000 rpm for 5 min, the supernatant was discarded, and the cell pellet was resuspended in fresh complete medium. The cells were subcultured at a 1 : 3 ratio.

4. Evaluation of the Proliferation Inhibitory Effect of Compounds at 50 μM on Pancreatic Cancer Cells

Three types of pancreatic cancer cells (Aspc-1, Bxpc-3, Panc-1) were seeded into 96-well plates at a density of $5\times10^3$ cells/well (100 μL). After the cells adhered, gemcitabine (50 μM), compound 2-11 (140-mer 2-11, 50 μM), 2-30 (70-mer 2-30, 50 μM), 2-31 (40-mer 2-31, 50 μM), 2-32 (30-mer 2-32, 50 μM), 2-33 (10-mer A 2-33, 50 μM), 2-34 (10-mer B 2-34, 50 μM), 2-35 (5-mer A 2-35, 50 μM), 2-36 (5-mer B 2-36, 50 μM), and 2-37 (5-mer C 2-37, 50 μM) were added, respectively. The cells were co-incubated for 72 h. Then, 10 μL of CCK-8 reagent was added to each well and incubated at 37°C for 2 h. After thorough mixing, the absorbance at 450 nm was measured by using a microplate reader. A cell survival rate was calculated by using the following formula:

$$\text{Cell survival rate (\%)} = (\text{OD sample} - \text{OD_blank}) / (\text{OD_control} - \text{OD_blank}) \times 100\%.$$

The structural formulas of compounds 2-11, 2-30, 2-31, 2-32, 2-33, 2-34, 2-35, 2-36, and 2-37 are as follows:

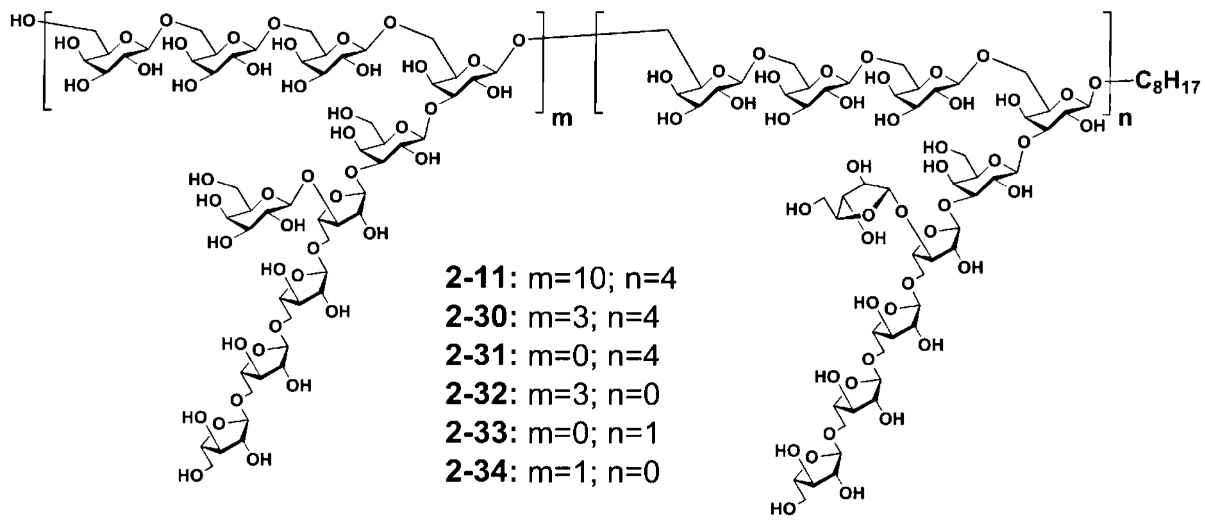

**2-11:** m=10; n=4
**2-30:** m=3; n=4
**2-31:** m=0; n=4
**2-32:** m=3; n=0
**2-33:** m=0; n=1
**2-34:** m=1; n=0

,

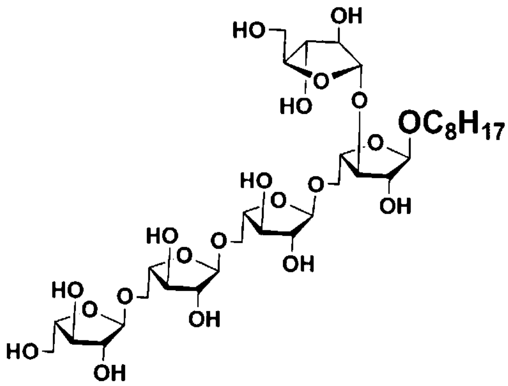

2-35

,

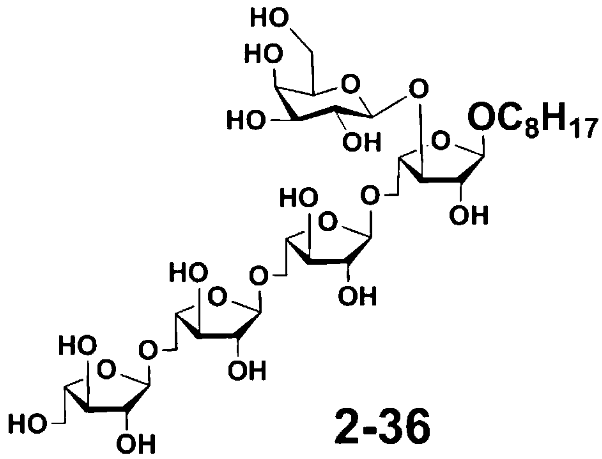

2-36

,

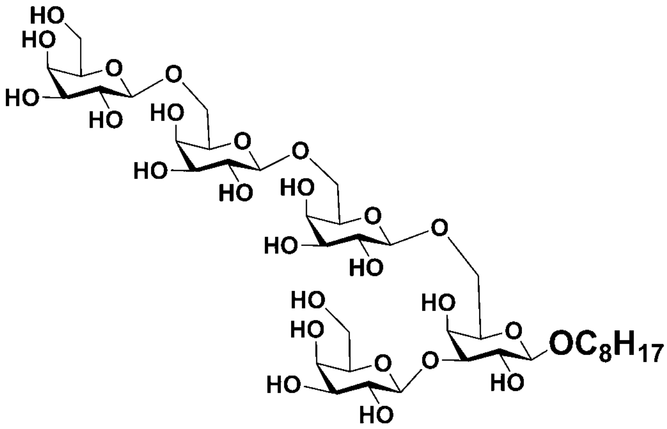

2-37

.

5. Evaluation of the Proliferation Inhibitory Effect of Compound 2-34 at Different Concentrations on Pancreatic Cancer Cells

Three types of pancreatic cancer cells (Aspc-1, Bxpc-3, and Panc-1) were seeded into 96-well plates at a density of $5\times10^3$ cells/well (100 μL). After the cells adhered, different concentrations of compound 2-34 (3.125 μM, 6.25 μM, 12.5 μM, 25 μM, 50 μM, 100 μM) were added. The cells were co-incubated for 72 h. Then, 10 μL of CCK-8 reagent was added to each well and incubated at 37°C for 2 h. After thorough mixing, the absorbance at 450 nm was measured by using a microplate reader, and the cell survival rate was calculated.

6. Evaluation of the Cytotoxicity of Compound 2-34 at Different Concentrations on LO2 Hepatocytes

LO2 hepatocytes were seeded into 96-well plates at a density of $5\times10^3$ cells/well (100 μL). After the cells adhered, different concentrations of compound 2-34 (3.125 μM, 6.25 μM, 12.5 μM, 25 μM, 50 μM, 100 μM) and the positive control drug gemcitabine (3.125 μM, 6.25 μM, 12.5 μM, 25 μM, 50 μM, 100 μM) were added, respectively. The cells were co-incubated for 72 h. Then, 10 μL of CCK-8 reagent was added to each well and incubated at 37°C for 2 h. After thorough mixing, the absorbance at 450 nm was measured by using a microplate reader, and the cell survival rate was calculated.

7. Evaluation of the Effect of Compound 2-34 at Different Concentrations on Apoptosis of Pancreatic Cancer Panc-1 Cells

Pancreatic cancer Panc-1 cells were seeded into 24-well plates at a density of $3\times10^4$ cells/well. After the cells adhered, different concentrations of compound 2-34 (25 μM, 50 μM, 100 μM) were added and co-incubated for 72 h. All

cells were collected, washed 2 times with PBS, and resuspended in binding buffer (Beyotime, Shanghai, China). Then, 5 μL of Annexin V-FITC (fluorescein isothiocyanate) and 10 μL of PI (propidium iodide) dye were added in sequence, and the cells were stained in the dark for 20 min. The apoptosis rate was detected by using a flow cytometer (Beckman CytoFLEX), and the specific apoptosis ratio was analyzed by using CytoFLEX software.

8. Evaluation of the Effect of Compound 2-34 at Different Concentrations on the Cell Cycle of Pancreatic Cancer Panc-1 Cells

Pancreatic cancer Panc-1 cells were seeded into 24-well plates at a density of $6\times10^4$ cells/well. After the cells adhered, different concentrations of compound 2-34 (25 μM, 50 μM, 100 μM) were added and co-incubated for 72 h. All cells were collected, washed 2 times with PBS, and fixated in 70% (volume fraction) ethanol overnight. The fixated cells were collected, washed 2 times with PBS, and stained with PI staining solution containing RNase (ribonuclease) in the dark for 30 min. The nucleic acid content in the cells was detected by using a flow cytometer, and the percentage of cells in each cycle phase was analyzed by using ModFit software.

9. Test Results

The anti-pancreatic cancer activity of the intact *Panax notoginseng* glycan (140-mer 2-11) and its fragments (70-mer 2-30, 40-mer 2-31, 30-mer 2-32, 10-mer A 2-33, 10-mer B 2-34, 5-mer A 2-35, 5-mer B 2-36, 5-mer C 2-37) was evaluated *in vitro* by using three pancreatic cancer cell lines: Aspc-1, Bxpc-3, and Panc-1. First, the proliferation inhibitory effects of each glycan molecule on the three pancreatic cancer cell lines were evaluated at a concentration of 50 μM. The three pancreatic cancer cell lines were co-incubated with the compounds for 72 h, and the specific cell survival was detected by using the CCK-8 method. The experimental results show (panel a in FIG. 2) that at 50 μM, the intact *Panax notoginseng* glycan and most of the *Panax notoginseng* glycan fragments show almost no significant inhibitory effect on the proliferation of the three pancreatic cancer cells. However, compound 2-34 exhibits desirable activity (inhibition rates: 74.9% for Aspc-1, 29.5% for Bxpc-3, and 72.1% for Panc-1). It is worth mentioning that the inhibition rates of compound 2-34 against Aspc-1 and Panc-1 cells exceed those of the first-line clinical anti-pancreatic cancer drug gemcitabine (gemcitabine inhibition rates: 61.4% for Aspc-1, 23.2% for Panc-1). Furthermore, for all three pancreatic cancer cell lines, the proliferation inhibition by compound 2-34 shows a significant concentration dependence (panel b in FIG. 2). To further assess the safety of compound 2-34, its cytotoxicity against human normal liver LO2 cells was tested. The experimental results show that even at a concentration up to 100 μM, compound 2-34 exhibits no cytotoxicity towards the growth of LO2 hepatocytes, demonstrating a superior safety profile compared with the positive control drug gemcitabine (panel c in FIG. 2).

Since Panc-1 cells exhibit some resistance to gemcitabine but are relatively sensitive to compound 2-34, the present disclosure further investigated how compound 2-34 acts on Panc-1 cells (FIG. 3). Panc-1 cells were co-incubated with three different concentration gradients of compound 2-34 (25 μM, 50 μM, 100 μM) for 72 h. The effect of compound 2-34 on cell apoptosis was explored by using the Annexin V-FITC/PI double-staining method combined with flow cytometry analysis. The results show that as the drug concentration increases, the proportion of apoptotic cells increases. The apoptosis rate is 16.7% in the blank control group, 31.99% in the 25 μM group, 31.92% in the 50 μM group, and reaches 67.59% in the 100 μM group. The results demonstrate that compound 2-34 inhibits tumor cell proliferation by promoting cell apoptosis. To further explore how compound 2-34 promotes apoptosis, the present disclosure has focused on the cell cycle. Panc-1 cells were co-incubated with compound 2-34 at three different concentrations (25 μM, 50 μM, 100 μM) for 72 h. The effect of compound 2-34 on the cell cycle was investigated by using flow cytometry analysis. The results show that as the drug concentration increases, the percentage of cells in different phases changes significantly. The proportion of cells in the G1 phase decreases from 58.43% (blank control) to 51.33% (25 μM), 46.99% (50 μM), and 42.43% (100 μM). In contrast, the proportion of cells in the S phase increases from 15.17% (blank control) to 22.19% (25 μM), 27.66% (50 μM), and 36.29% (100 μM). The number of cells in the G2 phase remains relatively stable with minor changes. The cell cycle experiment results indicate that compound 2-34 could arrest Panc-1 cells in the S phase. Therefore, it is hypothesized that the mechanism of action of compound 2-34 primarily involves arresting the cell cycle in the S phase, thereby promoting tumor cell apoptosis and subsequently exerting antitumor efficacy.

The above are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

**1.** A glycan having a structure shown in Formula I:

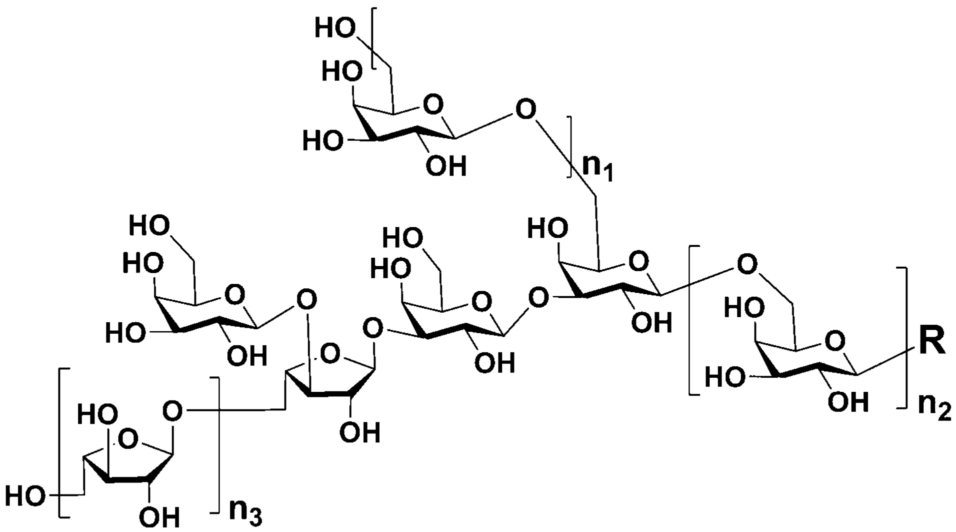

Formula I,

wherein in the Formula I, $n_1$ is 0 to 6, $n_2$ is 0 to 2, $n_3$ is 0 to 3, and R is C1-C10 alkoxy.

**2.** The glycan of claim 1, wherein $n_1$ is 1 to 5.

**3.** The glycan of claim 2, wherein $n_1$ is 2 to 4.

**4.** The glycan of claim 1, wherein $n_2$ is 0 to 1.

**5.** The glycan of claim 1, wherein $n_3$ is 1 to 3.

**6.** The glycan of claim 5, wherein $n_3$ is 2 to 3.

**7.** The glycan of claim 1, wherein R is selected from the group consisting of methoxy, ethoxy, propoxy, butoxy, pentoxy, hexoxy, heptoxy, octoxy, nonoxy, and decoxy.

**8.** The glycan of claim 7, wherein R is C5-C10 alkoxy.

**9.** The glycan of claim 8, wherein R is linear alkoxy.

**10.** The glycan of any one of claims 1 to 9, wherein $n_1$ is 3, $n_2$ is 0, $n_3$ is 3, and R is n-octoxy.

**11.** A method for preparing the glycan of any one of claims 1 to 10, comprising the following steps:

mixing a compound 1-3, silver trifluoromethanesulfonate, *p*-toluenesulfenyl chloride, and an organic solvent, and conducting a first activation to obtain a first activation product system;
mixing the first activation product system and a compound 1-4, and conducting a first glycosylation coupling to obtain a first coupling product system;
mixing the first coupling product system, the silver trifluoromethanesulfonate, and the *p*-toluenesulfenyl chloride, and conducting a second activation to obtain a second activation product system;
mixing the second activation product system and a compound 1-5, and conducting a second glycosylation coupling to obtain a compound 1-2; and
subjecting the compound 1-2 to debenzoylation and debenzylation-debenzalization in sequence to obtain the glycan having the structure shown in the Formula I,
wherein the compound 1-3, the compound 1-4, the compound 1-5, and the compound 1-2 have the following structural formulas, respectively:

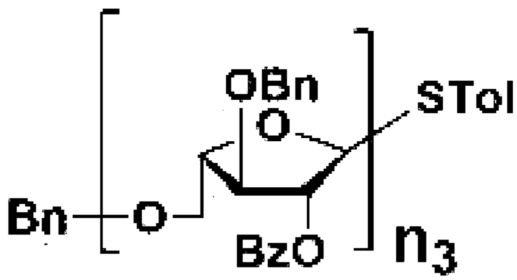

1-3 ,

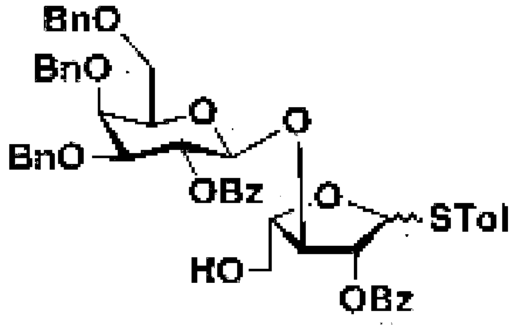

1-4 ,

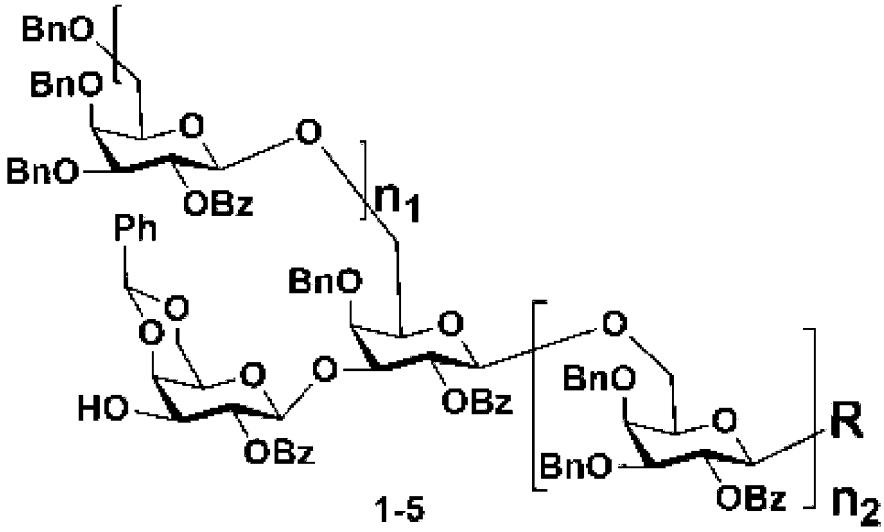

1-5 ,

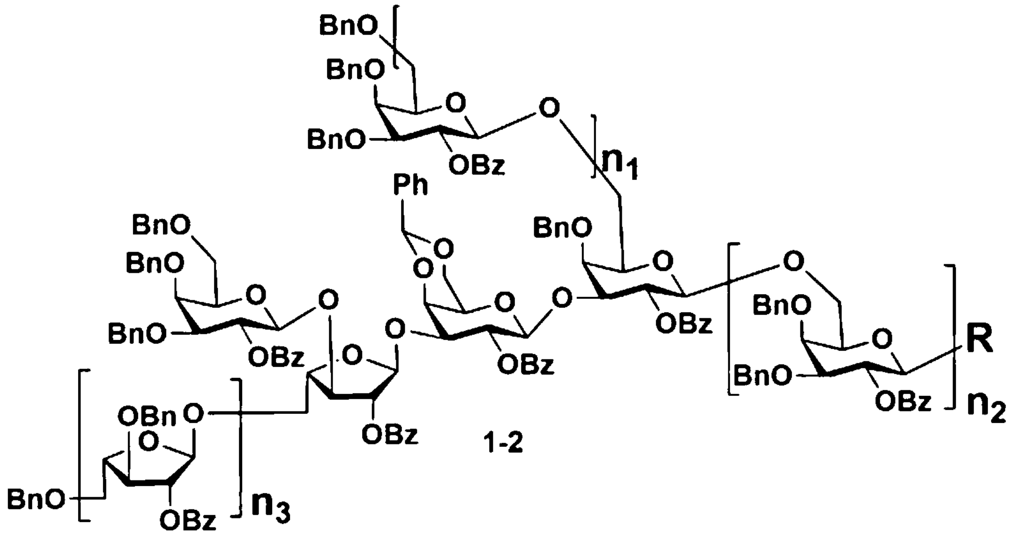

1-2 ,

wherein $n_1$, $n_2$, $n_3$, and R are as defined in the Formula I.

**12.** The method of claim 11, wherein in raw materials for the first activation, a molar ratio of the compound 1-3, the silver trifluoromethanesulfonate, and the *p*-toluenesulfenyl chloride is in a range of 1 : 1.2-5 : 1.

**13.** The method of claim 11 or 12, wherein the first activation is conducted at a temperature of -60°C to -78°C for 2 minutes to 10 minutes.

**14.** The method of claim 11, wherein in raw materials for the first glycosylation coupling, a molar ratio of the compound 1-3 to the compound 1-4 is in a range of 1 : 0.85-0.92.

**15.** The method of claim 11 or 14, wherein the first glycosylation coupling comprises conducting a first stage reaction and a second stage reaction in sequence; wherein the first stage reaction is conducted at a temperature of -60°C to -78°C for 5 minutes to 15 minutes; and after completion of the first stage reaction, an obtained reaction product is subjected to natural heating from the temperature for the first stage reaction to conduct the second stage reaction, and the second stage reaction is conducted for 1.5 hours to 2.5 hours.

**16.** The method of claim 11, wherein in raw materials for the second activation, a molar ratio of the compound 1-4, the silver trifluoromethanesulfonate, and the *p*-toluenesulfenyl chloride is in a range of 1 : 1.2-5 : 1.

**17.** The method of claim 11 or 16, wherein the second activation is conducted at a temperature of -60°C to -78°C for 2 minutes to 10 minutes.

**18.** The method of claim 11, wherein in raw materials for the second glycosylation coupling, a molar ratio of the compound 1-4 to the compound 1-5 is in a range of 0.8-0.85 : 1-2.

**19.** The method of claim 11 or 18, wherein the second glycosylation coupling comprises conducting a first stage reaction and a second stage reaction in sequence; wherein the first stage reaction is conducted at a temperature of -60°C to -78°C for 5 minutes to 15 minutes; and after completion of the first stage reaction, an obtained reaction product is subjected to natural heating from the temperature for the first stage reaction to conduct the second stage reaction, and the second stage reaction is conducted for 1.5 hours to 2.5 hours.

**20.** The method of claim 11, wherein subjecting the compound 1-2 to the debenzoylation and the debenzylation-debenzalization in sequence comprises:

mixing the compound 1-2 and an organic solvent, and conducting the debenzoylation in a basic environment to obtain a debenzoylated compound; and
mixing the debenzoylated compound, a palladium-on-carbon catalyst, an organic solvent, and water, and conducting the debenzylation-debenzalization in a hydrogen atmosphere.

**21.** The method of claim 20, wherein the basic environment has a pH value of 9 to 10; and a basic reagent providing the basic environment is a solution of sodium methoxide in methanol.

**22.** The method of claim 20 or 21, wherein the debenzoylation is conducted at a temperature of 10°C to 30°C for 5 hours to 24 hours.

**23.** The method of claim 20, wherein the debenzylation-debenzalization is conducted at a temperature of 10°C to 30°C under a hydrogen pressure of 0.3 MPa to 10 MPa for 5 hours to 24 hours.

**24.** Use of the glycan of any one of claims 1 to 10 in preparation of an antitumor drug.

**25.** The use of claim 24, wherein a tumor is selected from the group consisting of pancreatic cancer, gastric cancer, liver cancer, and cervical cancer..

**26.** The use of claim 24 or 25, wherein the antitumor drug comprises the glycan and a pharmaceutically acceptable carrier, and the glycan in the antitumor drug has a mass fraction of 0.1% to 99.9%.

**27.** The use of claim 26, wherein a dosage form of the antitumor drug is selected from the group consisting of an injection and an oral tablet.

**28.** The use of claim 27, wherein an administration route of the antitumor drug is selected from the group consisting of oral administration, intravenous injection, and subcutaneous injection.

**29.** An antitumor drug, comprising the glycan of any one of claims 1 to 10 and a pharmaceutically acceptable carrier.

**30.** The antitumor drug of claim 29, wherein the glycan in the antitumor drug has a mass fraction of 0.1% to 99.9%.

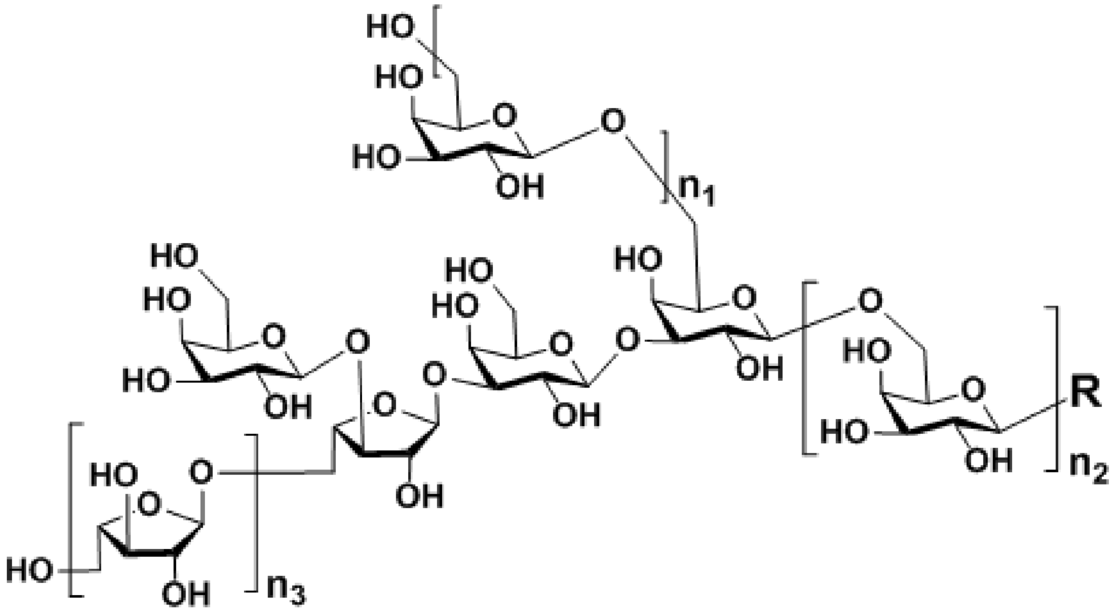

FIG. 1

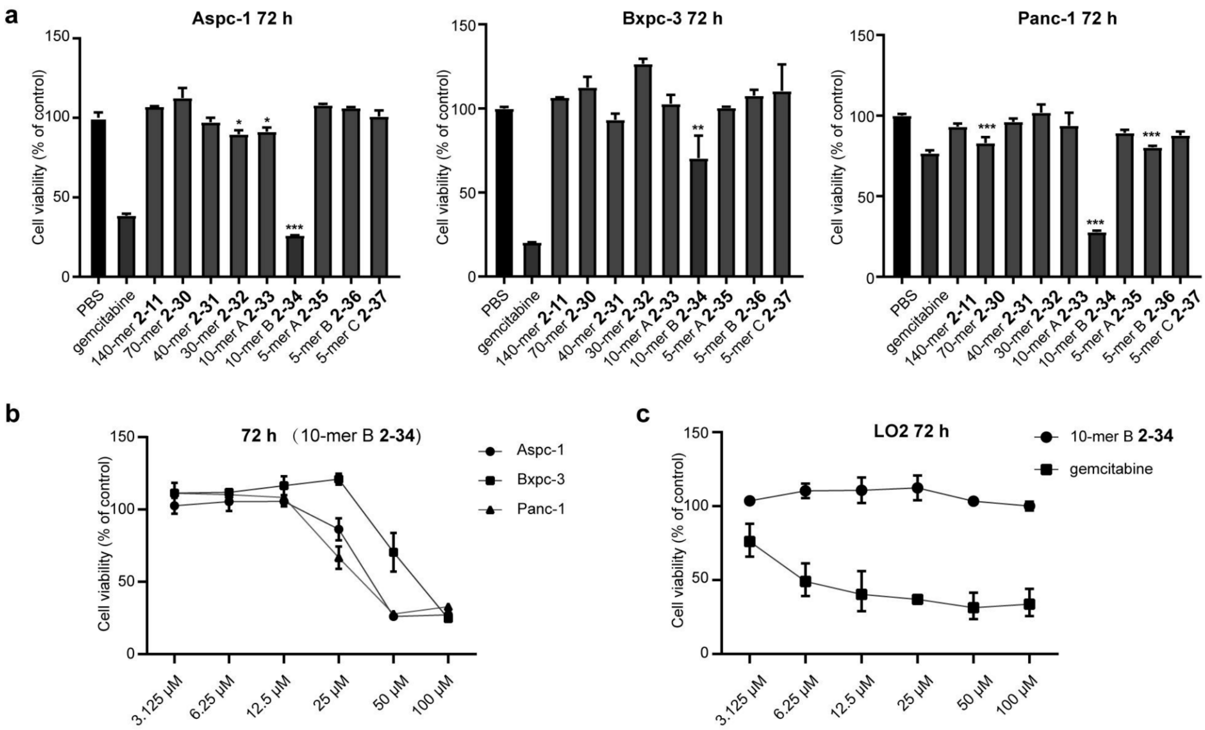
a
Aspc-1 72 h
Bxpc-3 72 h
Panc-1 72 h
Cell viability (% of control)
150
100
50
0
PBS
gemcitabine
140-mer 2-11
70-mer 2-30
40-mer 2-31
30-mer 2-32
10-mer A 2-33
10-mer B 2-34
5-mer A 2-35
5-mer B 2-36
5-mer C 2-37
b
72 h (10-mer B 2-34)
Aspc-1
Bxpc-3
Panc-1
c
LO2 72 h
10-mer B 2-34
gemcitabine
3.125 μM
6.25 μM
12.5 μM
25 μM
50 μM
100 μM

FIG. 2

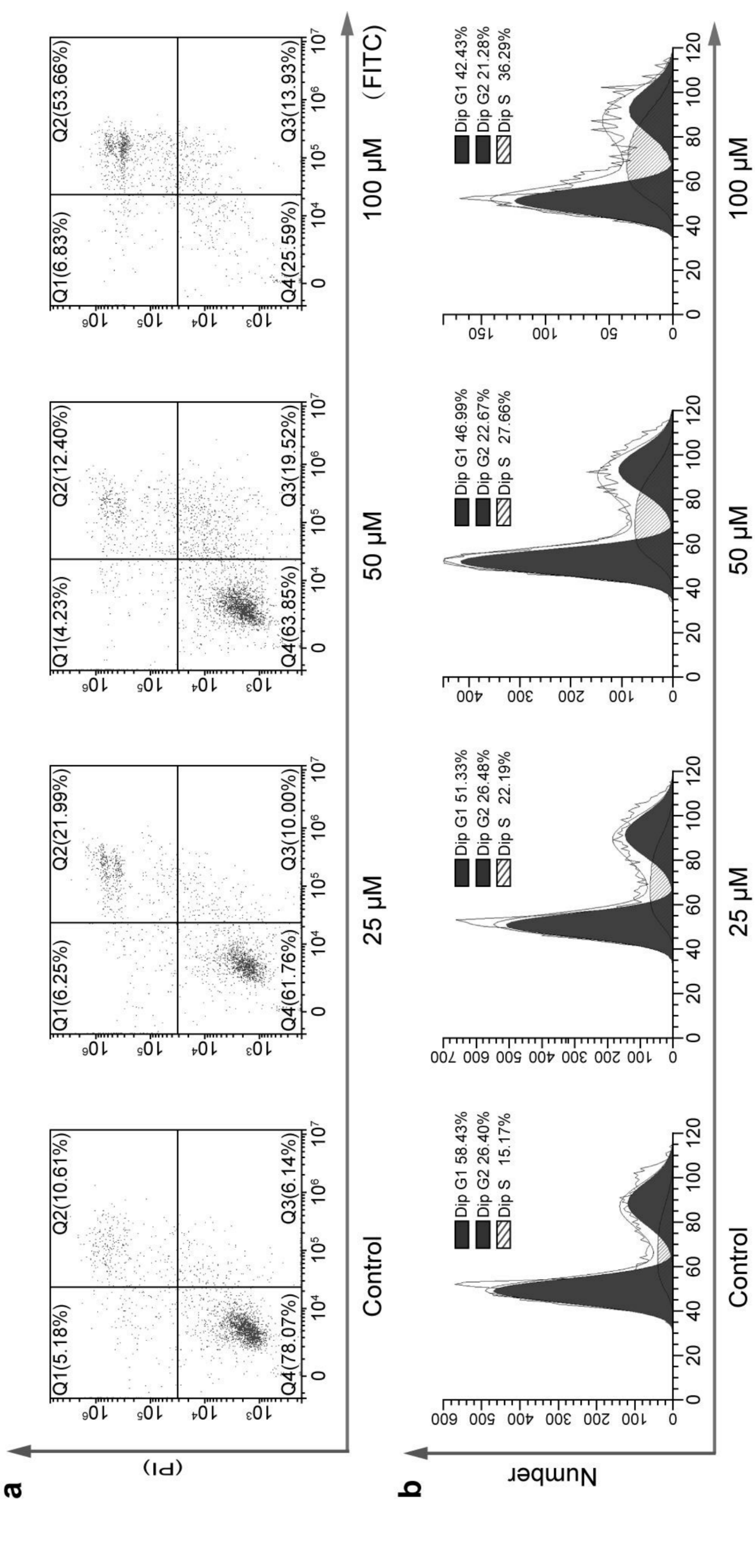
a
(PI)
(FITC)
Q1(5.18%)
Q2(10.61%)
Q3(6.14%)
Q4(78.07%)
Control
Q1(6.25%)
Q2(21.99%)
Q3(10.00%)
Q4(61.76%)
25 µM
Q1(4.23%)
Q2(12.40%)
Q3(19.52%)
Q4(63.85%)
50 µM
Q1(6.83%)
Q2(53.66%)
Q3(13.93%)
Q4(25.59%)
100 µM
b
Number
Dip G1 58.43%
Dip G2 26.40%
Dip S 15.17%
Control
Dip G1 51.33%
Dip G2 26.48%
Dip S 22.19%
25 µM
Dip G1 46.99%
Dip G2 22.67%
Dip S 27.66%
50 µM
Dip G1 42.43%
Dip G2 21.28%
Dip S 36.29%
100 µM

FIG. 3

**INTERNATIONAL SEARCH REPORT**

International application No.
**PCT/CN2024/117022**

**A. CLASSIFICATION OF SUBJECT MATTER**

C08B 37/00(2006.01)i; A61K 31/715(2006.01)i

According to International Patent Classification (IPC) or to both national classification and IPC

**B. FIELDS SEARCHED**

Minimum documentation searched (classification system followed by classification symbols)

IPC: C08B 37/-; A61K 31/-

Documentation searched other than minimum documentation to the extent that such documents are included in the fields searched

Electronic data base consulted during the international search (name of data base and, where practicable, search terms used)

CNTXT, WPABSC, ENTXT, VEN, CNKI, STN: 多糖, 聚糖, 糖基化偶联, 脱苯, 脱苯甲酰基, 羟基, 苄氧基, glycan, pancreatic, glycosylation, coupling, activation

**C. DOCUMENTS CONSIDERED TO BE RELEVANT**

| Category* | Citation of document, with indication, where appropriate, of the relevant passages | Relevant to claim No. |
|---|---|---|
| PX | CN 116925255 A (PEKING UNIVERSITY) 24 October 2023 (2023-10-24)<br>description, paragraphs [0005]-[0026] | 1-30 |
| PX | YAO, Wenlong et al. "Donor Preactivation-Based Glycan Assembly: from Manual to Automated Synthesis"<br>*Account of Chemical Research,* 16 April 2024 (2024-04-16),<br>page 1589 | 1-30 |
| A | 吴勇等 (WU, Yong et al.). "多糖化学合成研究进展 (Recent Advances in Chemical Synthesis of Polysaccharides)"<br>*化学学报 (Acta Chimica Sinica),* 15 July 2019 (2019-07-15),<br>page 584, right-hand column, paragraph 4 to pages 589, figure 13 | 1-30 |
| A | LIU, Mingli et al. "Glycan Assembly Strategy: From Concept to Application"<br>*Chemical Record,* 30 November 2021 (2021-11-30),<br>page 3271, right-hand column, paragraph 1, and figure 41 | 1-30 |
| A | CN 111184704 A (SOUTH CHINA INSTITUTE OF COLLABORATIVE INNOVATION) 22 May 2020 (2020-05-22)<br>description, paragraphs [0010]-[0030] | 1-30 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 November 2024** | **29 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/117022**

| Patent document cited in search report | Publication date (day/month/year) | Patent family member(s) | Publication date (day/month/year) |
|---|---|---|---|
| CN 116925255 A | 24 October 2023 | None | |
| CN 111184704 A | 22 May 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311139456